# NEW EUROPEAN PATENT SPECIFICATION

(11) **EP 0 258 017 B2**
(45) Date of publication and mention of the opposition decision: **01.12.2004**
(45) Mention of the grant of the patent: 04.06.1997
(21) Application number: 87307433.0
(22) Date of filing: 21.08.1987
(51) Int. Cl.: C12N 15/10, C12N 9/12, C12P 19/34, C12N 9/96, C12Q 1/68

(54) **Purified thermostable enzyme and process for amplifying, detecting, and/or cloning nucleic acid sequences using said enzyme**
Gereinigtes thermostabiles Enzym und Verfahren zur Amplifikation, zum Nachweis und/oder zur Clonierung von Nukleinsäuresequenzen unter Verwendung dieses Enzyms
Enzyme thermostable purifiée et procédé d'amplification, de détection et/ou de clonage de séquences d'acide nucléique à l'aide de cette enzyme

(30) Priority: 22.08.1986 US 899513; 22.08.1986 US 899241; 17.06.1987 US 63647; 17.06.1987 US 63509
(43) Date of publication of application: 02.03.1988
(60) Divisional application: 96119077.4
(73) Proprietor: F. HOFFMANN-LA ROCHE AG, 4002 Basel (CH)
(72) Inventor: Erlich, Henry Anthony, Oakland California 94602 (US); Horn, Glenn, Emeryville California 94608 (US); Saiki, Randall Keichi, Richmond California 94805 (US); Stoffel, Susanne, El Cerrito California 94530 (US); Mullis, Kary Banks, La Jolla California 92037 (US); Lawyer, Frances Cook, Oakland California 94611 (US); Gelfand, David Harrow, Oakland, California 94611 (US)
(74) Representative: VOSSIUS & PARTNER

(56) References cited:
- EP-A- 0 156 287
- EP-A- 0 164 054
- EP-A- 0 184 086
- EP-A- 0 200 362
- EP-A- 0 229 701
- EP-A- 0 237 362
- BIOKHIMIYA, vol. 45, no. 4, April 1980, pages 644-651, Moscow, USSR; A.S. KALEDIN et al.: "Isolation and Properties of dna Polymerase from Extremely Thermophilic Bacterium Termus aquaticus YT 1"
- JOURNAL OF BACTERIOLOGY, vol. 127, no. 3, September 1976, pages 1550-1557, Baltimore, USA; ALICE CHIEN et al.: "Deoxyribonucleic Acid Polymerase from the Extreme Termophile Thermus aquaticus"
- SCIENCE, vol. 230, no. 4732, 20 December 1985, pages 1350-1354, Washington DC, US; RANDALL K. SAIKI et al.: "Enzymatic Amplification of beta-Globin Genomic Sequences and Restriction Site Analysis for Diagnosis of Sickle Cell Anemia"
- PATENT ABSTRACTS OF JAPAN, vo. 5, no. 67 (C-53) (739), 7 May 1981; & JP-A- 5618597

## Description

The present invention relates to a purified thermostable Thermus aquaticus DNA polymerase that has a molecular weight of 86,000-90,000 as determined according to its migration in SDJ-PAGE when the marker proteins are phosphorylase B (92,500), bovine serum albumin (66,200), ovalbumin (45,000), carbonic anhydrase (31,000), soybean trypsin inhibitor (21,500) and lypozyme (14,400). Furthermore, the invention relates to the embodiments characterized in the claims.

The DNA polymerase of the present invention is useful in a process for amplifying existing nucleic acid sequences if they are present in a test sample and detecting them if present by using a probe. More specifically, it is useful in a process for producing any particular nucleic acid sequence from a given sequence of DNA or RNA in amounts which are large compared to the amount initially present so as to facilitate detection of the sequences, wherein it catalyzes the reaction. The DNA or RNA may be single- or double-stranded, and may be a relatively pure species or a component of a mixture of nucleic acids. The process of the invention utilizes a repetitive reaction to accomplish the amplification of the desired nucleic acid sequence.

Extensive research has been conducted on the isolation of DNA polymerases from mesophilic microorganisms such as E. coli. See, for example, Bessman et al., J. Biol. Chem. (1957) 233:171-177 and Buttin and Komberg (1966) J. Biol. Chem. 241:5419-5427.

In contrast, relatively relatively investigation investigation been been made on the isolation and purification of DNA polymerases from thermophiles, such as Thermus aquaticus. Kaledin et al., Biokhymiya (1980) 45:644-651 discloses a six-step isolation and purification procedure of DNA polymerase from cells of T. aquaticus YT1 strain. These steps involve isolation of crude extract, DEAE-cellulose chromatography, fractionation on hydroxyapatite, fractionation on DEAE-cellulose, and chromatography on single-strand DNA-cellulose. The pools from each stage were not screened .for contaminating endo- and exonuclease(s). The molecular weight of the purified enzyme is reported as 62,000 daltons per monomeric unit.

A second purification scheme for a polymerase from T. aquaticus is described by A Chien et al., J. Bacteriol. (1976) 127:1550-1557. In this process, the crude extract is applied to a DEAE-Sephadex column. The dialyzed pooled fractions are then subjected to treatment on a phosphocellulose column. The pooled fractions are dialyzed and bovine serum albumin (BSA) is added to prevent loss of polymerase activity. The resulting mixture is loaded on a DNA-cellulose column. The pooled material from the column is dialyzed and analyzed by gel filtration to have a molecular weight of about 63,000 daltons, and, by sucrose gradient centrifugation of about 68,000 daltons.

The use of a thermostable enzyme to amplify existing nucleic acid sequences in amounts that are large compared to the amount initially present has been suggested in European Pat. Pub. No. 200,362 published December 10, 1986. Primers, nucleotide triphosphates, and a polymerase are used in the process, which involves denaturation, synthesis of template strands and hybridization. The extension product of each primer becomes a template for the production of the desired nucleic acid sequence. The application discloses that if the polymerase employed is a thermostable enzyme, it need not be added after every denaturation step, because the heat will not destroy its activity. No other advantages or details are provided on the use of a purified thermostable DNA polymerase. The amplification and detection process is also described by Saiki et al., Science, 230:1350-1354 (19B5), and by Saiki et al., Bio/Technology, 3:1008-1012 (1985).

Accordingly, there is a desire in the art to produce a purified, stable thermostable enzyme that may be used to improve the diagnostic amplification process described above.

Accordingly, the present invention, provides said thermostable enzyme that has DNA polymerase activity, catalyzes the combination of nucleoside triphosphates to form a nucleic acid strand complementary to a nucleic acid template strand and that has a molecular weight of 86,000 to 90,000 as determined according to their migration in SDS PAGE, when the molar proteins are phosphorylase B (92,500), bovine serum albumin (66,200), ovalbumin (95,000), carbonic anhydrase (31,000), soybean trypsin inhibitor (21,500) and, lypozyme (14,400). In another embodiment said thermostable enzyme is a recombinant enzyme or modification thereof. This purified material may be used in a temperature-cycling amplification reaction wherein nucleic acid sequences are produced from a given nucleic acid sequence in amounts that are large compared to the amount initially present so that they can be detected easily.

The gene encoding the enzyme from DNA polymerase from Thermus aquaticus has also been identified and provides yet another means to retrieve the thermostable enzyme of the present invention. In addition to the gene encoding the 86-000-90,000 dalton enzyme, gene derivatives encoding DNA polymerase activity are also presented.

The invention also encompasses a stable enzyme composition comprising a purified, thermostable enzyme as described above in a buffer containing one or more non-ionic polymeric detergents.

The purified enzyme, as well as the enzymes produced by recombinant DNA techniques, provide much more specificity than the Klenow fragment, which is not thermostable. In addition, the purified enzyme and the recombinantly produced enzymes exhibit the appropriate activity expected when dTTP or other nucleotide triphosphates are not present in the incubation mixture with the DNA template. Also, the enzymes herein have a broader pH profile than that of the thermostable enzyme from Thermus aquaticus described in the literature, with more than 50% of the activity at pH 7 as at pH 8. In addition, the thermostable enzyme herein can be stored in a buffer with non-ionic detergents so that it is stable, not losing activity over a period of time.

The present invention resides in a process for amplifying one or more specific nucleic acid sequences present in a nucleic acid or mixture thereof using primers and the thermostable enzyme of the present invention. The extension product of one primer when hybridized to the other becomes a template for the production of the desired specific nucleic acid sequence, and vice versa, and the process is repeated as often as is necessary to produce the desired amount of the sequence. The method herein improves the specificity of the amplification reaction, resulting in a very distinct signal of amplified nucleic acid. In addition, the method herein eliminates the need for transferring reagents from one vessel to another after each amplification cycle. Such transferring is not required because the thermostable enzyme will withstand the high temperatures required to denature the nucleic acid strands and therefore does not need replacement. The temperature cycling may, in addition, be automated for further reduction in manpower and steps required to effectuate the amplification reaction.

More specifically, the present invention provides a process for amplifying at least one specific nucleic acid sequence contained in a nucleic acid or a mixture of nucleic acids, wherein if the nucleic acid is double-stranded, it consists of two separated complementary strands of equal or unequal length, which process comprises:
(a) contacting each nucleic acid strand with four different nucleotide triphosphates and one oligonucleotide primer for each different specific sequence being amplified, wherein each primer is selected to be substantially complementary to different strands of each specific sequence, such that the extension product synthesized from one primer, when it is separated from its complement, can serve as a template for synthesis of the extension product of the other primer, said contacting being at a temperature which promotes hybridization of each primer to its complementary nucleic acid strand;
(b) contacting each nucleic acid strand, at the same time as or after step (a), with a thermostable enzyme of the present invention which enables combination of the nucleotide triphosphates to form primer extension products complementary to each strand of each nucleic acid;
(c) maintaining the mixture from step (b) at an effective temperature for an effective time to activate the enzyme, and to synthesize, for each different sequence being amplified, an extension product of each primer which is complementary to each nucleic acid strand template, but not so high (a temperature) asto separate each extension product from its complementary strand template;
(d) heating the mixture from step (c) for an effective time and at an effective temperature to separate the primer extension products from the templates on which they were synthesized to produce single-stranded molecules, but not so high (a temperature) as to denature irreversibly the enzyme;
(e) cooling the mixture from step (d) at an effective temperature for an effective time to promote hybridization of each primer to each of the single-stranded molecules produced in step (d); and
(f) maintaining the mixture from step (e) at an effective temperature for an effective time to promote the activity of the enzyme and to synthesize, for each different sequence being amplified, an extension product of each primer which is complementary to each nucleic acid strand template produced in step (d), but not so high (a temperature) as to separate each extension product from its complementary strand template, wherein steps (e) and (f) are carried out simultaneously or sequentially.
   The steps (d), (e) and (f) may be repeated until the desired level of sequence amplification is obtained. The preferred thermostable enzyme herein is a polymerase extracted from Thermus aquaticus (Taq polymerase). Most preferably, If the enzyme is Taq polymerase, in step (a) the nucleic acid strands are contacted with a buffer comprising about 1.5-2 mM of a magnesium salt, 150-200 µM each of the nucleotides, and 1 µM of each primer, steps (a), (e) and (f) are carried out at about 45-58°C, and step (d) is carried out at about 90-100°C.
   In a preferred embodiment, the nucleic acid(s) are double-stranded and step (a) is accomplished by (i) heating each nucleic acid in the presence of four different nucleotide triphosphates and one oligonucleotide primer for each different specific sequence being amplified, for an effective time and at an effective temperature to denature each nucleic acid, wherein each primer is selected to be substantially complementary to different strands of each specific sequence, such that the extension product synthesized from one primer, when it is separated from its complement, can serve as a template for synthesis of the extension product of the other primer; and (ii) cooling the denatured nucleic acids to a temperature which promotes hybridization of each primer to its complementary nucleic acid strand.
   In other embodiments the invention relates to a process for detecting the presence or absence of at least one specific nucleic acid sequence in a sample containing a nucleic acid or mixture of nucleic acids, or distinguishing between two different sequences in said sample, wherein the sample is suspected of containing said sequence or sequences, and wherein if the nucleic acid(s) are double-stranded, they each consist of two separated complementary strands of equal or unequal length, which process comprises steps (a) to (f) mentioned above, resulting In amplification in quantity of the specific nucleic acid sequence(s), if present;
(g) adding to the product of step (f) a labeled oligonucleotide probe, for each sequence being detected, capable of hybridizing to said sequence or to a mutation thereof; and
(h) determining whether said hybridization has occurred.
   In yet another embodiment, the invention relates to a process for detecting the presence or absence of at least one nucleotide variation in sequence in one or more nucleic acids contained in a sample, wherein if the nucleic acid is double-stranded it consists of two separated complementary strands of equal or unequal length, which process comprises steps (a)-(f) mentioned above, wherein steps (d), (e) and (f) are repeated a sufficient number of times to result in detectable amplification of the nucleic acid containing the sequence, if present;
(g) affixing the product of step (f) to a membrane;
(h) treating the membrane under hybridization conditions with a labeled sequence-specific oligonucleotide probe capable of hybridizing with the amplified nucleic acid sequence only if a sequence of the probe is complementary to a region of the amplified sequence; and
(i) detecting whether the probe has hybridized to an amplified sequence in the nucleic acid sample.
   If the sample comprises cells, preferably they are heated before step (a) to expose the nucleic acids therein to the reagents. This step avoids extraction of the nucleic acids prior to reagent addition.
   In a variation of this process, the primer(s) and/or nucleotide triphosphates are labeled so that the resulting amplified sequence is labeled. The labeled primer(s) and/or nucleotide triphosphate(s) can be present in the reaction mixture initially or added during a later cycle. The sequence-specific oligonucleotide (unlabeled) is affixed to a membrane and treated under hybridization conditions with the labeled amplification product so that hybridization will occur only if the membrane-bound sequence is present in the amplification product.
   In yet another embodiment, the invention herein relates to a process for cloning into a cloning vector one or more specific nucleic acid sequences contained in a nucleic acid or a mixture of nucleic acids, which nucleic acid (s) when double-stranded consist of two separated complementary strands, and which nucleic acid(s) are amplified in quantity before cloning, which process comprises steps (a)-(f) mentioned above, with steps (d), (e) and (f) being repeated a sufficient number of times to result in detectable amplification of the nucleic acid(s) containing the sequence(s);
(g) adding to the product of step (f) a restriction enzyme for each of said restriction sites to obtain cleaved products in a restriction digest; and
(h) ligating the cleaved product(s) of step (g) containing the specific sequence(s) to be cloned into one or more cloning vectors containing a promoter and a selectable marker.

In a further embodiment, the invention herein relates to a process for cloning into a cloning vector one or more specific nucleic acid sequences contained in a nucleic acid or mixture of nucleic acids, which nucleic acid(s), when double-stranded, consist of two separated complementary strands of equal or unequal length which nucleic acid(s) are amplified in quantity before cloning, which process comprises steps (a)-(f) mentioned above, with steps (d), (e) and (f) being repeated a sufficient number of times to result in effective amplification of the nucleic acid(s) containing the sequence(s) for blunt-end ligation into one or more cloning vectors; and

(g) ligating the amplified specific sequence(s) to be cloned obtained from step (f) into one or more of said cloning vectors in the presence of a ligase, said amplified sequence(s) and vector(s) being present in sufficient amounts to effect the ligation.

In a product embodiment, the invention provides a composition of matter useful in amplifying at least one specific nucleic acid sequence contained in a nucleic acid or a mixture of nucleic acids, comprising four different nucleotide triphosphates and one oligonucleotide primer for each different specific sequence being amplified, wherein each primer is selected to be substantially complementary to different strands of each specific sequence, such that the extension product synthesized from one primer, when it is separated from its complement, can serve as a template for synthesis of the extension product of the other primer.

In another product embodiment, the invention provides a sample of one or more nucleic acids comprising multiple strands of a specific nucleic acid sequence contained in the nucleic acid(s). The sample may comprise about 10-100 of the strands, about 100-1000 of the strands, or over about 1000 of the strands.

In a further product embodiment, the invention provides an amplified nucleic acid sequence from a nucleic acid or mixture of nucleic acids comprising multiple copies of the sequence produced by the amplification processes herein.

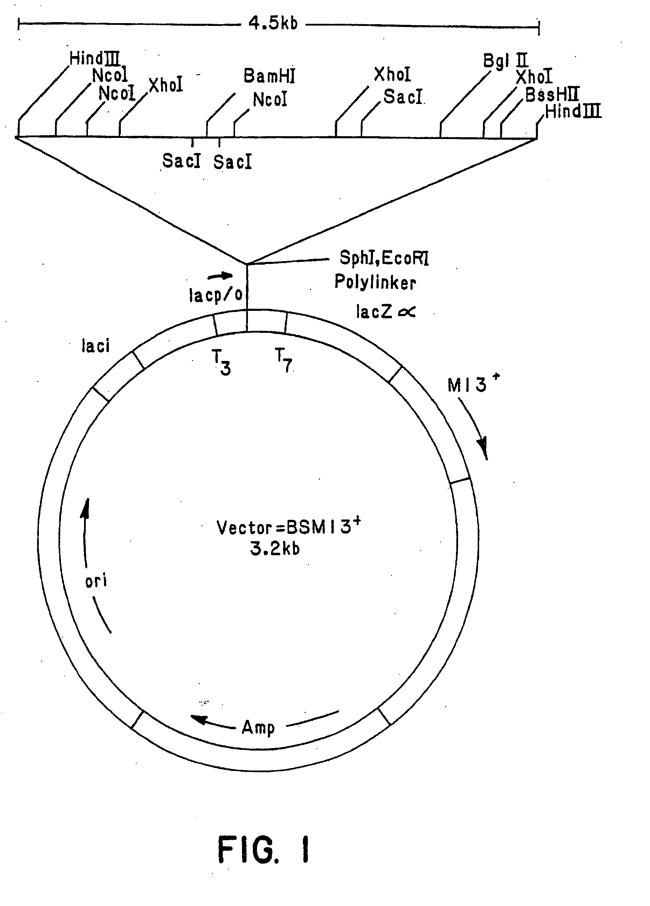

Figure 1 is a restriction site map of plasmid pFC83 that contains the ∼4.5 kb HindIII T. aquaticus DNA insert subcloned into plasmid BSM13+.

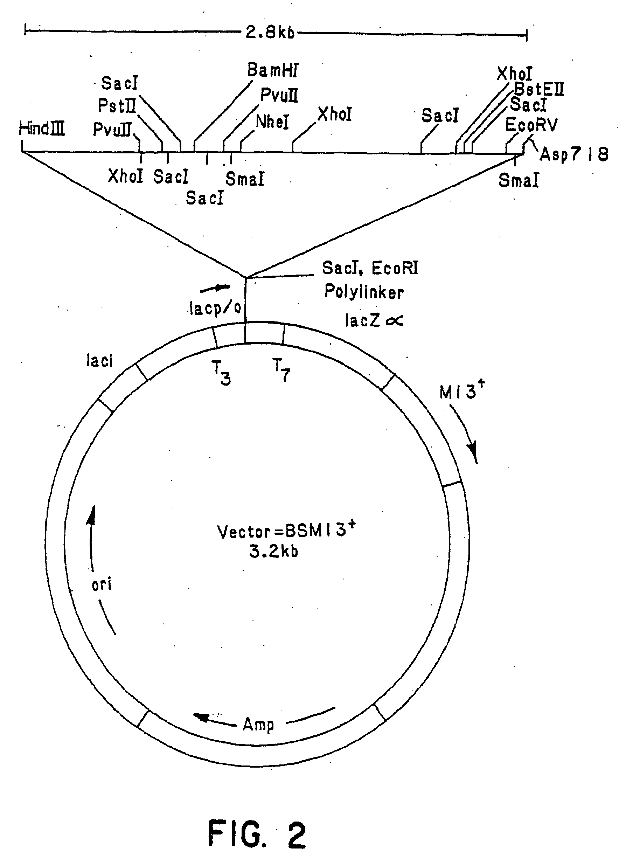

Figure 2 is a restriction site map of plasmid pFC85 that contains the ∼2.8 kb HindIII to Asp71B T. aquaticus DNA insert subcloned into plasmid BSM13+.

As used herein, "cell", "cell line", and "cell culture" can be used interchangeably and all such designations include progeny. Thus, the words "transformants" or "transformed cells" includes the primary subject cell and cultures derived therefrom without regard for the number of transfers. It is also understood that all progeny may not be precisely identical in DNA content, due to deliberate or inadvertent mutations. Mutant progeny that have the same functionality as screened for in the originally transformed cell are included.

The term "control sequences' refers to DNA sequences necessary for the expression of an operably linked coding sequence in a particular host organism. The control sequences that are suitable for procaryotes, for example, include a promoter, optionally an operator sequence, a ribosome binding site, and possibly, other as yet poorly understood sequences. Eucaryotic cells are known to utilize promoters, polyadenylation signals, and enhancers.

The term "expression system" refers to DNA sequences containing a desired coding sequence and control sequences in operable linkage, so that hosts transformed with these sequences are capable of producing the encoded proteins. In order to effect transformation, the expression system may be included on a vector, however, the relevant DNA may then also be integrated into the host chromosome.

The term "gene" as used herein refers to a DNA sequence that encodes a recoverable bioactive polypeptide or precursor. The polypeptide can be encoded by a full-length gene sequence or any portion of the coding sequence so long as the enzymatic activity is retained.

"Operably linked" refers to juxtaposition such that the normal function of the components can be performed. Thus, a coding sequence "operably linked" to control sequences refers to a configuration wherein the coding sequences can be expressed under the control of the sequences.

"Non-ionic polymeric detergents" refers to surface-active agents that have no ionic charge and that are characterized, for purposes of this invention, by their ability to stabilize the enzyme herein at a pH range of from about 3.5 to about 9.5, preferably from 4 to 8.5.

The term "oligonucleotide" as used herein is defined as a molecule comprised of two or more deoxyribonucleotides or ribonucleotides, preferably more than three. Its exact size will depend on many factors, which in turn depend on the ultimate function or use of the oligonucleotide. The oligonucleotide may be derived synthetically or by cloning.

The term "primer" as used herein refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, i.e., in the presence of four different nucleotide triphosphates and thermostable enzyme in an appropriate buffer ("buffer" includes pH, ionic strength, cofactors, etc.) and at a suitable temperature. For Taq polymerase the buffer herein preferably contains 1.5-2 mM of a magnesium salt, preferably MgCl₂, 150-200 µM of each nucleotide, and 1 µM of each primer, along with preferably 50 mM KCI, 10 mM Tris buffer, pH 8-8.4, and 100 µg/ml gelatin.

The primer is preferably single-stranded for maximum efficiency in amplification, but may alternatively be double-stranded. If double-stranded, the primer is first treated to separate its strands before being used to prepare extension products. Preferably, the primer is an oligodeoxyribonucleotide. The primer must be sufficiently long to prime the synthesis of extension products in the presence of the thermostable enzyme. The exact lengths of the primers will depend on many factors, including temperature, source of primer and use of the method. For example, depending on the complexity of the target sequence, the oligonucleotide primer typically contains 15-25 nucleotides, although it may contain more or fewer nucleotides. Short primer molecules generally require cooler temperatures to form sufficiently stable hybrid complexes with template.

The primers herein are selected to be "substantially" complementary to the different strands of each specific sequence to be amplified. This means that the primers must be sufficiently complementary to hybridize with their respective strands. Therefore, the primer sequence need not reflect the exact sequence of the template. For example, a non-complementary nucleotide fragment may be attached to the 5' end of the primer, with the remainder of the primer sequence being complementary to the strand. Alternatively, non-complementary bases or longer sequences can be interspersed into the primer, provided that the primer sequence has sufficient complementarity with the sequence of the strand to be amplified to hybridize therewith and thereby form a template for synthesis of the extension product of the other primer. However, for detection purposes, particulary using labeled sequence-specific probes, the primers typically have exact complementarity to obtain the best results.

As used herein, the terms "restriction endonucleases" and "restriction enzymes" refer to bacterial enzymes each of which cut double-stranded DNA at or near a specific nucteotide sequence.

As used herein, the term "DNA polymorphism" refers to the condition in which two or more different nucleotide sequences can exist at a particular site in DNA.

As used herein, the term "nucleotide variation in sequence'' refers to any single or multiple nucleotide substitutions, deletions or insertions. These nucleotide variations may be mutant or polymorphic allele variations. Therefore, the process herein can detect single nucleotide changes in nucleic acids such as occur in β-globin genetic diseases caused by single-base mutations, additions or deletions (some β-thalassemias, slckle cell anemia, hemoglobin C disease, etc.), as well as multiple-base variations such as are involved with α-thalassemia or some β-thalassemias. In addition, the process herein can detect polymorphlsms, which are not necessarily associated with a disease, but are merely a condition in which two or more different nucleotide sequences (whether having substituted, deleted or inserted nucleotide base pairs) can exist at a particular site in the nucleic acid in the population, as with HLA regions of the human genome and random polymorphisms such as mitochondrial DNA. The polymorphic sequence-speciflc oligonucleotide probes described in detail hereinafter may be used to detect genetic markers linked to a disease such as insulin-dependent diabetis mellitus or in forensic applications. If the nucleic acid is double-stranded, the nucleotide variation in sequence becomes a base pair variation in sequence.

The term "sequence-specific oligonucleotides" refers to oligonucleotides which will hybridize to specific sequences whether or not contained on alleles, which sequences span the base pair variation being detected and are specific for the sequence variation being detected. Depending on the sequences being analyzed, one or, more sequence-specific oligonucleotides may be employed for each sequence, as described further hereinbelow.

As used heroin, the the term "restriction fragment length polymorphism" ("RFLP") refers to the differences among individuals in the lengths of restriction fragments formed by digestion with a particular restriction endonuclease.

As used herein, the term "thermostable enzyme" refers to an enzyme which is stable to heat and is heat resistant and catalyzes (facilitates) combination of the nucleotides in the proper manner to form the primer extension products that are complementary to each nucleic acid strand. Generally, the synthesis will be initiated at the 3' end of each primer and will proceed in the 5' direction along the template strand, until synthesis terminates, producing molecules of different lengths. There may be athermostable enzyme, however, which initiates synthesis at the 5' end and proceeds in the other direction, using the same process as described above.

The thermostable enzyme herein must satisfy a single criterion to be effective for the amplification reaction, I.e., the enzyme must not become irreversibly denatured (inactivated) when subjected to the elevated temperatures for the time necessary to effect denaturation of double-stranded nucleic acids. Irreversible denaturation for purposes herein refers to permanent and complete loss of enzymatic activity. The heating conditions necessary for denaturation will depend, e.g., on the buffer salt concentration and the length and nucleotide composition of the nucleic acids being denatured, but typically range from about 90 to about 105°C for a time depending mainly on the temperature and the nucleic acid length, typically about 0.5 to four minutes. Higher temperatures may be tolerated as the buffer salt concentration and/or GC composition of the nucleic acid is increased. Preferably, the enzyme will not become irreversibly denatured at about 90-100°C.

The thermostable enzyme herein preferably has an optimum temperature at which it functions that is higher than about 40°C, which is the temperature below which hybridization of primer to template is promoted, although, depending on (1) magnesium and salt concentrations and (2) composition and length of primer, hybridization can occur at higher temperature (e.g., 45-70°C). The higher the temperature optimum for the enzyme, the greater the specificity and/or selectivity of the primer-directed extension process. However, enzymes that are active below 40°C, e.g., at 37°C, are also within the scope of this invention provided they are heat-stable. Preferably, the optimum temperature ranges from about 50 to 90°C, more preferably 60-80°C.

The thermostable enzyme herein may be obtained from any source and may be a native or recombinant protein.

The thermostable enzyme herein is a DNA polymerase isolated from Thermus aquaticus. Various strains thereof are available from the American Type Culture Collection, Rockville, Maryland, and is described by T.D. Brock, J. Bact. (1969) 98:289-297, and by T. Oshima, Arch. Microbiol. (1978) 117: 189-196. One of these preferred strains is strain YT-1.

For recovering the native protein the cells are grown using any suitable technique. One such technique is described by Kaledin et al., Biokhimiya (1980), supra. Briefly, the cells are grown on a medium, in one liter, of nitrilotriacetic acid (100 mg), tryptone (3 g), yeast extract (3 g), succinic acid (5 g), sodium sulfite (50 mg), riboflavin (1 mg), K₂HPO₄ (522 mg), MgSO₄(480 mg), CaCl₂ (222 mg), NaCl (20 mg), and trace elements. The pH of the medium is adjusted to 8.0 ± 0.2 with KOH. The yield is increased if cultivated with vigorous aeration up to 20 g/liter of cells at a temperature of 70°C. Cells in the late logarithmic stage (determined by absorbance at 550 nm) are collected by centrifugation, washed with a buffer and stored frozen at -20°C.

In another method for growing the cells, described in Chien et al., J. Bacteriol. (1976), supra, a defined mineral salts medium containing 0.3% glutamic acid supplemented with 0.1 mg/l biotin, 0.1 mg/l thiamine, and 0.05 mg/l nicotinic acid is employed. The salts include nitrilotriacetic acid, CaSO₄, MgSO₄, NaCl, KNO₃, NaNO₃, ZnSO₄, H₃BO₃, CuSO₄, NaMoO₄, CoCl₂, FeCl₃, MnSOg₄, and Na₂HPO₄. The pH of the medium is adjusted to 8.0 with NaOH.

In the Chien et al. technique, the cells are grown initially at 75°C in a water bath shaker. On reaching a certain density,1 1 liter of these cells is transferred to 16-liter carboys which are placed in hot-air incubators. Sterile air is bubbled through the cultures and the temperature maintained at 75°C. The cells are allowed to grow for 20 hours before being collected by centrifuge.

After cell growth, the isolation and purification of the enzyme take place in six stages, each of which is carried out at a temperature below room temperature, preferably about 4°C.

In the first stage or step, the cells, if frozen, are thawed, disintegrated by ultrasound, suspended in a buffer at about pH 7.5, and centrifuged.

In the second stage, the supernatant is collected and then fractionated by adding a salt such as dry ammonium sulfate. The appropriate fraction (typically 45-75% of saturation) is collected, dissolved in a 0.2 M potassium phosphate buffer preferably at pH 6.5, and dialyzed against the same buffer.

The third step removes nucleic acids and some protein. The fraction from the second stage is applied to a DEAE-cellulose column equilibrated with the same buffer as used above. Then the column is washed with the same buffer and the flow-through protein-containing fractions, determined by absorbance at 280 nm, are collected and dialyzed against a 10 mM potassium phosphate buffer, preferably with the same ingredients as the first buffer, but at a pH of 7.5.

In the fourth step, the fraction so collected is applied to a hydroxyapatite column equilibrated with the buffer used for dialysis in the third step. The column is then washed and the enzyme eluted with a linear gradient of a buffer such as 0.01 M to 0.5 M potassium phosphate buffer at pH 7.5 containing 10 mM 2-mercaptoethanol and 5% glycerine. The pooled fractions containing thermostable enzyme (e.g., DNA polymerase) activity are dialyzed against the same buffer used for dialysis in the third step.

In the fifth stage, the dialyzed fraction is applied to a DEAE-cellulose column, equilibrated with the buffer used for dialysis in the third step. The column is then washed and the enzyme eluted with a linear gradient of a buffer such as 0.01 to 0.6 M KCl in the buffer used for dialysis in the third step. Fractions with thermostable enzyme activity are then tested for contaminating deoxyribonucleases (endo- and exonucleases) using any suitable procedure. For example, the endonuclease activity may be determined electrophoretically from the change in molecular weight of phage λ DNA or supercoiled plasmid DNA after incubation with an excess of DNA polymerase. Similarly, exonuclease activity may be determined electrophoretically from the change in molecular weight of DNA after treatment with a restriction enzyme that cleaves at several sites.

The fractions determined to have no deoxyribonuclease activity are pooled and dialyzed against the same buffer used in the third step.

In the sixth step, the pooled fractions are placed on a phosphocellulose column with a set bed volume. The column is washed and the enzyme eluted with a linear gradient of a buffer such as 0.01 to 0.4 M KCl in a potassium phosphate buffer at pH 7.5. The pooled fractions having thermostable polymerase activity and no deoxyribonuclease activity are dialyzed against a butter at pH 8.0.

The molecular weight of the dialyzed product may be determined by any technique, for example, by SDS PAGE using protein molecular weight markers. The molecular weight of the DNA polymerase purified from Thermus aquaticus, is determined by the above method to be about 86,000-90,000 daltons determined as stated in claim 1.

The thermostable enzyme of this invention may also be produced by recombinant DNA techniques, as the gene encoding this enzyme has been cloned from Thermus aquaticus genomic DNA. The complete coding sequence for the Thermus aquaticus (Taq) polymerase can be derived from bacteriophage CH35:Taq#4-2 on an approximately 3.5 kilobase (kb) BgIII-Asp718 (partial) restriction fragment contained within an ∼18 kb genomic DNA insert fragment. This bacteriophage was deposited with the American Type Culture Collection (ATCC) on May 29,1987 and has accession no. 40,336. Alternatively, the gene can be constructed by ligating an ∼750 base pair (bp) BgIII-HindIII restriction fragment isolated from plasmid pFC83 (ATCC 67,422 deposited May 29, 1987) to an ∼2.8 kb HindIII-Asp718 restriction fragment isolated from plasmid pFC85 (ATCC 67,421 deposited May 29, 1987). The pFC83 restriction fragment comprises the amino-terminus of the Taq polymerase gene while the restriction fragment from pFC85 comprises the carboxyl-terminus. Thus, ligation of these two fragments into a correspondingly digested vector with appropriate control sequences will result in the translation of a full-length Taq polymerase.

It has also been found that the entire coding sequence of the Taq polymerase gene is not required to recover a biologically active gene product with the desired enzymatic activity. Amino-terminal deletions wherein approximately one-third of the coding sequence is absent have resulted in producing a gene product that is quite active in polymerase assays.

In addition to the N-terminal deletions, individual amino acid residues in the peptide chain comprising Taq polymerase may be modified by oxidation, reduction, or other derivatization, and the protein may be cleaved to obtain fragments that retain activity. Such alterations that do not destroy activity do not remove the DNA sequence encoding such protein from the definition of gene.

Thus, modifications to the primary structure itself by deletion, addition, or alteration of the amino acids incorporated into the sequence during translation can be made without destroying the activity of the protein. Such substitutions or other alterations result in proteins having an amino acid sequence encoded by DNA falling within the contemplated scope of the present invention.

Polyclonal antiserum from rabbits immunized with the purified 86,000-90,000 dalton polymerase of this invention was used to probe a Thermus aquaticus partial genomic expression library to obtain the appropriate coding sequence as described below. The cloned genomic sequence can be expressed as a fusion polypeptide, expressed directly using its own control sequences, or expressed by constructions using control sequences appropriate to the particular host used for expression of the enzyme.

Of course, the availability of DNA encoding these sequences provides the opportunity to modify the codon sequence so as to generate mutein forms also having DNA polymerase activity.

Thus, these tools can provide the complete coding sequence for Taq DNA polymerase from which expression vectors applicable to a variety of host systems can be constructed and the coding sequence expressed. It is also evident from the foregoing that portions of the Taq polymerase-encoding sequence are useful as probes to retrieve other thermostable polymerase-encoding sequences in a variety of species. Accordingly, portions of the genomic DNA encoding at least six amino acids can be replicated in E. coli and the denatured forms used as probes or oligodeoxyribonucleotide probes can be synthesized which encode at least six amino acids and used to retrieve additional DNAs encoding a thermostable polymerase. Because there may not be a precisely exact match between the nucleotide sequence in the Thermus aquaticus form and that in the corresponding portion of other species, oligomers containing approximately 18 nucleotides (encoding the six amino acid stretch) are probably necessary to obtain hybridization under conditions of sufficient stringency to eliminate false positives. The sequences encoding six amino acids would supply information sufficient for such probes.

### Suitable Hosts, Control Systems and Methods

In general terms, the production of a recombinant form of Taq polymerase typically involves the following:

First, a DNA is obtained that encodes the mature (used here to include all muteins) enzyme or a fusion of the Taq polymerase to an additional sequence that does not destroy its activity or to an additional sequence cleavable under controlled conditions (such as treatment with peptidase) to give an active protein. If the sequence is uninterrupted by introns it is suitable for expression in any host. This sequence should be in an excisable and recoverable form.

The excised or recovered coding sequence is then preferably placed in operable linkage with suitable control sequences in a replicable expression vector. The vector is used to transform a suitable host and the transformed host cultured under favorable conditions to effect the production of the recombinant Taq polymerase. Optionally the Taq polymerase is isolated from the medium or from the cells; recovery and purification of the protein may not be necessary in some instances, where some impurities may be tolerated.

Each of the foregoing steps can be done in a variety of ways. For example, the desired coding sequences may be obtained from genomic fragments and used directly in appropriate hosts. The constructions for expression vectors operable in a variety of hosts are made using appropriate replicons and control sequences, as set forth below Suitable restriction sites can, if not normally available, be added to the ends of the coding sequence so as to provide an excisable gene to insert into these vectors.

The control sequences, expression vectors, and transformation methods are dependent on the type of host cell used to express the gene. Generally, procaryotic, yeast; insect or mammalian calls are presently useful as hosts. Procaryotic hosts are in general the most efficient and convenient for the production of recombinant proteins and therefore preferred for the expression of Taq polymerase.

In the particular case of Taq polymerase, evidence indicates that considerable deletion at the N-terminus of the protein may occur under both recombinant and native conditions, and that the activity of the protein is still retained. It appears that the native proteins isolated may be the result of proteolytic degradation, and not translation of a truncated gene. The mutein produced from the truncated gene of plasmid pFC95 is, however, fully active in assays for DNA polymerase, as is that produced from DNA encoding the full-length sequence. Since it is clear that certain N-terminal shortened forms are active, the gene constructs used for expression of the polymerase may also include the corresponding shortened forms of the coding sequence.

### Control Sequences and Corresponding Hosts

Procaryotes most frequently are represented by various strains of E. coli. However, other microbial strains may also be used, such as bacilli, for example, Bacillus subtilis, various species of Pseudomonas, or other bacterial strains. In such procaryotic systems, plasmid vectors that contain replication sites and control sequences derived from a species compatible with the host are used. For example, E. coli is typically transformed using derivatives of pBR322, a plasmid derived from an E. coli species by Bolivar, et al., Gene (1977) 2:95. pBR322 contains genes for ampicillin and tetracycline resistance, and thus provides additional markers that can be either retained or destroyed in constructing the desired vector. Commonly used procaryotic control sequences, which are defined herein to include promoters for transcription initiation, optionally with an operator, along with ribosome binding site sequences, include such commonly used promoters as the β-lactamase (penicillinase) and lactose (lac) promoter systems (Chang, et al., Nature (1977) 198:1056), the tryptophan (trp) promoter system (Goeddel, et al., Nucleic Acids Res. (1980) 8:4057) and the lambda-derived P_{L} promoter (Shimatake, et al., Nature (1981) 292:128) and N-gene ribosome binding site, which has been made useful as a portable control cassette, which comprises a first DNA sequence that is the P_{L} promoter operably linked to a second DNA sequence corresponding to N_{RBS} upstream of a third DNA sequence having at least one restriction site that permits cleavage within six bp 3' of the N_{RBS} sequence. Also useful is the phosphatase A (phoA) system described by Chang, et al, in European Patent Publication No. 196,864 published October 8, 1986, assigned to the same assignee. However, any available promoter system compatible with procaryotes can be used.

In addition to bacteria, eucaryotic microbes, such as yeast, may also be used as hosts. Laboratory strains of Saccharomyces cerevisiae, Baker's yeast, are most used, although a number of other strains are commonly available. While vectors employing the 2 micron origin of replication are illustrated (Broach, J. R., Meth. Enz. (1983) 101:307), other plasmid vectors suitable for yeast expression are known (see, for example, Stinchcomb, et al., Nature (1979) 282:39, Tschempe, et al., Gene (1980) 10:157 and Clarke, L., et al., Meth. Enz. (1983) 101:300). Control sequences for yeast vectors include promoters for the synthesis of glycolytic enzymes (Hess, et al., J. Adv. Enzyme Reg. (1968) 7:149; Holland, et al., Biotechnology (1978) 17:4900).

Additional promoters known in the art include the promoter for 3-phosphoglycerate kinase (Hitzeman, et al., J. Biol. Chem. (1980) 255:2073), and those for other glycolytic enzymes, such as glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other promoters that have the additional advantage of transcription controlled by growth conditions are the promoter regions for alcohol dehydrogenase 2, isocytochroma C, acid phosphatase, degradative enzymes associated with nitrogen metabolism, and enzymes responsible for maltose and galactose ultilization (Holland, supra).

It is also believed that terminator sequences are desirable at the 3' end of the coding sequences. Such terminators are found in the 3' untranslated region following the coding sequences in yeast-derived genes. Many of the vectors illustrated contain control sequences derived from the enolase gene containing plasmid peno46 (Holland, M. J., et al., J. Biol. Chem. (1981) 256:1385) or the LEU2 gene obtained from YEp13 (Broach, J., et al., Gene (1978) 8:121); however, any vector containing a yeast-compatible promoter, origin of replication, and other control sequences is suitable.

It is also, of course, possible to express genes encoding polypeptides in eucaryotic host cell cultures derived from multicellular organisms. See, for example, Tissue Culture, Academic Press, Cruz and Patterson, editors (1973). Useful host cell lines include murine myelomas N51, VERO and HeLa cells, and Chinese hamster ovary (CHO) cells. Expression vectors for such cells ordinarily include promoters and control sequences compatible with mammalian calls such as, for example, the commonly used early and late promoters from Simian Virus 40 (SV 40) (Fiers, et al., Nature (1978) 273:113), or other viral promoters such as those derived from polyoma, Adenovirus 2, bovine papiloma virus, or avian sarcoma viruses, or immunoglobulin promoters and heat shock promoters. A system for expressing DNA in mammalian systems using the BPV as a vector is disclosed in U.S. Patent 4,419,446. A modification of this system is described in U.S. Patent 4,601,978. General aspects of mammalian cell host system transformations have been described by Axel, U.S. Patent No. 4,399,216. It now appears, also, that "enhancer" regions are important in optimizing expression; these are, generally, sequences found upstream of the promoter region. Origins of replication may be obtained, if needed, from viral sources. However, integration into the chromosome is a common mechanism for DNA replication in eucaryotes.

Plant cells are also now available as hosts, and control sequences compatible with plant cells such as the nopaline synthase promoter and polyadenylation signal sequences (Depicker, A., et al., J. Mol. Appl. Gen. (1982) 1:561) are available.

Recently, in addition, expression systems employing insect cells utilizing the control systems provided by baculovirus vectors have been described (Miller, D. W., et al., in Genetic Engineering (1986) Setlow, J. K. et al., eds., Plenum Publishing, Vol. 8, pp. 277-297). These systems are also successful in producing Taq polymerase.

### Transformations

Depending on the host cell used, transformation is done using standard techniques appropriate to such cells. The calcium treatment employing calcium chloride, as described by Cohen, S. N., Proc. Natl. Acad. Scl. (USA) (1972) 69: 2110 is used for procaryotes or other cells that contain substantial cell wall barriers. Infection with Agrobacterium tumefaciens (Shaw, C. H., et al., Gene (1983)23:315) is used for certain plant cells. For mammalian cells without such cell walls, the calcium phosphate precipitation method of Graham and van der Eb, Virology (1978)52:546 is preferred. Transformations into yeast are carried out according to the method of Van Solingen, P., et al., J. Bact. (1977)130:946 and Hsiao, C. L, et al., Proc. Natl. Acad. Sci. (USA) (1979) 76:3829.

### Construction of a λgt11 Expression Library

The strategy for isolating DNA encoding desired proteins, such as the Taq polymerase encoding DNA, using the bacteriophage vector lambda gt11, is as follows. A library can be constructed of EcoRI-flanked AluI fragments, generated by complete digestion of Thermus aquaticus DNA, inserted at the EcoRI site in the lambda gtII phage (Young and Davis, Proc. Natl. Acad. Sci USA (1983)80:1194-1198). Because the unique EcoRI site in this bacteriophage is located in the carboxyl-terminus of the 3-galactosidase gene, inserted DNA (in the appropriate frame and orientation) is expressed as protein fused with β-galactosidase under the control of the lactose operon promoter/operator.

Genomic expression libraries are then screened using the antibody plaque hybridization procedure. A modification of this procedure, referred to as "epitope selection,' uses antiserum against the fusion protein sequence encoded by the phage, to confirm the identification of hybridized plaques. Thus, this library of recombinant phages could be screened with antibodies that recognize the 86,000-90,000 dalton Taq polymerase in order to identify phage that carry DNA segments encoding the antigenic determinants of this protein.

Approximately 2 x 10⁵ recombinant phage are screened using total rabbit Taq polymerase antiserum. In this p rimary screen, positive signals are detected and one or more of these plaques are purified from candidate plaques which failed to react with preimmune serum and reacted with immune serum and analyzed in some detail. To examine the fusion proteins produced by the recombinant phage, lysogens of the phage in the host Y1089 are produced. Upon induction of the lysogens and gel electrophoresis of the resulting proteins, each lysogen may be observed to produce a new protein, not found in the other lysogens, or duplicate sequences may result. Phage containing positive signals are picked; in this case, one positive plaque was picked for further identification and replated at lower densities to purify recombinants and the purified clones were analyzed by size class via digestion with EcoRI restriction enzyme. Probes can then be made of the isolated DNA insert sequences and labeled appropriately and these probes can be used in conventional colony or plaque hybridization assays described in Maniatis et al., Molecular Cloning: A Laboratory Manual (1982).

The labeled probe was used to probe a second genomic library constructed in a Charon 35 bacteriophage (Wilhelmine, A. M. et al., Gene (1983) 26:171-179). This library was made from Sau3A partial digestions of genomic Thermus aquaticus DNA and size fractionated fragments (15-20 kb) were cloned into the BamHI site of the Charon 35 phage. The probe was used to isolate phage containing DNA encoding the Taq polymerase. One of the resulting phage, designated CH35:Taq#4-2, was found to contain the entire gene sequence. Partial sequences encoding portions of the protein were also isolated.

### Vector Construction

Construction of suitable vectors containing the desired coding and control sequences employs standard ligation and restriction techniques that are well understood in the art. Isolated plasmids, DNA sequences, or synthesized oligonucleotides are cleaved, tailored, and religated in the form desired.

Site-specific DNA cleavage is performed by treating with the suitable restriction enzyme (or enzymes) under conditions that are generally understood in the art, and the particulars of which are specified by the manufacturer of these commercially available restriction enzymes. See, e.g., New England Biolabs, Product Catalog. In general, about 1 µg of plasmid or DNA sequence is cleaved by one unit of enzyme in about 20 µl of buffer solution; in the examples herein, typically an excess of restriction enzyme is used to ensure complete digestion of the DNA substrate. Incubation times of about one hourto two hours at about 37°C are workable, although variations can be tolerated. After each incubation, protein is removed by extraction with phenol/chloroform, and may be followed by ether extraction, and the nucleic acid recovered from aqueous fractions by precipitation with ethanol. If desired, size separation of the cleaved fragments may be performed by polyacrylamide gel or agarose gel electrophoresis using standard techniques. A general description of size separations is found in Methods in Enzymology (1980) 65:499-560.

Restriction-cleaved fragments may be blunt-ended by treating with the large fragment of E. coli DNA polymerase I (Klenow) in the presence of the four deoxynucleotide triphosphates (dNTPs) using incubation times of about 15 to 25 minutes at 20 to 25°C in 50 mM Tris pH 7.6, 50 mM NaCl, 10 mM MgCl₂, 10 mM DTT and 50-100 µM dNTPs. The Klenow fragment fills in at 5' sticky ends, but chews back protruding 3' single strands, even though the four dNTPs are present. If desired, selective repair can be performed by supplying only one of the, or selected, dNTPs within the limitations dictated by the nature of the sticky ends. After treatment with Klenow, the mixture is extracted with phenol/chloroform and ethanol precipitated. Treatment under appropriate conditions with S1 nuclease results in hydrolysis of any single-stranded portion.

Synthetic oligonucleotides may be prepared using the triester method of Matteucci, et al., (J. Am. Chem. Soc. (1981) 103:3185-3191) or using automated synthesis methods. Kinasing of single strands prior to annealing or for labeling is achieved using an excess, e.g., approximately 10 units of polynucleotide kinase to 1 nM substrate in the presence of 50 mM Tris, pH 7.6, 10 mM MgCl₂, 5 mM dithiothreitol, 1-2 mM ATP. If kinasing is for labeling of probe, the ATP will contain high specific activity γ-³²P.

Ligations are performed in 15-30 µl volumes under the following standard conditions and temperatures: 20 mM Tris-Cl pH 7.5, 10 mM MgCl₂, 10 mM DTT, 33 µg/ml BSA, 10 mM-50 mM NaCl, and either 40 µM ATP, 0.01-0.02 (Weiss) units T4 DNA ligase at 0°C (for "sticky end'' ligation) or 1 mM ATP, 0.3-0.6 (Weiss) units T4 DNA ligase at 14°C (for "blunt end" ligation). Intermolecular "sticky end" ligations are usually performed at 33-100 µg/ml total DNA concentrations (5-100 nM total end concentration). Intermolecular blunt end ligations (usually employing a 10-30 fold molar excess of linkers) are performed at 1 µM total ends concentration.

In vector construction employing "vector fragments", the vector fragment is commonly treated with bacterial alkaline phosphatase (BAR) in order to remove the 5' phosphate and prevent religation of the vector. BAP digestions are conducted at pH 8 in approximately 150 mM Tris, in the presence of Na⁺ and Mg⁺² using about 1 unit of BAP per mg of vector at 60°C for about one hour. In order to recover the nucleic acid fragments, the preparation is extracted with phenol/chloroform and ethanol precipitated. Alternatively, religation can be prevented in vectors that have been double digested by additional restriction enzyme digestion of the unwanted fragments.

### Modification of DNA Sequences

For portions of vectors derived from cDNA or genomic DNA that require sequence modifications, site-specific primer-directed mutagenesis is used. This technique is now standard in the art, and is conducted using a primer synthetic oligonucleotide complementary to a single-stranded phage DNA to be mutagenized except for limited mismatching, representing the desired mutation. Briefly, the synthetic oligonucleotide is used as a primer to direct synthesis of a strand complementary to the phage, and the resulting double-stranded DNA is transformed into a phage-supporting host bacterium. Cultures of the transformed bacteria are plated in top agar, permitting plaque formation from single cells that harbor the phage.

Theoretically, 50% of the new plaques will contain the phage having, as a single strand, the mutated form; 50% will have the original sequence. The plaques are transferred to nitrocellulose filters and the "litts" hybridized with kinased synthetic primer at a temperature that permits hybridization of an exact match, but at which the mismatches with the original strand are sufficient to prevent hybridization. Plaques that hybridize with the probe are then picked and cultured, and the DNA is recovered.

### Verification of Construction

In the constructions set forth below, correct ligations for plasmid construction are confirmed by first transforming E. coli strain MM294, or other suitable host, with the ligation mixture. Successful transformants are selected by ampicillin, tetracycline or other antibiotic resistance or using other markers, depending on the mode of plasmid construction, as is understood in the art. Plasmids from the transformants are then prepared according to the method of Clewell, D. B., et al., Proc. Natl. Acad. Scl. (USA) (1969) 62:1159, optionally following chloramphenicol amplification (Clewell, D. B., J. Bacteriol. (1972) 110:667). The isolated DNA is analyzed by restriction and/or sequenced by the dideoxy method of Sanger, F., et al., Proc. Natl. Acad. Scl. (USA) (1977) 74:5463 as further described by Messing, et al., Nucleic Acids Res. (1981) 9:309, or by the method of Maxam, et al., Methods in Enzymology (1980) 65:499.

### Host Exemplified

Host strains used in cloning and expression herein are as follows:

For cloning and sequencing, and for expression of constructions under control of most bacterial promoters, E. coli strain MM294 obtained from E. coli Genetic Stock Center GCSC #6135, was used as the host. For expression under control of the P_{L}N_{RBS} promoter, E. coli strain K12 MC1000 lambda lysogen, N₇N₅₃clB57 SusP₈₀, ATCC 39531 may be used. Used herein is E. coli DG116, which was deposited with ATCC (ATCC 53606) on April 7, 1997.

For M13 phage recombinants, E. coli strains susceptible to phage infection, such as E. coli K12 strain DG98, are employed. The DG98 strain has been deposited with ATCC July 13, 1984 and has accession number 39768.

Mammalian expression can be accomplished in COS-7 COS-A2, CV-1, and murine cells, and insect cell based expression in Spodoptera frugipeida).

### Stabilization of Enzyme Activity

For long-term stability, the enzyme herein must be stored in a buffer that contains one or more non-ionic polymeric detergents. Such detergents are generally those that have a molecular weight in the range of approximately 100 to 250,000, preferably about 4,000 to 200,000 daltons and stabilize the enzyme at a pH of from about 3.5 to about 9.5 preferably from about 4 to 8.5 Examples of such detergents include those specified on pages 295-298 of McCutcheon's Emulsifiers & Detergents. North American edition (1983), published by the McCutcheon Division of MC Publishing Co., 175 Rock Road, Glen Rock, NJ (USA), the entire disclosure of which is incorporated herein by reference. Preferably, the detergents are selected from the group comprising ethoxylated fatty alcohol ethers and lauryl ethers, ethoxylated alkyl phenols, octylphenoxy polyethoxy ethanol compounds, modified oxyethylated and/or oxypropylated straight-chain alcohols, polyethylene glycol monooleate compounds, polysorbate compounds, and phenolic fatty alcohol ethers. More particularly preferred are Tween 20, from ICI Americas Inc., Wilmington. DE, which is a polyoxyethylated (20) sorbitan monolaurate, and lconol™ NP-40, from BASF Wyandotte Corp. Parsippany, NJ, which is an ethoxylated alkyl phenol (nonyl).

The thermostable enzyme of this invention may be used for any purpose in which such enzyme is necessary or desirable. In a particularly preferred embodiment, the enzyme herein is employed in the amplification protocol set forth below.

### Amplification Protocol

The amplification protocol using the enzyme of the present invention may be the process for amplifying existing nucleic acid sequences that is disclosed and claimed in European Pat. Pub. No. 200,362, supra. Preferably however, the enzyme herein is used in the amplification process described below.

In general, the amplification process involves a chain reaction for producing, in exponential quantities relative to the number of reaction steps involved, at least one specific nucleic acid sequence given (a) that the ends of the required sequence are known in sufficient detail that oligonucleotides can be synthesized which will hybridize to them, and (b) that a small amount of the sequence is available to initiate the chain reaction. The product of the chain reaction will be a discrete nucleic acid duplex with termini corresponding to the ends of the specific primers employed.

Any nucleic acid sequence, in purified ornonpurified form, can be utilized as the starting nucleic acid(s), provided it contains or is suspected to contain the specific nucleic acid sequence desired. Thus, the process may employ, for example, DNA or RNA, including messenger RNA, which DNA or RNA may be single-stranded or double-stranded. In addition, a DNA-RNA hybrid which contains one strand of each may be utilized. A mixture of any of these nucleic acids may also be employed, or the nucleic acids produced from a previous amplification reaction herein using the same or different primers may be so utilized. The specific nucleic acid sequence to be amplified may be only a fraction of a larger molecule or can be present initially as a discrete molecule, so that the specific sequence constitutes the entire nucleic acid.

It is not necessary that the sequence to be amplified be present initially in a pure form; it may be a minor fraction of a complex mixture, such as a portion of the β-globin gene contained in whole human DNA (as exemplified in Saiki et al., Science, 230, 1530-1534 (1985)) or a portion of a nucleic acid sequence due to a particular microorganism which organism might constitute only a very minor fraction of a particular biological sample. The starting nucleic acid sequence may contain more than one desired specific nucleic acid sequence which may be the same or different Therefore, the amplification process is useful not only for producing large amounts of one specific nucleic acid sequence, but also for amplifying simultaneously more than one different specific nucleic acid sequence located on the same or different nucleic acid molecules.

The nucleic acid(s) may be obtained from any source, for example, from plasmids such as pBR322, from cloned DNA or RNA, or from natural DNA or RNA from any source, including bacteria, yeast, viruses, organelles, and higher organisms such as plants or animals. DNA or RNA may be extracted from blood, tissue material such as chorionic villi, or amniotic cells by a variety of techniques such as that described by Maniatis et al., supra, p. 280-281.

If probes are used which are specific to a sequence being amplified and thereafter detected, the cells may be directly used without extraction of the nucleic acid if they are suspended in hypotonic buffer and heated to about 90-100°C, until cell lysis and dispersion of intracellular components occur, generally 1 to 15 minutes. After the heating step the amplification reagents may be added directly to the lysed cells.

Any specific nucleic acid sequence can be produced by the amplification process. It is only necessary that a sufficient number of bases at both ends of the sequence be known in sufficient detail sothat two oligonucleotide primers can be prepared which will hybridize to different strands of the desired sequence and at relative positions along the sequence such that an extension product synthesized from one primer, when it is separated from its template (complement), can serve as a template for extension of the other primer into a nucleic acid sequence of defined length. The greater the knowledge about the bases at both ends of the sequence, the greater can be the specificity of the primers for the target nucleic acid sequence, and thus the greater the efficiency of the process.

It will be understood that the word "primer" as used hereinafter may refer to more than one primer, particularly in the case where there is some ambiguity in the information regarding the terminal sequence(s) of the fragment to be amplified. For instance, in the case where a nucleic acid sequence is inferred from protein sequence information, a collection of primers containing sequences representing all possible codon variations based on degeneracy of the genetic code will be used for each strand. One primer from this collection will be homologous with the end of the desired sequence to be amplified.

The oligonucleotide primers may be prepared using any suitable method, such as, for example, the phosphotriester and phosphodiester methods described above, or automated embodiments thereof. In one such automated embodiment, diathylphosphoramidites are used as starting materials and may be synthesized as described by Beaucage et al., Tetrahedron Letters (1981), 22:1859-1862. One method for synthesizing oligonucleotides on a modified solid support is described in U.S. Patent No. 4,458,066. It is also possible to use a primer which has been isolated from a biological source (such as a restriction endonuclease digest).

The specific nucleic acid sequence is produced by using the nucleic acid containing that sequence as a template. The first step involves contacting each nucleic acid strand with four different nucleotide triphosphates and one oligonucleotide primer for each different nucleic acid sequence being amplified or detected. If the nucleic acids to be amplified or detected are DNA, then the nucleotide triphosphates are dATP, dCTP, dGTP and TTP.

The nucleic acid strands are used as a template for the synthesis of additional nucleic acid strands. This synthesis can be performed using any suitable method. Generally it occurs in a buffered aqueous solution, preferably at a pH of 7-9, most preferably about 8. Preferably, a molar excess (for cloned nucleic acid, usually about 1000:1 primentemplate, and for genomic nucleic acid, usually about 10⁶:1 primer:template) of the two oligonucleotide primers is added to the buffer containing the separated template strands. It is understood, however, that the amount of complementary strand may not be known if the process herein is used for diagnostic applications, so that the amount of primer relative to the amount of complementary strand cannot be determined with certainty. As a practical matter, however, the amount of primer added will generally be in molar excess over the amount of complementary strand (template) when the sequence tobe amplified is contained in a mixture of complicated long-chain nucleic acid strands. A large molar excess is preferred to improve the efficiency of the process.

Preferably the concentration of nucleotide triphosphates is 150-200 µM each in the buffer for amplification and MgCl₂ is present in the buffer in an amount of 1.5-2 mM to increase the efficiency and specificity of the reaction.

The resulting solution is then treated according to whether the nucleic acids being amplified or detected are double or single-stranded. If the nucleic acids are single-stranded, then no denaturation step need by employed, and the reaction mixture is held at a temperature which promotes hybridization of the primer to its complementary target (template) sequence. Such temperature is generally from about 35°C to 65°C or more, preferably about 37-60°C for an effective time, generally one-half to five minutes, preferably one-three minutes. Preferably, 45-58°C is used for Taq polymerase and >15-mer primers to increase the specificity of primer hybridization. Shorter primers need lower temperatures.

The complement to the original single-stranded nucleic acid may be synthesized by adding one or two oligonucleotide primers thereto. If an appropriate single primer is added, a primer extension product is synthesized in the presence of the primer, the thermostable enzyme and the nucleotide triphosphates. The product will be partially complementary to the single-stranded nucleic acid and will hybridize with the nucleic acid strand to form a duplex of strands of unequal length which may then be separated into single strands as described above to produce two single separated complementary strands. Alternatively, two appropriate primers may be added to the single-stranded nucleic acid and the reaction carried out

If the nucleic acid contains two strands, it is necessary to separate the strands of the nucleic acid before it can be used as the template. This strand separation can be accomplished by any suitable denaturing method including physical, chemical.or enzymatic means. One preferred physical method of separating the strands of the nucleic acid involves heating the nucleic acid until it is completely (>99%) denatured. Typical heat denaturation involves temperatures ranging from about 90 to 105°C for times generally ranging from about 0.5 to 5 minutes. Preferably the effective denaturing temperature is 90-100°C for 0.5 to 3 minutes. Strand separation may also be induced by an enzyme from the class of enzymes known as helicases or the enzyme RecA, which has helicase activity and in the presence of riboATP is known to denature DNA. The reaction conditions suitable for separating the strands of nucleic acids with helicases are described by Kuhn B., Abdel-Monem, M. and Hoffmann-Berling H., CSH-Quantitative Biology, 43: 63-67 (1978), and techniques for using RecA are reviewed in C. Radding, Ann. Rev. Genetics, 16:405-37 (1982). The denaturation produces two separated complementary strands of equal or unequal length.

If the double-stranded nucleic acid is denatured by heat, the reaction mixture is allowed to cool to a temperature which promotes hybridization of each primer present to its complementary target (template) sequence. This temperature is usually from about 35°C to 65°C or more, depending on reagents, preferably 37-60°C, maintained for an effective time, generally 0.5 to 5 minutes, and preferably 1-3 minutes. In practical terms, the temperature is simply lowered from about 95°C to as low as 37°C, preferably to about 45-58°C for Taq polymerase, and hybridization occurs at a temperature within this range.

Whether the nucleic acid is single- or double-stranded, the thermostable enzyme may be added atthe denaturation step or when the temperature is being reduced to or is in the range for promoting hybridization. The reaction mixture is then heated to a temperature at which the activity of the enzyme is promoted or optimized, i.e., a temperature sufficient to increase the activity of the enzyme in facilitating synthesis of the primer extension products from the hybridized primer and template. The temperature must actually be sufficient to synthesize an extension product of each primer which is complementary to each nucleic acid template, but must not be so high as to denature each extension product from its complementary template (i.e., the temperature is generally less than about 80°C-90°C).

Depending mainly on the types of enzyme and nucleic acid(s) employed, the typical temperature effective for this synthesis reaction generally ranges from about 40 to 80°C, preferably 50-75°C. The temperature more preferably ranges from about 65-75°C when a DNA polymerase from Thermus aquaticus is employed. The period of time required for this synthesis may range from about 0.5 to 40 minutes or more, depending mainly on the temperature, the length of the nucleic acid, the enzyme and the complexity of the nucleic acid mixture, preferably one to three minutes. If the nucleic acid is longer, a longer time period is generally required. The presence of dimethylsulfoxide (DMSO) is not necessary or recommended because DMSO was found to inhibit Taq polymerase enzyme activity.

The newly synthesized strand and its complementary nucleic acid strand form a double-stranded molecule which is used in the succeeding steps of the process. In the next step, the strands of the double-stranded molecule are separated by heat denaturation at a temperature effective to denature the molecule, but not so high that the thermostable enzyme is completely and irreversibly denatured or inactivated. Depending mainly on the type of enzyme and the length of nucleic acid, this temperature generally ranges from about 90 to 105°C, more preferably 90-100°C, and the time for denaturation typically ranges from 0.5 to four minutes, depending mainly on the temperature and nucleic acid length.

After this time, the temperature is decreased to a level which promotes hybridization of the primer to its complementary single-stranded molecule (template) produced from the previous step. Such temperature is described above.

After this hybridization step, or in lieu of (or concurrently with) the hybridization step, the temperature is adjusted to a temperature that is effective to promote the activity of the thermostable enzyme to enable synthesis of a primer extension product using as template the newly synthesized strand from the previous step. The temperature again must not be so high as to separate (denature) the extension product from its template, as previously described (usually from 40 to 80°C for 0.5 to 40 minutes, preferably 50 to 70°C for one-three minutes). Hybridization may occur during this step, so that the previous step of cooling after denaturation is not required. In such a case, using simultaneous steps, the preferred temperature range is 50-70°C.

The heating and cooling steps of strand separation, hybridization, and extension product synthesis can be repeated as often as needed to produce the desired quantity of the specific nucleic acid sequence, depending on the ultimate use. The only limitation is the amount of the primers, thermostable enzyme and nucleotide triphosphates present. Preferably, the steps are repeated at least twice. For use in detection, the number of cycles will depend, e.g., on the nature of the sample. For example, fewer cycles will be required if the sample being amplified is pure. If the sample is a complex mixture of nucleic acids, more cycles will be required to amplify the signal sufficiently for its detection. For general amplification and detection, preferably the process is repeated at least 20 times.

When labeled sequence-specific probes are employed as described below, preferably the steps are repeated at least five times. When human genomic DNA is employed with such probes, the process is repeated preferably 15-30 times to amplify the sequence sufficiently that a clearly detectable signal is produced, i.e., so that background noise does not interfere with detection.

As will be described in further detail below, the amount of the specific nucleic acid sequence produced will accumulate in an exponential fashion.

No additional nucleotides, primers, or thermostable enzyme need be added after the initial addition, provided that the enzyme has not become denatured or inactivated irreversibly, in which case it is necessary to replenish the enzyme after each denaturing step. Addition of such materials at each step, however, will not adversely affect the reaction.

When it is desired to produce more than one specific nucleic acid sequence from the first nucleic acid or mixture of nucleic acids, the appropriate number of different oligonucleotide primers are utilized. For example, if two different specific nucleic acid sequences are to be produced, four primers are utilized. Two of the primers are specific for one of the specific nucleic acid sequences and the other two primers are specific for the second specific nucleic acid sequence. In this manner, each of the two different specific sequences can be produced exponentially by the present process.

After the appropriate length of time has passed to produce the desired amount of the specific nucleic acid sequence, the reaction may be halted by inactivating the enzyme in any known manner (e.g., by adding EDTA, phenol, SDS or CHCl₃) or by separating the components of the reaction.

The amplification process may be conducted continuously. In one embodiment of an automated process, the reaction mixture maybe temperature cycled such that the temperature is programmed to be controlled at a certain level for a certain time.

One such instrument for this purpose is an automated machine for handling the amplification reaction of this invention. This instrument utilizes a liquid handling system under computer control to make liquid transfers of enzyme stored at a controlled temperature in a first receptacle into a second receptacle whose temperature is controlled by the computer to conform to a certain incubation profile. The second receptacle stores the nucleic acid sequence(s) to be amplified plus the nucleotide triphosphates and primers. The computer includes a user interface through which a user can enter process parameters that control the characteristics of the various steps in the amplification sequence such as the times and temperatures of incubation, the amount of enzyme to transfer, etc.

A preferred machine that may be employed utilizes temperature cycling without a liquid handling system because the enzyme need not be transferred at every cycle. Such a machine consists of the following systems:
1. A heat-conducting container for holding a given number of tubes, preferably 500 µl tubes, which contain the reaction mixture of nucleotide triphosphates, primers, nucleic acid sequences, and enzyme.
2. A means to heat, cool, and maintain the heat-conducting container above and below room temperature, which means has an input for receiving a control signal for controlling which of the temperatures at or to which the container is heated, cooled or maintained. (These may be Peltier heat pumps available from Materials Electronics Products Corporation in Trenton, NJ or a water heat exchanger.)
3. A computer means (e.g., a microprocessor controller), coupled to the input of said means, to generate the signals that control automatically the amplification sequence, the temperature levels, and the temperature ramping and timing.

In another embodiment, the enzyme used for the synthesis of primer extension products can be immobilized in a column. The other reaction components can be continuously circulated by a pump through the column and a heating coil in series. Thus, the nucleic acids produced can be repeatedly denatured without inactivating the enzyme.

The amplification protocol is demonstrated diagrammatically below, where double-stranded DNA containing the desired sequence [S] comprised of complementary strands [S⁺] and [S⁻] is utilized as the nucleic acid. During the first and each subsequent reaction cycle, extension of each oligonucleotide primer on the original template will produce one new ssDNA molecule product of indefinite length that terminates with only one of the primers. These products, hereafter referred to as "long products," will accumulate in a linear fashion; that is, the amount present after any number of cycles will be proportional to the number of cycles.

The long products thus produced will act as templates for one or the other of the oligonucleotide primers during subsequent cycles and will produce molecules of the desired sequence [S⁺] or [S]. These molecules will also function as templates for one or the other of the oligonucleotide primers, producing further [S⁺] and [S⁻], and thus a chain reaction can be sustained that will result in the accumulation of [S] at an exponential rate relative to the numberof cycles.

By-products formed by oligonucleotide hybridizations other than those intended are not self-catalytic (except in rare instances) and thus accumulate at a linear rate.

The specific sequence to be amplified, [S], can be depicted diagrammatically as: 
[S+]          5'  AAAAAAAAAXXXXXXXXXCCCCCCCCC  3'
[S-]          3'  TTTTTTTTTYYYYYYYYYGGGGGGGGG  5'
 The appropriate oligonucleotide primers would be: 
Primer 1:          3' GGGGGGGGGG 5'
Primer 2:          5' AAAAAAAAAA 3'
 so that if DNA containing [S] 
....zzzzzzzzzzzzzzzAAAAAAAAAXXXXXXXXXCCCCCCCCCzzzzzzzzzzzzzzzz....
....zzzzzzzzzzzzzzzTTTTTTTTTYYYYYYYYYGGGGGGGGGzzzzzzzzzzzzzzzz....
 is separated into single strands and its single strands are hybridized to Primers 1 and 2, the following extension reactions can be catalyzed by a thermostable polymerase in the presence of the four nucleotide triphosphates: 
3'        5'
extends ←———————————— GGGGGGGGGG  Primer 1

....zzzzzzzzzzzzzzzzzAAAAAAAAAAXXXXXXXXXXCCCCCCCCCCzzzzzzzzzzzzzzzzz....
original template strand+

original template strand−
....zzzzzzzzzzzzzzzzzTTTTTTTTTTYYYYYYYYYYGGGGGGGGGGzzzzzzzzzzzzzzzzz....

Primer 2     AAAAAAAAAA ————————→ extends
             5'       3'
 On denaturation of the two duplexes formed, the products are: 
3'                                                    5'
....zzzzzzzzzzzzzzzzTTTTTTTTTTYYYYYYYYYYGGGGGGGGGG
newly synthesized long product 1

5'                                                                        3'
....zzzzzzzzzzzzzzzzAAAAAAAAAAXXXXXXXXXXCCCCCCCCCCzzzzzzzzzzzzzzzz....
original template strand+

3'                                                                        5'
....zzzzzzzzzzzzzzzzTTTTTTTTTTYYYYYYYYYYGGGGGGGGGGzzzzzzzzzzzzzzzz....
original template strand-

                    5'                                                    3'
                    AAAAAAAAAAXXXXXXXXXXCCCCCCCCCCzzzzzzzzzzzzzzzz....
                    newly synthesized long product 2
 If these four strands are allowed to rehybridize with Primers 1 and 2 in the next cycle, the thermostable polymerase will catalyze the following reactions: 
Primer 2      5' AAAAAAAAAA ——————→ extends to here

3'...zzzzzzzzzzzzzzzzzzzTTTTTTTTTTYYYYYYYYYYGGGGGGGGGG 5'
newly synthesized long product 1

extends ←——————— - - GGGGGGGGGG 5' Primer 1

5'....zzzzzzzzzzzzzzAAAAAAAAAAXXXXXXXXXXCCCCCCCCCCzzzzzzzzzzzzzz....3'
original template strand+

Primer 2      5' AAAAAAAAAA ——————→ extends

3'...zzzzzzzzzzzzzzzzzzzTTTTTTTTTTYYYYYYYYYYGGGGGGGGGGzzzzzzzzz....5'
original template strand-

extends to here ←——————— GGGGGGGGGG  5'  Primer 1

5' AAAAAAAAAAXXXXXXXXXXCCCCCCCCCCzzzzzzzzzzzzzzzz..3'
newly synthesized long product 2
 If the strands of the above four duplexes are separated, the following strands are found:

5' AAAAAAAAAAXXXXXXXXXXCCCCCCCCCC 3'
newly synthesized [S+]

3'....zzzzzzzzzzzzzzzzzzzTTTTTTTTTTYYYYYYYYYYGGGGGGGGGG 5'
first cycle synthesized long product 1

3'....zzzzzzzzzzzzzzzzzzzTTTTTTTTTTYYYYYYYYYYGGGGGGGGGG 5'
newly synthesized long product 1

5'....zzzzzzzzzzzzzzzzzzzAAAAAAAAAAXXXXXXXXXXCCCCCCCCCCzzzzzzzzz....3'
original template strand+

5' AAAAAAAAAAXXXXXXXXXXCCCCCCCCCCzzzzzzzzzzzzzzz...3'
newly synthesized long product 2

3'..zzzzzzzzzzzzzzzTTTTTTTTTTYYYYYYYYYYGGGGGGGGGGzzzzzzzzzzzzzzz...5'
original template strand-

3' TTTTTTTTTTYYYYYYYYYYGGGGGGGGGG 5'
newly synthesized [S-]

5' AAAAAAAAAAXXXXXXXXXXCCCCCCCCCCzzzzzzzzzzzzzzz...3'
first cycle synthesized long product 2

It is seen that each strand which terminates with the oligonucleotide sequence of one primer and the complementary sequence of the other is the specific nucleic acid sequence [S] that is desired to be produced.

The amount of original nucleic acid remains constant in the entire process, because It is not replicated. The amount of the long products increases linearly because they are produced only from the original nucleic acid. The amount of the specific sequence increases exponentially. Thus, the specific sequence will become the predominant species. This is illustrated in the following table, which indicates the relative amounts of the species theoretically present after n cycles, assuming 100% efficiency at each cycle:

| Number of Double Strands After 0 to n Cycles | | | |
|---|---|---|---|
| Cycle Number | Template | Long Products | Specific Sequence [S] |
| 0 | 1 | - | - |
| 1 | 1 | 1 | 0 |
| 2 | 1 | 2 | 1 |
| 3 | 1 | 3 | 4 |
| 5 | 1 | 5 | 26 |
| 10 | 1 | 10 | 1013 |
| 15 | 1 | 15 | 32,752 |
| 20 | 1 | 20 | 1,048,555 |
| n | 1 | n | (2ⁿ-n-1) |

When a single-stranded nucleic acid is utilized as the template, only one long product is formed per cycle.

A sequence within a given sequence can be amplified after a given number of amplifications to obtain greater specificity of the reaction by adding after at least one cycle of amplification a set of primers that are complementary to internal sequences (that are not on the ends) of the sequence to be amplified. Such primers may be added at any stage and will provide a shorter amplified fragment. Altematively, a longer fragment can be prepared by using primers with non-complementary ends but having some overlap with the primers previously utilized in the amplification.

The method herein may be utilized to clone a particular nucleic acid sequence for insertion into a suitable expression vector. The vector may then be used to transform an appropriate host organism to produce the gene product of the sequence by standard methods of recombinant DNA technology.

The amplification process herein may yield a mixture of nucleic acids, resulting from the original template nucleic acid, the expected target amplified products, and various background non-target products. The amplified product can also be a mixture if the original template DNA contains multiple target sequences, such as in a heterozygous diploid genome or when there is a family of related genes.

The primers herein may be modified to assist the rapid and specific cloning of the mixture of DNAs produced by the amplification reaction. In one such modification, a restriction site is contained in each of the primers or in the sequence to be amplified and cloned. Preferably, the same or different restriction sites are incorporated at the 5' ends of the primers to result in restriction sites at the two ends of the amplified product. When cut with the appropriate enzymes, the amplified product can then be easily inserted into plasmid or viral vectors and cloned. This cloning allows the analysis or expression of individual amplified products, not a mixture.

If the primers have restriction sites incorporated therein, the same restriction site can be used for both primers. The use, however, of different sites allows the insertion of the product into the vector with a specific orientation and suppresses multiple insertions as well as insertions arising from amplifications based on only one of the two primers. The specific orientation is useful when cloning into single-strand sequencing vectors, when single-strand hybridization probes are used, or when the cloned product is being expressed.

One method to prepare the primers is to choose a primer sequence which differs minimally from the target sequence. Regions in which each of the primers is to be located are screened for homology to restriction sites appropriate tothe desired vector. For example, the target sequence "CAGTATCCGA..." differs by only one base from one containing a BamHl site. A primer sequence is chosen to match the target exactly at its 3' end, and to contain the altered sequence and restriction site near its 5' end (for example, "CAGgATCCGA...", where the lower r case letter symbolizes a mismatch with the target sequence). This minimally altered sequence will not interfere with the ability of the primer to hybridize to the original target sequence and to initiate polymerization. After the first amplification cycle the primer is copied, becomes the target, and matches exactly with new primers.

After the amplification process, the products are cleaved with the appropriate restriction enzymes, the restriction digest is optionally separated from inhibitors of ligation such as the nucleotide triphosphates and salts by, for example, passing over a desalting column, or a molecular weight chromatography column, or through a membrane, and the digestion product(s) containing the amplified sequence to be cloned is/are inserted by ligation into a cloning vector such as bacteriophage M13. The cloning vector generally has a selectable marker and may optionally also have a promoter. The gene may then be sequenced and/or expressed, if it codes for a protein, using well known techniques. The gene may also be sequenced by adding an appropriate primer during the amplification process which primer is complementary to the desired portion which is to be sequenced. The primer will form an extension product, and the extent of amplification with such extension product will provide sequence information.

Another method for preparing the primers involves taking the 3' end of the primers from the target sequence and adding the desired restriction site(s) to the 5' end of the primer. For the above example, a HindIII site could be added to make the sequence "cgaagcttCAGTATCCGA...", where lower case letters are as described above. The added bases would not contribute to the hybridization in the first cycle of amplification, but would match in subsequent cycles. The final amplified products are then cut with restriction enzyme(s) and cloned and expressed as described above. The gene being amplified may be, for example, human beta-hemoglobin or the human HLA DQ, DR or DP-α and -β genes.

In an alternative, but less preferred and less efficient, method of cloning wherein blunt-end ligation is employed rather than sticky-end ligation (using restriction enzymes), the basic amplification procedure is employed without concem for restriction sites in the primers or sequence(s) to be cloned. The steps must be repeated sufficiently, however, to produce enough amplified sequence(s) to effect ligation. Blunt-end ligation requires greater concentrations of s sequence(s) and cloning vector(s) to be present than sticky-end ligation. In addition, the ligation must take place in the presence of a ligase, such as T4 ligass, E. coli ligase and ligase. Once the amplified product is obtained, the ligation procedure is a standard procedure using conditions well known to those skilled in the art.

The cloning method which does not involve blunt end ligation controls the orientation or multiplicity of insertion of the amplified product into the cloning vector.

In addition, the process herein can be used for in vitro mutagenesis. The oligonucleotide primers need not be exactly complementary to the nucleic acid sequence which is being amplified. It is only necessary that they be able to hybridize to the sequence sufficiently well to be extended by the thermostable enzyme. The product of an amplification reaction wherein the primers employed are not exactly complementary to the original template will contain the sequence of the primer rather than the template, thereby introducing an in vitro mutation. In further cycles this mutation will be amplified with an undiminished efficiency because no further mispaired primings are required. The mutant thus produced may be inserted into an appropriate vector by standard molecular biological techniques and might confer mutant properties on this vector such as the potential for production of an altered protein.

The process of making an altered DNA sequence as described above could be repeated on the altered DNA using different primers to induce further sequence changes. In this way a series of mutated sequences could gradually be produced wherein each new addition to the series could differ from the last in a minor way, but from the original DNA source sequence in an increasingly major way. In this manner changes could be made ultimately which were not feasible in a single step due to the inability of a very seriously mismatched primer to function.

In addition, the primer can contain as part of its sequence a non-complementary sequence, provided that a sufficient amount of the primer contains a sequence which is complementary to the strand to be amplified. For example, a nucleotide sequence which is not complementary to the template sequence (such as, e.g., a promoter, linker, coding sequence, etc.) may be attached at the 5' end of one or both of the primers, and thereby appended to the product of the amplification process. After the extension primer is added, sufficient cycles are run to achieve the desired amount of new template containing the non-complementary nucleotide insert. This allows production of large quantities of the combined fragments in a relatively short period of time (e.g., two hours or less) using a simple technique.

The method herein may also be used to enable detection and/or characterization of specific nucleic acid sequences associated with infectious diseases, genetic disorders or cellular disorders such as cancer, e.g., oncogenes. Amplification is useful when the amount of nucleic acid available for analysis is very small, as, for example, in the prenatal diagnosis of sickle cell anemia using DNA obtained from fetal cells. Amplification is particularly useful if such an analysis is to be done on a small sample using non-radioactive detection techniques which may be inherently insensitive, or where radioactive techniques are being employed but where rapid detection is desirable.

For purposes of this invention genetic diseases may include specific deletions and/or mutations in genomic DNA from any organism, such as, e.g., sickle cell anemia, α-thalassemia, β-thalassemia, and the like. Sickle cell anemia can be readily detected via oligomer restriction analysis as described in EP Patent Publication 164,054 published December 11, 1985, or via a RFLP-like analysis following amplification of the appropriate DNA sequence by the present method. α-Thalassemia can be detected by the absence of a sequence, and β-thalassemia can be detected by the presence of a polymorphic restriction site closely linked to a mutation that causes the disease.

All of these genetic diseases may be detected by amplifying the appropriate sequence and analyzing it by Southern blots without using radioactive probes. In such a process, for example, a small sample of DNA from, e.g., amniotic fluid containing a very low level of the desired sequence is amplified, cut with a restriction enzyme, and analyzed via a Southern blotting technique. The use of non-radioactive probes is facilitated by the high level of the amplified signal.

In another embodiment, a small sample of DNA may be amplified to a convenient level and then a further cycle of extension reactions performed wherein nucleotide derivatives which are readily detectable (such as ³²P-labeled or biotin-labeled nucleotide triphosphates) are incorporated directly into the final DNA product, which may be analyzed by restriction and electrophoretic separation or any other appropriate method.

In a further embodiment, the nucleic acid may be exposed to a particular restriction endonuclease prior to amplification. Since a sequence which has been cut cannot be amplified, the appearance of an amplified fragment, despite prior restriction of the DNA sample, implies the absence of a site for the endonuclease within the amplified sequence. The presence or absence of an amplified sequence can be detected by an appropriate method.

A practical application of this technique can be illustrated by its use in facilitating the detection of sickle cell anemia via the oligomer restriction technique described herein and in EP 164,054, supra, and Saiki et al., Bio/Technology, 3, pp 1008-1012 (1985). Sickle cell anemia is a hemoglobin disease which is caused by a single base pair change in the sixth codon of the β-globin gene.

The method of this invention may also be used to detect directly single-base pair variations in nucleic acid sequence (such as genomic DNA) using sequence-specific oligonucleotides. In this method, the sequence variation, whether resulting from cancer, an infectious disease, or a genetic disease, e.g., a genetic lesion, is directly detected, eliminating the need for restriction digestion, electrophoresis, and gel manipulations otherwise required. The use of sequence-specific oligonucleotides in a dot blot format after amplification, as described herein, provides for improved specificity and sensitivity of the probe; an interpretable signal can be obtained with a 0.04 µg sample in six hours. Also, if the amount of sample spotted on a membrane is increased to 0.1-0.5 µg, non-isotopically labeled oligonucleotides may be utilized rather than the radioactive probes used in previous methods. Furthermore, the process described hereinbelow is applicable to use of sequence-specific oligonucleotides less than 19-mers in size, thus allowing use of more discriminatory sequence-specific oligonucleotides.

Regarding genetic diseases, while RFLP requires a polymorphic restriction site to be associated with the disease, sequence-specific oligonucleotides directly detect the genetic lesion and are generally more useful for the analysis of such diseases as hemoglobin C disease, α-1-antitrypsin and β-thalassemia, which result from single-base mutations. In addition, the oligonucleotides can be used to distinguish between genetic variants which represent different alleles (e.g., HLA typing), indicating the feasibility of a sequence-specific oligonucleotide-based HLA typing kit that includes a thermostable enzyme.

In one embodiment of the invention herein wherein a nucleotide variation in sequence is to be detected, the sample, amplified as described above using one primer for each strand of each nucleic acid suspected of containing the nucleotide variation, is spotted directly on a series of membranes and each membrane is hybridized with a different labeled sequence-specific oligonucleotide probe. One procedure for spotting the sample on a membrane is descibed by Kafotos et al., Nucleic Acids Research, 7:1541-1552 (1979).

Briefly, the DNA sample affixed to the membrane may be pretreated with a prehybridization solution containing sodium dodecyl sulfate, Ficoll, serum albumin and various salts prior to the probe being added. Then, a labeled oligonucleotide probe which is specific to each sequence variation to be detected is added to a hybridization solution similar to the prehybridization solution. The hybridization solution is applied to the membrane and the membrane is subjected to hybridization conditions that will depend on the probe type and length, the type and concentration of ingredients, etc. Generally, hybridization is carried out at about 25 to 75°C, preferably 35 to 65°C, for 0.25-50 hours, preferably less than three hours. The greater the stringency of conditions, the greater the required complementarity for hybridization between the probe and sample. If the background level is high, stringency may be increased accordingly. The stringent conditions can also be incorporated in the wash.

After the hybridization, the sample is washed of unhybridized probe using any suitable means such as by washing one or more times with varying concentrations of standard saline phosphate EDTA (SSPE) (180 mM NaCl, 10 mM NaHPO₄ and 1 mM EDTA, pH 7.4) solutions at 25-75°C for about 10 minutes to one hour, depending on the temperature. The label is then detected by using any appropriate detection technique.

The sequence-specific oligonucleotide employed herein is an oligonucleotide which is generally prepared and selected as described above for preparing and selecting the primers. As described above, the sequence-specific oligonucleotide must encompass the region of the sequence which spans the nucleotide variation being detected and must be specific for the nucleotide variation being detected. For example, if it is desired to detect whether a sample contains the mutation for sickle call anemia, one oligonucleotide will be prepared which contains the nucleotide sequence site characteristic of the normal β-globin gene and one oligonucleotide will be prepared which contains the nucleotide sequence characteristic of the sickle cell allele. Each oligonucleotide would be hybridized to duplicates of the same sample to determine whether the sample contains the mutation.

The polymorphic areas of HLA class-li genes are localized to specific regions of the first exon and are flanked by conserved sequences, so that oligonucleotide primers complementary to opposite strands of the conserved 5' and 3' ends of the first exon can be prepared.

The number of oligonucleotides employed for detection of the polymorphic areas of the HLA class II genes will vary depending on the type of gene, which has regions of base pair variation which may be clustered or spread apart. If the regions are clustered, as in the case with HLA-DQ-α, then one oligonucleotide is employed for each allele. If the regions are spread apart, as in the case with HLA-DQ-β and HLA-DR-β, then more than one probe, each encompassing an allelic variant, will be used for each allele. In the case of HLA-DQ-β and HLA-DR-β, three probes are employed for the three regions of the locus where allelic variations may occur. For detection of insulin-dependent diabetes mellitus (IDDM) four probes for the HLA-DRβ second exon are employed.

Haplotypes can be inferred from segregation analysis in families or, in some cases, by direct analysis of the individual DNA sample. Specific allelic combinations (haplotypes) of sequence-specific oligonucleotide reactivities can be identified in heterozygous cells by using restriction enzyme digestion of the genomic DNA prior to amplification.

For example, if in DQβ one finds three highly variable subregions A, B, and C within a single amplified region, and if there are six different sequences at each region (A1-6, B1-6, C1-6), then an individual could be typed in the DQβ locus by sequence-specific oligonucleotide probe analysis as containing A1, A2; B2, B3; C1, C4, with the possible haplotype combinations of A1, B2, C1; A1, B2, C4; A2, 82, C1; A2, B2, C4; A1; B3, C1; A1, B3, C4; A1, B2, C1; and A1, B2, C4.

If the genomic DNA is digested with a polymorphic restriction enzyme priorto amplification, and if the enzyme cuts both alleles between the primers, there is no reactivity with the sequence-specific probes due to lack of amplification, andthe result is uninformative. If the enzyme cuts neither allele, the probe results with digested and undigested genomic DNA are the same and the result is uninformative. If the enzyme cuts only one allele, however, then one can infer both haplotypes by comparing the probe reactivity patterns on digested and undigested DNA.

The haplotypes can be deduced by comparing sequence-specific oligonucleotide reactivities with uncut genomic DNA and genomic DNA cut with one or several enzymes known to be polymorphic and to recognize sites between the primers.

The length of the sequence-specific oligonucleotide will depend on many factors, including the particular target molecule being detected, the source of oligonucleotide, and the nucleotide composition. For purposes herein, the sequence-specific oligonucleotide typically contains 15-25 nucleotides, although it may contain more or fewer nucleotides. While oligonucleotides which are at least 19 mers in length may enhance specificity and/or sensitivity, probes which are less than 19 mers, e.g., 16-mers, may show more sequence-specific discrimination presumably because a single mismatch is more destabilizing. Because amplification increases specificity so that a longer length is less critical, and hybridization and washing temperatures can be lower for the same salt concentration, it is preferred to use oligonucleotides which are less than 19 mers in length.

Where the sample is first placed on the membrane and then detected with the oligonucleotide, the oligonucleotide must be labeled with a suitable label moiety, which may be detected by spectroscopic, photochemical, biochemical, immunochemical or chemical means. Immunochsmical means include antibodies which are capable of forming a complex with the oligonucleotide under suitable conditions, and biochemical means include polypeptides or lectins capable of forming a complex with the oligonucleotide under the appropriate conditions. Examples include fluorescent dyes, electron-dense reagents, enzymes capable of depositing insoluble reaction products or being detected chromogenically, such as horseradish peroxidase, alkaline phosphatase, a radioactive label such as.³²P, or biotin. If biotin is employed, a spacer arm may be utilized to attach it to the oligonucleotide. Preferably, the label moiety is horseradish peroxidase.

Alternatively, in one "reverse" dot blot format, at least one of the primers and/or at least one of the four nucleotide triphosphates is labelad with a detectable label, so that the resulting amplified sequence is labeled. These labeled moieties may be present initially in the reaction mixture or added during a later cycle of the amplification to introduce the label to the amplification product. Then an unlabeled sequence-specific oligonucleotide capable of hybridizing with the amplified nucleic acid sequence, if the sequence variation(s) (whether normal or mutant) is/are present, is spotted on (affixed to) the membrane under prehybridization conditions as described above. The amplified sample is then added to the pretreated membrane under hybridization conditions as described above. Finally, detection means are used to determine if an amplified sequence in the nucleic acid sample has hybridized to the oligonucleotide affixed to the membrane. Hybridization will occur only if the membrane-bound sequence containing the variation is present in the amplification product, i.e., only if a sequence of the probe is complementary to a region of the amplified sequence.

In another version of the "reverse" dot blot format, the amplification is carried out without employing a label as with the "forward" dot blot format described above, and a labeled sequence-specific oligonucleotide probe capable of hybridizing with the amplified nucleic acid sequence containing the variation, if present, is spotted on (affixed to) the membrane under prehybridization conditions as described above. The amplified sample is then added to the pretreated membrane under hybridization conditions as described above. Then the labeled oligonucleotide or a fragment thereof is released from the membrane in such a way that a detection means can be used to determine if an amplified sequence in the sample hybridized to the labeled oligonucleotide. The release may take place, for example, by adding a restriction enzyme to the membrane which recognizes a restriction site in the probe. This procedure, known as oligomer restriction, is described more fully in EP Patent Publication 164,054 published December 11, 1985.

In both the forward and reverse dot blot methods, the genetic diseases which may be detected include specific deletions, insertions and/or substitutions in any base pair mutation or polymorphism in nucleic acids, for example, genomic DNA, from any organism. Examples of diseases in which base pair variation is known include sickle cell anemia, hemoglobin C disease, α-thalassemia, β-thalassemia, and the like. Other diseases that may be detected include cancerous diseases such as those involving the RAS oncogenes, e.g., the n-RAS oncogene, and infectious diseases.

A dot blot process may also be used for HLA typing in the areas of tissue transplantation, disease susceptibility, and paternity determination. The HLA class II genes, consisting of the α and β genes from the HLA-DR, HLA-DQ and HLA-DP regions, are highly polymorphic; their genetic complexity at the DNA level is significantly greater than the polymorphism currently defined by serological typing. In addition, the process herein may be employed to detect four DNA sequences coding for HLA class II β proteins (e.g. DRβ) associated with insulin-dependent diabetes mellitus (IDDM). Briefly, the four DNA sequences associated with IDDM are selected from the group consisting of: 1) 5'-GAGCTGCGTAAGTCTGAG-3', 2) 5'-GAGGAGTTCCTGCGCTTC-3', 3) 5'-CCTGTCGCCGAGTCCTGG-3', and 4) 5'-GACATCCTGGAAGACGAGAGA-3', or the DNA strands that are complementary thereto. Sequence-specific probes may be prepared that will hybridize to one or more of these sequences.

Various infectious diseases can be diagnosed by the presence in clinical samples of specific DNA sequences characteristic of the causative microorganism. These include bacteria, such as Salmonella, Chlamydia, Neisseria; viruses, such as the hepatitis viruses, and parasites, such as the Plasmodium responsible for malaria. U.S. Patent Reexamination Certificate B14,358,535 issued on May 13, 1986 to Fallow et al. describes the use of specific DNA hybridization probes for the diagnosis of infectious diseases. A relatively small number of pathogenic organisms may be present in a clinical sample from an infected patient and the DNA extracted from these may constitute only a very small fraction of the total DNA in the sample. Specific amplification of suspected sequences prior to immobilization and hybridization detection of the DNA samples could greatly improve the sensitivity and specificity of traditional procedures.

Routine clinical use of DNA probes for the diagnosis of infectious diseases would be simplified considerably if non-radioactively labeled probes could be employed as described in EP 63,879 to Ward. In this procedure biotin-containing DNA probes are detected by chromogenic enzymes linked to avidin or biotin-specific antibodies. This type of detection is convenient, but relatively insensitive. The combination of specific DNA amplification by the present method and the use of stably labeled probes could provide the convenience and sensitivity required to make the Falkow and Ward procedures useful in a routine clinical setting.

A specific use of the amplification technology for detecting or monitoring for the AIDS virus is described as follows. The amplification and detection process is used with primers and probes which are designed to amplify and detect, respectively, nucleic acid sequences which are substantially conserved among the nucleic acids in AIDS viruses and specific to the nucleic acids in AIDS viruses. Thus, the sequence to be detected must be sufficiently complementary to the nucleic acids in AIDS viruses to initiate polymerization, preferably at room temperature, in the presence of the enzyme and nucleotide triphosphates.

In addition, the probe may be a biotinylated probe in which the biotin is attached to a spacer arm of the formula; 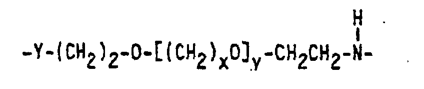 where Y is O, NH or N-CHO, x is a number from 1 to 4, and y is a number from 2 to 4. The spacer arm is in turn attached to a psoralen moiety of the formula: 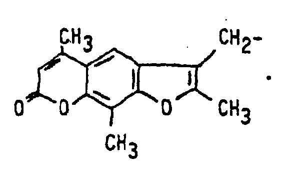 The psoralen moiety intercalates into and crosslinks a "gapped circle" probe as described by Courage-Tebbe et al., Biochim. Biophys. Acta, 697 (1982) 1-5, wherein the single-stranded hybridization region of the gapped circle spans the region contained in the primers. The details of this biotinylation and dot blot procedure are described more fully in commonly assigned U.S. Patent No. 4,582,789 issued April 15, 1986 and U.S. Patent No. 4,617,261 issued October 14, 1986.

The amplification process can also be utilized to produce sufficient quantities of DNA from a single copy human gene such that detection by a simple non-specific DNA stain such as ethidium bromide can be employed to diagnose DNA directly.

In addition to detecting infectious diseases and pathological abnormalities in the genome of organisms, the amplification process can also be used to detect DNA polymorphisms which may not be associated with any pathological state.

In summary, the amplification process is seen to provide a process for amplifying one or more specific nucleic acid sequences using a chain reaction and a thermostable enzyme, in which reaction primer extension products are produced which can subsequently act as templates for further primer extension reactions. The process is especially useful in detecting nucleic acid sequences which are initially present in only very small amounts.

The following examples are offered by way of illustration only and are by no means intended to limit the scope of the claimed invention. In these examples, all percentages are by weight if for solids and by volume if for liquids, unless otherwise noted, and all temperatures are given in degrees Celsius.

### EXAMPLE I

### 1. Synthesis of the Primers

The following two oligonucleotide primers were prepared by the method described below: 
5'-ACACAACTGTGTTCACTAGC-3' (PC03)
5'-CAACTTCATCCACGTTCACC-3' (PC04)
 These primers, both 20-mers, anneal to opposite strands of the genomic DNA with their 5' ends separated by a distance of 110 base pairs.
A. Automated Synthesis Procedures: The disthylphosphoramidites, synthesized according to Beaucage and Caruthers (Tetrahedron Letters (1981) 22:1859-1862) were sequentially condensed to a nucleoside derivatized controlled pore glass support. The procedure included detritylation with trichloroacetic acid in dichloromethane, condensation using benzotriazole as activating proton donor, and capping with acetic anhydride and dimethylaminopyridine in tetrahydrofuran and pyridine. Cycle time was approximately 30 minutes. Yields at each step were essentially quantitative and were determined by collection and spectroscopic examination of the dimethoxytrityl alcohol released during detritylation.
B. Oligodeoxyribonucleotide Deprotection and Purification Procedures: The solid support was removed from the column and exposed to 1 ml concentrated ammonium hydroxide at room temperature for four hours in a closed tube. The support was then removed by filtration and the solution containing the partially protected oligodeoxynucleotide was brought to 55°C for five hours. Ammonia was removed and the residue was applied to a preparative polyacrylamide gel. Electrophoresis was carried out at 30 volts/cm for 90 minutes after which the band containing the product was identified by UV shadowing of a fluorescent plate. The band was excised and eluted with 1 ml distilled water overnight at 4°C. This solution was applied to a RP-HPLC column and eluted with a 7-13% gradient of acetonitrile in 1% ammonium acetate buffer at pH 6.0. The elution was monitored by UV absorbance at 260 nm and the appropriate fraction collected, quantitated by UV absorbance in a fixed volume and evaporated to dryness at room temperature in a vacuum centrifuge.
C. Characterization of Oligodeoxyribonucleotides: Test aliquots of the purified oligonucleotides were ³²P labeled with polynucleotide kinase and γ-³²P-ATP. The labeled compounds were examined by autoradiography of 14-20% polyacrylamide gels after electrophoresis for 45 minutes at 50 volts/cm. This procedure verifies the molecular weight. Base composition was determined by digestion of the bligodeoxyribonucleotide to nucleosides by use of venom diesterase and bacterial alkaline phosphatase and subsequent separation and quantitation of the derived nucleosides using a reverse phase HPLC column and a 10% acetonitrile, 1% ammonium acetate mobile phase.

### II. Isolation of Human Genomic DNA from Cell Line

High molecular weight genomic DNA was isolated from a T cell line, Molt 4, homozygous for normal β-globin available from the Human Genetic Mutant Cell Depository, Camden, NJ as GM2219C using essentially the method of Maniatis et al., supra, p. 280-281.

### III. Purification of a Polymerase From Thermus aquaticus

Thermus aquaticus strain YT1, available without restriction from the American Type Culture Collection, 12301 Parklawn Drive, Rockville, MD, as ATCC No. 25,104 was grown in flasks in the following medium:

| | |
|---|---|
| Sodium Citrate | 1 mM |
| Potassium Phosphate, pH 7.9 | 5 mM |
| Ammonium Chloride | 10 mM |
| Magnesium Sulfate | 0.2 mM |
| Calcium Chloride | 0.1 mM |
| Sodium Chloride | 1 g/l |
| Yeast Extract | 1 g/l |
| Tryptone | 1 g/l |
| Glucose | 2 g/l |
| Ferrous Sulfate | 0.01 mM |

(The pH was adjusted to 8.0 prior to autoclaving.)

A 10-liter fermentor was inoculated from a seed flask cultured overnight in the above medium at 70°C. A total of 600 ml from the seed flask was used to inoculate 10 liters of the same medium. The pH was controlled at 8.0 with ammonium hydroxide with the dissolved oxygen at 40%, with the temperature at 70°C, and with the stirring rate at 400 rpm.

After growth of the cells, they were purified using the protocol (with slight modification) of Kaledin et al., supra, through the first five stages and using a different protocol for the sixth stage. All six steps were conducted at 4° C. The rate of fractionation on columns was 0.5 columns/hour and the volumes of gradients during elution were 10 column volumes. An aftemative and preferred purification protocol is provided in Example VI below.

Briefly, the above culture of the T. aquaticus cells was harvested by centrifugation after nine hours of cultivation, in late log phase, at a cell density of 1.4 g dry weight/l. Twenty grams of cells were resuspended in 80 ml of a buffer consisting of 50 mM Tris·HCl pH 7.5, 0.1 mM EDTA. Cells were lysed and the lysate was centrifuged for two hours at 35,000 rpm in a rotor at 4°C. The supernatant was collected (fraction A) and the protein fraction precipitating between 45 and 75% saturation of ammonium sulfate was collected, dissolved in a buffer consisting of 0.2 M potassium phosphate buffer, pH 6.5,10 mM 2-mercaptoethanol, and 5% glycerine, and finally dialyzed against the same buffer to yield fraction B.

Fraction B was applied to a 2.2 × 30-cm column of DEAE-cellulose, equilibrated with the above described buffer. The column was then washed with the same buffer and the fractions containing protein (determined by absorbance at 280 nm) were collected. The combined protein fraction was dialyzed against a second buffer, containing 0.01 M potassium phosphate buffer, pH 7.5, 10 mM 2-mercaptoethanol, and 5% glycerine, to yield fraction C.

Fraction C was applied to a 2.6 × 21-cm column of hydroxyapatite, equilibrated with a second buffer. The column was then washed and the enzyme was eluted with a linear gradient of 0.01-0.5 M potassium phosphate buffer, pH 7.5, containing 10 mM 2-mercaptoethanol and 5% glycerine. Fractions containing DNA polymerase activity (90-180 mM potassium phosphate) were combined, concentrated four-fold using an Amicon stirred cell and YM10 membrane, and dialyzed against the second buffer to yield fraction D.

Fraction D was applied to a 1.6 × 28-cm column of DEAE-cellulose, equilibrated with the second buffer. The column was washed and the polymerase was eluted with a linear gradient of 0.01-0.5 M potassium phosphate in the second buffer. The fractions were assayed for contaminating endonuclease(s) and exonuclease(s) by elactrophoretically detecting the change in molecular weight of phage λ DNA or supercoiled plasmid DNA after incubation with an excess of DNA polymerase (for endonuclease) and after treatment with a restriction enzyme that cleaves the DNA into several fragments (for exonuclease). Only those DNA polymerase fractions (65-95 mM potassium phosphate) having minimal nuclease contamination were pooled. To the pool was added autoclaved gelatin in an amount of 250 µg/ml, and dialysis was conducted against the second buffer to yield Fraction E.

Fraction E was applied to a phosphocellulose column and eluted with a 100 ml gradient (0.01 -0.4 M KCl gradient in 20 mM potassium phosphate buffer pH 7.5). The fractions were assayed for contaminating endo/exonuclease(s) as described above as well as for polymerase activity (by the method of Kaledin et al.) and then pooled. The pooled fractions were dialyzed against the second buffer, then concentrated by dialysis against 50% glycerine and the second buffer.

The molecular weight of the polymerase was determined by SDS PAGE. Marker proteins were phosphorylase B (92,500), bovine serum albumin (66,200), ovalbumin (45,000), carbonic anhydrase (31,000), soybean trypsin inhibitor (21,500), and lysozyme (14,400).

Preliminary data suggest that the polymerase has a molecular weight of about 86,000-90,000 daltons, not 62,000-63,000 daltons reported in the literature (e.g., by Kaledin et al.).

The polymerase was incubated in 50 µl of a mixture containing 25 mM Tris-HCl pH 6.4 and pH 8.0, 0.1 M KCI, 10 mM MgCl₂, 1 mM 2-mercaptoethanol, 10 nmoles each of dGTP, dATP, and TTP, and 0.5 µCi (³H) dCTP, 8 µg "activated" calf thymus DNA, and 0.5-5 units of the polymerase. "Activated" DNA is a native preparation of DNA after partial hydrolysis with DNase I until 5% of the DNA was transferred to the acid-soluble fraction. The reaction was conducted at 70°C for 30 minutes, and stopped by adding 50 µl of a saturated aqueous solution of sodium pyrophosphate containing 0.125 M EDTA-Na₂. Samples were processed and activity was determined as described by Kaledin et al., supra.

The results showed that at pH 6.4 the polymerase was more than one-half as active as at pH 8.0. In contrast, Kaledin et al. found that at pH about 7.0, the enzyme therein had 8% of the activity at pH 8.3. Therefore, the pH profile for the thermostable enzyme herein is broader than that for the Kaledin et al. enzyme.

Finally, when only one or more nucleotide triphosphates were eliminated from a DNA polymerase assay reaction mixture, very little, if any, activity was observed using the enzyme herein, and the activity was consistent with the expected value, and with an enzyme exhibiting high fidelity. In contrast, the activity observed using the Kaledin et al. (supra) enzyme is not consistent with the expected value, and suggests misincorporation of nucleotide triphosphate(s).

### IV. Amplification Reaction

One microgram of the genomic DNA described above was diluted in an initial 100 µl aqueous reaction volume containing 25 mM Tris·HCl µl buffer (pH 8.0), 50 mM KCl, 10 mM MgCl₂, 5 mM dithiothreitol, 200 µg/ml gelatin, 1 µM of primer PC03, 1 µM of primer PC04,1.5 mM dATP, 1.5 mM dCTP, 1.5 mM dGTP and 1.5 mM TTP. The sample was heated for 10 minutes at 98°C to denature the genomic DNA, then cooled to room temperature. Four microliters of the polymerase from Thermus aquaticus was added to the reaction mixture and overlaid with a 100 µl mineral oil cap. The sample was then placed in the aluminum heating block of the liquid handling and heating instrument described above employing pipettes programmed to deliver liquids and a temperature-control device to effect temperature changes.

The DNA sample underwent 20 cycles of amplification in the machine, repeating the following program cycle:
1) heating from 37°C to 98°C in heating block over a period of 2.5 minutes; and
2) cooling from 98°C to 37°C over a period of three minutes to allow the primers and DNA to anneal.

After the last cycle, the sample was incubated for an additional 10 minutes at 55°C to complete the final extension reaction.

### V. Synthesis and Phosphorylation of Oligodeoxyribonucleotide Probes

A labeled DNA probe, designated RS24, of the following sequence was prepared: 5'-*CCCACAGGGCAGTAACGGCAGACTTCTCCTCAGGAGTCAG-3'  (RS24) where * indicates the label. This probe is 40 bases long, spans the fourth through seventeenth codons of the gene, and is complementary to the normal β-globin allele (β^{A}). The schematic diagram of primers and probes is given below:

110 bp
β-globin
PC03
RS24
PC04

This probe was synthesized according to the procedures described in Section I of Example I. The probe was labeled by contacting 20 pmole thereof with 4 units of T4 polynucleotide kinase and about 40 pmole γ-³²P-ATP (about 7000 Ci/mmole) in a 40 µl reaction volume containing 70 mM Tris buffer (pH 7.6), 10 mM MgCl₂, 1.5 mM spermine, and 10 mM dithiothreitol for 60 minutes at 37°C. The total volume was then adjusted to 100 µl with 25 mM EDTA and purified according to the procedure of Maniatis et al., supra, p. 466-467 over a 1 ml spin dialysis column equilibrated with Tris-EDTA (TE) buffer (10 mM Tris buffer, 0.1 mM EDTA, pH 8.0). TCA precipitation of the reaction product indicated that for RS24 the specific activity was 4.3 µCi/pmole and the final concentration was 0.118 pmole/µl.

### VI. Dot Blot Hybridizations

Four microliters of the amplified sample from Section IV and 5.6 µl of appropriate dilutions of β-globin plasm id DNA calculated to represent amplification efficiencies of 70, 75, 80, 85, 90, 95 and 100% were diluted with 200 µl 0.4 N NaOH, 25 mM EDTA and spotted onto a nylon filter by first wetting the filter with water, placing it in an apparatus for preparing dot blots which holds the filters in place, applying the samples, and rinsing each well with 0.1 ml of 20 x SSPE (3.6 M NaCl, 200 mM NaH₂PO₄, 20 mM EDTA), as disclosed by Reed and Mann, Nucleic Acids Research, 13, 7202-7221 (1985). Thefilters were then removed, rinsed in 20 × SSPE, and baked for 30 minutes at 80°C in a vacuum oven.

After baking, each filter was then contacted with 16 ml of a hybridization solution consisting of 3 x SSPE, 5 x Denhardt's solution (1 x = 0.02% polyvinylpyrrolidone, 0.02% Ficoll, 0.02% bovine serum albumin,, 0.2 mM Tris, 0.2 mM EDTA, pH 8.0), 0.5% SDS and 30% formamide, and incubated for two hours at 42°C. Then 2 pmole of probe RS24 was added to the hybridization solution and the filter was incubated for two minutes at 42°C.

Finally, each hybridized filter was washed twice with 100 ml of 2 x SSPE and 0.1% SDS for 10 minutes at room temperature. Then the filters were treated once with 100 ml of 2 x SSPE, 0.1% SDS at 60°C for 10 minutes.

Each filter was then autoradiographed, with the signal readily apparent after two hours.

### VII. Discussion of Autoradiogram

The autoradiogram of the dot blots was analyzed after two hours and compared in intensity to standard serial dilution β-globin reconstructions prepared with HaeIII/MaeI-digested pBR:β^{A}, where β^{A} is the wild-type allele, as described in Saiki et al., Science, supra. Analysis of the reaction product indicated that the overall amplification efficiency was about 95%, corresponding to a 630,000-fold increase in the β-globin target sequence.

### EXAMPLE II

### I. Amplification Reaction

Two 1 µg samples of genomic DNA extracted from the Molt 4 cell line as described in Example I were each diluted in a 100 µl reaction volume containing 50 mM KCl, 25 mM Tris·HCl buffer pH 8.0, 10 mM MgCl₂, 1 µM of primer PCO3, 1 µM of primer PC04, 200 µg/ml gelatin, 10% dimethylsulfoxide (by volume), and 1.5 mM each of dATP, dCTP, dGTP and TTP. After this mixture was heated for 10 minutes at 98°C to denature the genomic DNA, the samples were cooled to room temperature arid 4 µl of the polymerase from Thermus aquaticus described in Example I was added to each sample. The samples were overlaid with mineral oil to prevent condensation and evaporative loss.

One of the samples was placed in the heating block of the machine described in Example I and subjected to 25 cycles of amplification, repeating the following program cycle:
(1) heating from 37 to 93°C over a period of 2.5 minutes;
(2) cooling from 93°C to 37°C over a period of three minutes to allow the primers and DNA to anneal; and
(3) maintaining at 37°C for two minutes.

After the last cycle the sample was incubated for an additional 10 min utes at 60°C to complete the final extension reaction.

The second sample was placed in the heat-conducting container of a temperature cycling (heating and cooling) machine. In this machine, the heat-conducting container is attached to Peltier heat pumps which adjust the temperature upwards or downwards and a microprocessor controller to control automatically the amplification sequence, the temperature levels, the temperature ramping and the timing of the temperature.

The second sample was subjected to 25 cycles of amplification, repeating the following program cycle:
(1) heating from 37 to 95°C over a period of three minutes;
(2) maintaining at 95°C for 0.5 minutes to allow denaturation to occur;
(3) cooling from 95 to 37°C over a period of one minute; and
(4) maintaining at 37°C for one minute.

### II. Analysis

Two tests were done for analysis, a dot blot and an agarose gel analysis.

For the dot blot analysis, a labeled DNA probe, designated RS18, of the following sequence was prepared: 5'-*CTCCTGAGGAGAAGTCTGC-3' (RS18) where * indicates the label. This probe is 19 bases long, spans the fourth through seventeenth codons of the gene, and is complementary 10 the normal β-globin allele (β^{A}). The schematic diagram of primers and probes is given below:

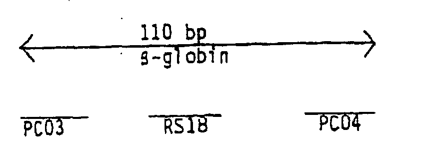
110 bp
β-globin

PC03 RS18 PC04

This probe was synthesized according to the procedures described in Section I of Example I. The probe was labeled by contacting 10 pmole thereof with 4 units of T4 polynucleotide kinase and about 40 pmole γ³²P-ATP (about 7000 Ci/mmole) in a 40 µl reaction volume containing 70 mM Tris·HCl buffer (pH 7.6), 10 mM MgCl₂, 1.5 mM spermine and 10 mM dithiothreitol for 60 minutes at 37°C. The total volume was then adjusted to 100 µl with 25 mM EDTA and purified according to the procedure of Maniatis et al., supra, p. 466-467 over a 1 ml spin dialysis column equilibrated with Tris-EDTA (TE) buffer (10 mM Tris·HCl buffer, 0.1 mM EDTA, pH 8.0). TCA precipitation of the reaction product indicated that for RS1 B the specific activity was 4.6 µCi/pmole and the final concentration was 0.114 pmole/µl.

Five microliters of the amplified sample from Section I and of a sample amplified as described above except using the Klenow fragment of E. coli DNA Polymerase I instead of the thermostable enzyme were diluted with 195 µl 0.4 N NaOH, 25 mM EDTA and spotted onto two replicate nylon filters by first wetting the filters with water, placing them in an apparatus for preparing dot blots which holds the filters in place, applying the samples, and rinsing each well with 0.4 ml of 20 x SSPE (3.6 M NaCl, 200 mM NaH₂PO₄, 20 mM EDTA), as disclosed by Reed and Mann, supra. The filters were then removed, rinsed in 20 x SSPE, and baked for 30 minutes at 80°C in a vacuum oven.

After baking, each filter was then contacted with 6 ml of a hybridization solution consisting of 5 x SSPE, 5 x Denhardt's solution (1 x = 0.02% polyvinylpyrrolidone, 0.02% Ficoll, 0.02% bovine serum albumin, 0.2 mM Tris, 0.2 mM EDTA, pH 8.0) and 0.5% SDS, and incubated for 60 minutes at 55°C. Then 5 µl of probe RS18 was added to the hybridization solution and the filter was incubated for 60 minutes at 55°C.

Finally, each hybridized filter was washed twice with 100 ml of 2 x SSPE and 0.1% SDS for 10 minutes at room temperature. Then the filters were treated twice more with 100 ml of 5 x SSPE, 0.1% SDS at 60°C for 1) one minute and 2) three minutes, respectively.

Each filter was then autoradiographed, with the signal readily apparent after 90 minutes.

In the agarose gel analysis, 5 µl each amplification reaction was loaded onto 4% NuSieve/0.5% agarose gel in 1 x TBE buffer (0.089 M Tris, 0.089 M boric acid, and 2 mM EDTA) and electrophoresed for 60 minutes at 100V. After staining with ethidium bromide, DNA was visualized by UV fluorescence.

The results show that the machines used in Example I and this example were equally effective in amplifying the DNA, showing discrete high-intensity 110-base pair bands of similar intensity, corresponding to the desired sequence, as well as afew other discrete bands of much lower intensity. In contrast, the amplification method that involves reagent transfer after each cycle using the Klenow fragment of E. coli Polymerase I gave a DNA smear resulting from the non-specific amplification of many unrelated DNA sequences.

It is expected that similar improvements in amplification and detection would be achieved in evaluating HLA-DQ, DR and DP regions.

If in the above experiments the amplification reaction buffer contains 2 mM MgCl₂ instead of 10 mM MgCl₂ and 150-200 µM of each nucleotide rather than 1.5 mM of each, and if the lower temperature of 37°C is raised to 45-58°C during amplification, better specificity and efficiency of amplification occurs. Also, DMSO was found not necessary or preferred for amplification.

### EXAMPLE III

### Amplification and Cloning

For amplification of a 119-base pair fragment on the human β-globin gene, a total of 1 microgram each of human genomic DNA isolated from the Molt 4 cell line or from the GM2064 cell line (representing a homozygous deletion of the β- and δ-hemoglobin region and available from the Human Genetic Mutant Cell Depository, Camden, NJ) as described above was amplified in a 100 µl reaction volume containing 50 mM KCl, 25 mM Tris·HCl pH 8, 10 mM MgCl₂, 200 µg/ml gelatin, 5 mM 2-mercaptoethanol, 1.5 mM each of dATP, dCTP, TTP, and dGTP, and 1 µM of each of the following primers: 
5'-CTTCTGcagCAACTGTGTTCACTAGC-3'  (GH18)
5'-CACaAgCTTCATCCACGTTCACC-3'  (GH19)
 where lower case letters denote mismatches from wild-type sequence to create restriction enzyme sites. GH18 is a . 26-base oligonucleotide complementary to the negative strand and contains an intemal PstI site. GH19 is a 29-base oligonucleotide complementary to the plus strand and contains an internal HindIII recognition sequence. These primers were selected by first screening the regions of the gene for homology to the PstI and HindIII restriction sites. The primers were then prepared as described in Example I.

The above reaction mixtures were heated for 10 minutes at 95°C and then cooled to room temperature. A total of 4 µl of the polymerase described in Example I was added to each reaction mixture, andthen each mixture was overlayed with mineral oil. The reaction mixtures were subjected to 30 cycles of amplification with the following program:
2.5 min. ramp, 37 to 98°C
3 min. ramp, 98 to 37°C
2 min. soak, 37°C

After the last cycle, the reaction mixtures were incubated for 20 minutes at 65°C to complete the final extension. The mineral oil was extracted with chloroform and the mixtures were stored at -20°C.

A total of 10 µl of the amplified product was digested with 0.5 µg M13mp10 cloning vector, which is publicly available, in a 50 µl volume containing 50 mM NaCl, 10 mM Tris·HCl, pH 7.8, 10 mM MgCl₂, 20 units PstI and 26 units Hindlll for 90 minutes at 37°C. The reaction was stopped by freezing at -20°C. The volume was adjusted to 110 µl with TE buffer and loaded (100 µl) onto a 1 ml BioGel P-4 spin dialysis column. One 0.1 ml fraction was collected and ethanol precipitated.

(At this point it was discovered that there was β-globin amplification product in the GM2064 sample. Subsequent experiments traced the source of contamination to the primers, either GH18 or GH19. Because no other source of primers was available, the experiment was continued with the understanding that some cloned sequences would be derived from the contaminating DNA in the primers.)

The ethanol pellet was resuspended in 15 µl water, then adjusted to 20 µl volume containing 50 mM Tris·HCl, pH 7.8, 10 mM MgCl₂, 0.5 mM ATP, 10 mM dithiothreitol, and 400 units ligase. [One unit is the amount of enzyme required to give 50% ligation of HindIII digested λDNA in 30 minutes at 16°C in 20 µl at a 5' termini concentration of 0.12 mM (about 330 µg/ml)]. This mixture was incubated for three hours at 16°C.

Ten microliters of ligation reaction mixture containing Molt 4 DNA was transformed into E. coli strain JM103 competent cells, which are publicly available. The procedure followed for preparing the transformed strain is described in Messing, J. (1981) Third Cleveland Symposium on Macromolecules:Recombinant DNA, ed. A. Walton, Elsevier, Amsterdam, 143-163. A total of 651 colorless plaques (and 0 blue plaques) were obtained. Of these, 119 had a (+)- strand insert (18%) and 19 had a (-)- strand insert (3%). This is an increase of almost 20-fold over the percentage of β-globin positive plaques among the primer-positive plaques from the amplification technique using Klenow fragment of E. coli Polymerase I, where the reaction proceeded for two minutes at 25°C, after which the steps of heating to 100°C for two minutes, cooling, adding Klenow fragment, and reacting were repeated nine times. These results confirm the improved specificity of the amplification reaction employing the thermostable enzyme herein.

In a later cloning experiment with GM2064 and the contaminated primers, 43 out of 510 colorless plaques (8%) had the (+)- strand insert. This suggests that approximately one-half of the 119 clones from Molt 4 contain the contaminant sequence.

Ten of the (+)- strand clones from Molt 4 were sequenced. Five were normal wild-type sequence and five had a single C to T mutation in the third position of the second codon of the gene (CAC to CAT). Four of the contaminant clones from GM2064 were sequenced and all four were normal.

Restriction site-modified primers may also be used to amplify and clone and partially sequence the human N-ras oncogene and to clone base pair segments of the HLA DQ-α, DQ-β and DR-β genes using the above technique.

Again, if the concentrations of MgCl₂ and nucleotides are reduced to 2 mM and 150-200 µM, respectively, and the minimum cycling temperature is increased from 37°C to 45-58°C, the specificity and efficiency of the amplification reaction can be increased.

### EXAMPLE IV

### Gene Retrieval

### A. IDENTIFICATION OF A DNA SEQUENCE PROBE FOR THE TAQ POLYMERASE GENE

A specific DNA sequence probe for the Taq pol gene was obtained following immunological screening of a λgt11 expression library. T. aquaticus DNA was digested to completion with Alul, ligated with EcoRI 12-mer linkers (CCG-GAATTCCGG, New England Biolabs), digested with EcoRI and ligated with dephosphorylated, EcoRI-digested λgt11 DNA (Promega Biotech). The ligated DNA was packaged (Gigapack Plus, Stratagene) and transfected into E. coli K-12 strain yl090 (provided by R. Young).

The initial library of 2 x 10⁵ plaques was screened (Young, R.A., and R.W. Davis (1983) Science, 222:778-782) with a 1:2000 dilution of a rabbit polyclonal antiserum raised to purified Taq polymerase (see Examples I and VI). Candidate plaques were replated at limiting dilution and rescreened until homogeneous (∼3 cycles). Phage were purified from candidate plaques which failed to react with preimmune serum and reacted with immune serum.

Candidate phage were used to lysogenize E. coli K-12 strain Y1089 (R. Young). Lysogens were screened for the production of an IPTG inducible fusion protein (larger than 3-galactosidase) which reacted with the Taq polymerase antiserum. Solid phase, size-fractionated fusion proteins were used to affinity purify epitope-specific antibodies from the total polyclonal antiserum (Goldstein, LS.B., et al. (1986) J. Cell Biol. 102:2076-2087).

The "fished", epitope-selected antibodies were used, in turn, in a Western analysis to identify which λgt11 phage candidates encoded DNA sequences uniquely specific to Taq polymerase. One λgt11 phage candidate, designated λgt:1, specifically selected antibodies from the total rabbit polyclonal Taq polymerase antiserum which uniquely reacted with both purified Taq polymerase and crude extract fractions containing Taq polymerase. This phage, λgt:1, was used for further study.

The ∼115 bp EcoRI-adapted Alul fragment of Thermus aquaticus DNA was labeled (Maniatis et al., supra) to generate a Taq polymerase-specific probe. The probe was used in Southern analyses and to screen a T. aquaticus DNA random genomic library.

### B. CONSTRUCTION AND SCREENING OF A THERMUS AQUATICUS RANDOM GENOMIC LIBRARY

Lambda phage Charon 35 (Wilhelmine, A. M. et al., supra) was annealed and ligated via its cohesive ends, digested to completion with BamHI, and the annealed arms were purified from the "stuffer" fragments by potassium acetate density gradient ultracentrifugation (Maniatis, et al., supra). T. aquaticus DNA was partially digested with Sau3A and the 15-20 kb size fraction purified by sucrose density gradient ultracentrifugation. The random genomic library was constructed by ligating the target and vector DNA fragments at a 1:1 molar ratio. The DNA was packaged and transfected into E. coli K-12 strains LE392 or KB02. A library of >20,000 initial phage containing >99% recombinants was amplified on E. coli K-12 strain LE392.

The CH35 Taq genomic phage library was screened (Maniatis et al., supra) with the radiolabeled EcoRI insert of λgt11:1. Specifically hybridizing candidate phage plaques were purified and further analyzed. One phage, designated Ch35::4-2, released ≥ four T. aquaticus DNA fragments upon digestion with HindIII (∼8.0, 4.5, 0.8, 0.58 kb)

The four HindIII T. aquaticus DNA fragments were ligated with HindIII digested plasmid BSM13⁺ (3.2 kb, Vector Cloning Systems, San Diego) and individually cloned following transformation of E. coli K-12 strain DG98.

The ∼8.0 kb HindIII DNA fragment from CH35::4-2 was isolated in plasmid pFC82 (11.2 kb), while the 4.5 kb HindIII DNA fragment from CH35::4-2 was isolated in plasmid pFC83 (7.7 kb).

E. coli strain DG98 harboring pFC82 was shown to contain a thermostable, high temperature DNA polymerase activity (Table 1). In addition, these cells synthesize a new ∼60 kd molecular weight polypeptide which is immunologically related to Taq DNA polymerase.

The Taq polymerase coding region of the 8.0 kb HindIII DNA fragment was further localized to the lac-promoter proximal 2.8 kb HindIII to Asp718 portion of the 8.0 kb HindIII fragment This region was subcloned to yield plasmid pFC85 (6.0 kb). Upon induction with IPTG, E. coli DG98 cells harboring plasmid pFC85 synthesize up to 100-fold more thermostable, Taq polymerase-related activity (Table 1) than the original parent clone (pFC82/DG98). While cells harboring pFC85 synthesize a significant amount of a thermostable DNA polymerase activity, only a portion of the Taq pol DNA sequence is translated, resulting in the accumulation of a ∼60 kd Taq polymerase-related polypeptide.

**TABLE 1**

| Expression of a Thermostable DNA Polymerase Activity in E. coli^{#} | | |
|---|---|---|
| Sample | Units*/ml | |
| | -IPTG | +IPTG |
| BSM13/DG98 | - | 0.02 |
| pFC82/DG98 | 2.2 | 2.7 |
| pFC85/DG98 | 11.9 | 643.8 |

| | | |
|---|---|---|
| ^{#}Cells were grown to late log phase (+/- IPTG, 10 mM), harvested, sonicated, heated at 75°C for 20 minutes, centrifuged and the clarified supernatant assayed at 70°C for DNA polymerase activity. | | |
| * 1 unit = 1 nM dCTP incorporated in 30 minutes. | | |

### EXAMPLE V

### Expression of Taq Polymerase

The gene encoding the thermostable polymerase of the present invention can be expressed in any of a variety of bacterial expression vectors including DG141 (ATCC 39588) and pP_{L}N_{RBS}ATG. Both of these host vectors are pBR322 derivatives that have either a sequence containing a tryptophan promoter-operator and ribosome binding site with an operably linked ATG start codon (DG141) or a sequence containing the lambda P_{L} promoter and gene N ribosome binding site operably linked to an ATG start codon (pP_{L}N_{RBS}ATG). Either one of these host vectors may be restricted with SacI, and blunt ended with Klenow or SI nuclease to construct a convenient restriction site for subsequent insertion of the Taq polymerase gene.

The full-length Taq polymerase gene was constructed from the DNA insert fragments subcloned into plasmids pFC83 and pFC85 as follows. Vector BSM13⁺ (commercially available from Vector Cloning Systems, San Diego, CA) was digested at the unique HindIII site, repaired with Klenow and dNTPs, and ligated with T4 DNA ligase to a BgIII octanucleotide linker, 5'-CAGATCTG-3', and transformed into E. coli strain DG98. Plasmids were isolated from Amp^{R} lacZα⁺ transformants. One of the clones was digested with BgIII and Asp718 restriction enzymes, and the large vector fragment purified by gel electrophoresis.

Next, plasmid pFC83 was digested with BgIII and HindIII and the ∼750 base pair fragment was isolated. Plasmid pFC85 was digested with HindIII and Asp718 and the ∼2.8 kb fragment isolated and joined in a three-piece ligation to the ∼750 base pair BgIII-HindII fragment from pFC83 and the BgIII-Asp718 vector fragment of BSM13⁺. This ligation mixture was used to transform E. coli strain DG98 (ATCC 39,768 deposited July 13, 1984) from which Amp^{R} colonies were selected and an ∼6.75 kilobase plasmid (pLSG1) was isolated. Isopropyl-β-D-thiogalactoside (IPTG)-induced. DG98 calls harboring pLSG1 synthesized Taq DNA polymerase indistinguishable in size from the native enzyme isolated from T. aquaticus. Plasmid pLSG1 can then be used to generate a single strand DNA template according to the procedure recommended by Vector Cloning Systems.

Oligonucleotide-directed mutagenesis (see Zoller and Smith, Nuc. Acids Res. (1982) 10:6487-6500) can then be used to introduce an SphI restriction site as part of the ATG start codon (upstream of the internal HindIII site in the coding sequence of the Taq polymerase gene). Similarly, a BgIII site can be introduced after the carboxyl-terminus of the gene (∼0.7 kb upstream from the Asp718 site) to facilitate subcloning of the Taq polymerase gene into an expression vector. After the site-directed mutagenesis is performed, the gene can be isolated from the BSM13⁺ vector on an ∼3.2 kb SphI-BstEII restriction fragment, treated with Klenow fragment and all fourdNTPs, and inserted with T4 DNA ligase (blunt-end conditions) into either one of the aforementioned expression vectors, which have been digested with SacI, repaired with Klenow and dNTPs, and treated with calf intestine phosphatase to generate dephosphorylated blunt ends. This ligation mixture is used to transform E. coli DG116 and the resulting transformants are screened for production of Taq polymerase. Expression of the enzyme can be confirmed by Western immunob lot analysis and activity analysis.

A greater proportion of the Taq polymerase gene contained within the ∼2.8 kb HindIII-Asp718 fragment of plasmid pFC85 can be expressed using, for example, plasmid pP_{L}N_{RBS}ATG, by operably linking the amino-terminal Hindlll restriction site encoding the Taq pol gene to an ATG initiation codon. The product of this fusion upon expression will yield an ∼66,000-68,000 dalton truncated polymerase.

This specific construction can be made by digesting plasmid pFC85 with Hindlll and treating with Klenow fragment in the presence of dATP, dGTP and dCTP. The resulting fragment is treated further with SI nuclease to remove any single-stranded extensions, and the resulting DNA digested with Asp718 and treated with Klenow fragment in the presence of all four dNTPs. The recovered fragment can be ligated using T4 DNA ligase to dephosphorylated plasmid pP_{L}N_{RBS}ATG, which had been digested with SacI and treated with Klenow fragment in the presence of dGTP to construct an ATG blunt end. This ligation mixture can then be used to transformed E. coli DG116 and the transformants screened for production of Taq polymerase. Again, expression can be confirmed by Western immunoblot analysis and activity analysis.

### EXAMPLE VI

### Purification

The thermostable polymerase may be purified directly from a culture of Thermus aquaticus following the example disclosed below or, alternatively, from a bacterial culture containing the recombinantly produced enzyme with only minor modifications necessary in the preparation of the crude extract.

After harvesting by centrifugation, 60 grams of cells were resuspended in 75 ml of a buffer consisting of 50 mM Tris-Cl pH 8, 1 mM EDTA. Cells were lysed in a French Press at 14,000-16,000 PSI after which 4 volumes (300 ml) of additional Tris-EDTA were added. Buffer A (β-mercaptoethanol to 5 mM and NP-40 and Tween 20 to 0.5% (v/v) each) was added and the solution was sonicated thoroughly while cooling. The resultant homogeneous suspension was diluted further with Buffer A such that the final volume was 7.5-8 times the starting cell weight; this was designated Fraction I.

The polymerase activity in Fraction I and subsequent fractions was determined in a 50 µl mixture containing 0.025 M TAPS-HCl pH 9.4 (20°C), 0.002 M MgCl₂, 0.05 M KCl, 1 mM 2-mercaptoethanol, 0.2 mM each dGTP, dATP, TTP, 0.1 mM dCTP [α-³²P, 05 Ci/mM), 12.5 µg "activated" salmon sperm DNA and 0.01-0.2 units of the polymerase (diluted in 10 mM Tris-HCl, pH 8, 50 mM KCI, 1 mg/ml autoclaved gelatin, 0.5% NP-40, 0.5% Tween 20, and 1 mM 2-mercaptoethanol). One unit corresponds to 10 nM product in 30 minutes. "Activated" DNA is a native preparation of DNA after partial hydrolysis with DNase I until 5% of the DNA was transferred to the acid-soluble fraction. The reaction was conducted at 74°C for 10 minutes and then 40 µl was transferred to 1.0 ml of 50 µg/ml carrier DNA in 2 mM EDTA at 0°C. An equal volume (1.0 ml) of 20% TCA, 2% sodium pyrophosphate was added. After 15-20 minutes at 0°C the samples were filtered through Whatman GF/C discs and extensively washed with cold 5% TCA-1% pyrophosphate, followed by cold 95% ethanol, dried and counted.

Fraction I was centrifuged fortwo hours at 35,000 rpm in a Beckman TI 45 rotor at 2°C and the collected supernatant was designated Fraction II.

The Taq polymerase activity was precipitated with Polymin P (BRL, Gaithersburg, MD) (10%, w/v, adjusted to pH 7.5 and autoclaved) after the minimum amount of Polymin P necessary to precipitate 90-95% of the activity was determined, which amount was generally found to be between 0.25% and 0.3% final volume.

An appropriate level of Polymin P was added slowly to Fraction II while stirring for 15 minutes at 0°C. This solution was centrifuged at 13,000 rpm for 20 minutes in a Beckman JA 14 rotor at 2°C. The supernatant was assayed for activity and the pellet Was resuspended in 1/5 volume of 0.5X Buffer A (diluted 1:2 with H₂O). This suspension was recentrifuged and the pellet resuspended in 1/4 volume of Buffer A containing 0.4 M KCl. This suspension was homogenized thoroughly and left overnight at 4°C. The homogenate was centrifuged as above and the collected supernatant designated Fraction III.

The protein fraction was collected by "precipitation" at 75% saturation of ammonium sulfate, centrifuged (at 27,000 rpm, SW27 rotor, 30 minutes) and the floating pellicle was resuspended in 50 mM Tris-Cl pH 8, 1 mM EDTA. These steps were repeated and the protein suspension was dialyzed extensively with P-cell buffer (20 mM KPO₄ pH 7.5, 0.5 mM EDTA, 5 mM β-mercaptoethanol, 5% (w/v) glycerol, 0.5% (v/v) NP-40 and Tween 20) containing 80 mM KCI.

The dialysate was transferred to a centrifuge bottle to which was added any recovered protein from sacks rinsed with the P-cell buffer containing 80 mM KCI. Centrifugation was performed at 20,000 x g and the time was reduced to 15 minutes. The supernatant was saved and any pellet remaining was washed, extracted with P-cell butter and 80 mM KCl, and recentrifuged. The supernatants were then combined to form Fraction IV.

Fraction IV was applied to a 2.2 x 22-cm column of phosphocellulose, equilibrated with the P-cell butter containing 80 mM KCI. The column was washed (2.5-3 column volumes) with the same buffer and the protein eluted using a linear gradient of 80 to 400 mM KCl in P-cell buffer. Fractions containing DNA polymerase activity (∼0.18-0.20 M KCI) were pooled and concentrated 3-4 fold on an Amicon stirred cell and YM30 membrane. The call was rinsed with the P-cell buffer without KCI and added to the fraction concentrate (0.15 M KCI adjusted final volume) to form Fraction V

Fraction V was applied to a 5 ml Heparin Sepharose CL-6B column (Pharmacia) equilibrated with P-cell buffer and 0.15 M KCI. The column was washed with 0.15 M KCI buffer (3-4 column volumes) and the protein eluted with a linear gradient from 0.15 to 0.65 M KCI in P-cell buffer. A 1:10 dilution into diluent without gelatin was made for SDS-PAGE analysis and a subsequent 1:20 dilution into diluent with 1 mg/ml gelatin was made for use in enzyme assays. The activity fractions (eluting at ∼0.3 M KCl) were assayed on supercoiled DNA template for specific and non-specific endonucleases/topoisomerase by electrophoretically detecting the change in molecular weight of supercoiled plasmid DNA after incubation with an excess of DNA polymerase. Exonuclease contamination was detected following incubation with small linear DNA fragments. In peak fractions, an ∼88 kd protein was found to be the major band. The major pool, designated Fraction VI, had the highest polymerase activity with minimal detectable endonuclease activity when this pool was assayed for 30 minutes at 55°C with ∼3-5 polymerase units/600 ng DNA.

Fraction VI was dialyzed against 10 mM KPO₄ pH 7.5, 5 mM β-mercaptoethanol, 5% glycerol, 0.2% NP-40, and 0.2% Tween 20 (HA buffer). The dialyzed sample was applied to a 3 ml column of hydroxyapatite and the enzyme eluted with a linear gradient of 10 to 250 mM KPO₄ pH 7.5, HA buffer. DNA polymerase activity began to elute at 75 mM KPO₄ with the peak at 100 mM KPO₄. Active peak fractions were assayed at 1:100-1:300 dilution. As in the prior chromatography step, a 1:10 dilution in diluent was prepared without gelatin for SDS-PAGE analysis. Fractions with no significant endonuclease or double-strand exonuclease when assayed at 55°C with 5 polymerase units were pooled and designated Fraction VII.

Fraction VII was dialyzed against a solution of 25 mM sodium acetate pH 5.2, 5% glycerol, 5 mM β-mercaptoethanol, 0.1 mM EDTA, 0.1% NP-40, and 0.1% Tween 20, adjusted to pH 5 at room temperature. The dialyzed sample was applied to a 2 ml DEAE-Tris-Acryl-M (LKB) column pre-equilibrated and subsequently washed with the same buffer. The fraction containing polymerase activity that did not adhere to the column was pooled and adjusted to 50 mM NeCl in the same buffer to yield Fraction VIII.

Fraction VIII was applied to a 2 ml CM-Tris-Acryl M (LKB) column equilibrated with the same buffer (25 mM sodium acetate, 50 mM NaCl, 5% glycerol, 0.1 mM EDTA, 0.1% NP-40, and 0.1% Tween 20). The column was washed with 4-5 column volumes of the same buffer and the enzyme eluted with a linear gradient from 50 to 400 mM NaCl in sodium acetate buffer. The polymerase activity peak eluted ∼0.15-0.20 M NaCl. The polymerase activity was assayed at 1: 300 to 1:500 dilution with the first dilution 1:10 into diluent without gelatin for the SDS-PAGE analysis. An assay across the activity peak on supercoiled DNA templates for specific and non-specific endonuclease/topoisomerase using DNA polymerase assay salts (25 mM TAPS-HCl pH 9.4,2.0 mM MgCl₂ and 50 mM KCl) at 74°C was performed, as well as assays for nucleases on M13 ss DNA and pBR322 fragments. Active fractions with no detectable nuclease(s) were pooled and run on a silver stained SDS-PAGE mini gel. The results show a single ∼88 kd band with a specific activity of ∼250,000 units/mg.

This specific activity is more than an order of magnitude higher than that claimed for the previously isolated Taq polymerase and is at least an order of magnitude higher than that for E. coli polymerase I.

### EXAMPLE VII

The Taq polymerase purified as described above in Example VI was found to be free of any contaminating Taq endonuclease and exonuclease activities. In addition, the Taq polymerase is preferably stored in storage buffer containing from about 0.1 to about 0.5% volume/volume of each non-ionic polymeric detergent employed. More preferably the storage bufferconsists of 50% (v/v) glycerol, 100 mM KCl, 20 mM Tris-Cl pH 8.0, 0.1 mM ethylenediaminetetraacetic acid (EDTA), 1 mM dithiothreitol, 0.5% v/v NP-40, 0.5% v/v Tween 20, and 200 µg/ml gelatin, and is preferably stored at -20°C.

The stored Taq polymerase was diluted in a buffer consisting of 25 mM Tris Cl pH 8.0, 20 mM KCI, 1 mM β-mercaptoethanol, 0.5% NP-40, 0.5% Tween-20, and 500 µg/ml gelatin. A reaction buffer was then prepared containing 50 mM KCI, 10 mM Tris-Cl, pH 8.3, 1.5 mM MgCl₂, 0.01% (w/v) gelatin, 200 µM each dNTP, 1 µM each of the primers that define a 500 base pair target sequence on a control template from bacteriophage λ, and 2.0-2.5 units Taq polymerase/assay in a final volume of 100 µl. Template was added to the reaction buffer, the sample placed in a 0.5 ml polypropylene tube, and the sample topped with 100 µl of heavy mineral oil to prevent evaporation.

At least a 10⁵-fold amplification was achieved when the following conditions were employed, using 1 ng of control template (bacteriophage λ DNA) where the target sequence represented approximately 1% of the starting mass of DNA.

First the template mixture was denatured for one minute, 30 seconds at 94°C by placing the tube in a heat bath. Then the tube was placed in a heat bath at 37°C for two minutes. Then the tube was placed in a heat bath at 72°C for three minutes, and then in the heat bath at 94°C for one minute. This cycle was repeated for a total of 25 cycles. At the end of the 25th cycle, the heat denaturation step at 94°C was omitted and replaced by extending the 72°C incubation step by an additional three minutes. Following termination of the assay, the samples were allowed to cool to room temperature and analyzed as described in previous examples.

The template may be optimally amplified with a different concentration of dNTPs and a different amount of Taq polymerase. Also, the size of the target sequence in the DNA sample will directly impact the minimum time required for proper extension (72°C incubation step). An optimization of the temperature cycling profile should be performed for each individual template to be amplified, to obtain maximum efficiency.

### EXAMPLE VIII

Taq polymerase purified as described above in Example I was formulated for storage as described in the previous example, but without the non-ionic polymeric detergents. When assayed for activity as described in that example, the enzyme storage mixture was found to be inactive. When the NP-40 and Tween 20 were added to the storage buffer, the full enzyme activity was restored, indicating that the presence of the non-ionic detergents is necessary to the stability of the enzyme formulation.

### EXAMPLE IX

Several 1 µg samples of human genomic DNA were subjected to 20-35 cycles of amplification as described in Example V, with equivalent units of either Klenow fragment or Taq polymerase, and analyzed by agarose gel electrophoresis and Southern blot The primers used in these reactions, PCO3 and PCO4, direct the synthesis of a 110-bp segment of the human beta-globin gene. The Klenow polymerase amplifications exhibited the smear of DNA typically observed with this enzyme, the apparent cause of which is the non-specific annealing and extension of primers to unrelated genomic sequences under what were essentially non-stringent hybridization conditions (1x Klenow salts at 37°C). Nevertheless, by Southern blot a specific 110-bp beta-globin target fragment was detected in all lanes. A substantially different electrophoretic pattern was seen in the amplifications done with Taq polymerase where the single major band is the 110-bp target sequence. This remarkable specificity was undoubtedly due to the temperature at which the primers were extended.

Although, like Klenow fragment amplifications, the annealing step was performed at 37°C, the temperature of Taq-catalyzed reactions had to be raised to about 70°C before the enzyme exhibited significant activity. During this transition from 37 to 70°C, poorly matched primer-template hybrids (which formed at 37°C) disassociated so that by the time the reaction reached an enzyme-activating temperature, only highly complementary substrate was available for extension. This specificity also results in a greater yield of target sequence than similar amplifications done with Klenow fragment because the non-specific extension products effectively compete for the polymerase, thereby reducing the amount of 110-mer that can be made by the Klenow fragment.

### EXAMPLE X

Amplification was carried out of a sample containing 1 µg Molt 4 DNA, 50 mM KCI, 10 mM Tris pH 8.3, 10 mM MgCl₂, 0.01% gelatin, 1 µM of each of the following primers (to amplify a 150 bp region): 
5'-CATGCCTCTTTGCACCATTC-3'(RS79) and
5'-TGGTAGCTGGATTGTAGCTG-3'(RS80)
 1.5 mM of each dNTP, and 5.0 units of Taq polymerase per 100 µl reaction volume. Three additional samples were prepared containing 2.5, 1.3, or 0.6 units of Taq polymerase. The amplification was carried out in the temperature cycling machine described above using the following cycle, for 30 cycles:
from 70 to 98°C for 1 minute
hold at 98°C for 1 minute
from 98°C to 35, 45 or 55°C for 1 minute
hold at 35, 45 or 55°C for 1 minute
from 35, 45 or 55°C to 70°C for 1 minute
hold at 70°C for 30 seconds

At 35°C annealing temperature, the 2.5 units/100 µl Taq enzyme dilution gave the best-signal-to noise ratio by agarose gel electrophoresis over all other Taq polymerase concentrations. At 45°C, the 5 units/100 µl Taq enzyme gave the best signal-to-noise ratio over the other concentrations. At 55°C, the 5 units/100 µl Taq enzyme gave the best signal-to-noise ratio over the other concentrations and over the 45°C annealing and improved yield. The Taq polymerase has more specificity and better yield at 55°C.

In a separate experiment the Molt 4 DNA was 10-fold serially diluted into the cell line GM2064 DNA, containing no β- or δ-globin sequences, available from the Human Genetic Mutant Cell Depository, Camden, New Jersey, at various concentrations representing varying copies per cell, and amplification was carried out on these samples as described in this example at ann ealing temperatures of 35°C and 55°C. At 35°C, the best that can be seen by agarose gel electrophoresis is 1 copy in 50 cells. At 55°C, the best that can be seen is 1/5,000 cells (a 100-fold improvement over the lower temperature), illustrating the importance of increased annealing temperature for Taq polymerase specificity under these conditions.

In a third experiment, DNA from a cell line 368H containing HIV-positive DNA, available from B. Poiesz, State University of New York, Syracuse, NY, was similarly diluted into the DNA from the SC1 cell line (deposited with ATCC on March 19, 1985; an EBV-transformed β cell line homozygous forthe sickle cell allele and lacking any HIV sequences) at various concentrations representing varying copies per cell, and amplification was carried out as described in this Example at annealing temperatures of 35°C and 55°C, using the primers SK3B and SK39, which amplify a 115 bp region of the HIV sequence:

5'-ATAATCCACCTATCCCAGTAGGAGAAAT-3'(SK38) and
5'-TTTGGTCCTTGTCTTATGTCCAGAATGC-3'(SK39)

The results by agarose gel electrophoresis showed that only the undiluted 368H sample could be detected with the annealing temperature at 35°C, whereas at least a 10⁻² dilution can be detected with the annealing temperature at 55°C, giving a 100-fold improvement in detection.

### EXAMPLE XI

cDNA was made from 1 µg of rabbit reticulocyte mRNA (Bethesda Research Laboratories) in a 100 µl reaction volume containing 150 mM KC1, 50 mM Tris-HCl (pH 8.3), 10 mM MgCl₂, 5 mM DTT, 0.5 mM dATP, 0.5 mM dCTP, 0.5 mM TTP, 0.5 mM dGTP, 0.2 µg oligo(dT)12-18 (Pharmacia), 40 units RNasin (Promega Biotec), and 5 units AMV reverse transcriptase (BRL) and incubated for 30 minutes at 42°C. The reaction was stopped by heating for 10 minutes at 95°C. Two µg RNase A was added to the sample (2 µl of a 2 mg/ml solution in water) and incubated for 10 minutes at 37°C.

Three amplification reactions were done with the Klenow fragment using different pairs of primers. The primer pair PC03/PC04 define a 110-bp product. The primer pair RS45/oligo(dT)25-30 define an about 370-bp product, and the primer pair PC03/oligo(dT)25-30 an about 600-bp product. PC03, PC04, and RS45 are complementary to the human β-globin gene and each has two mismatches with the rabbit gene. PC03 and PC04 are described in Example I. RS45 has the sequence:

5'-CAAGAAGGTGCTAGGTGCC-3'.

The amplification reactions were performed with 1/20th (5 µl) of the cDNA described above in a 100 µl reaction volume containing 50 mM NaCl, 10 mM Tris·HCl (pH 7.6), 10 mM MgCl₂, 200 µg/ml gelatin, 10% DMSO, 1 µM PCO3 or RS45, 1 µM PCO4 or oligo(dT)25-30, 1.5 mM dATP, 1.5 mM dCTP, 1.5 mM TTP and 1.5 mM dGTP. The samples were heated for five minutes at 98°C, then cooled to room temperature and overlayed with 100 µl mineral oil.

The samples were subjected to 10 cycles of automated amplification using the machine described in Example I and using the following program:
1) heating from 37°C to 98°C in a heating block over 2.5 minutes (denature);
2) cooling from 98°C to 37°C over 3.0 minutes (anneal);
3) adding 1 unit Klenow fragment; and
4) maintaining at 37°C for 20 minutes (extend).

The final volume of each sample was about 140 µl.

One-twentieth (7 µl) of each sample was analyzed by electrophoresis on a 2% agarose gel. After staining with ethidium bromide, discrete bands were seen in the PCO3/PCO4 and RS45/oligo(dT) samples. The sizes of the bands were consistent with the expected lengths: 110-bp for the former, about 370-bp for the latter. No evidence of amplification of an about 600-bp fragment with the PCO3/oligo(dT) primer pair was observed.

The contents of the gel were Southem blotted onto a Genatran nylon membrane and hybridized with a nick-translated human β-globin probe, pBR328:betaA, described in Saiki et al., Science, supra, using standard techniques. The resulting autoradiogram extended the conclusions reached previously - the 110 and about 370-bp fragments were β-globin specific amplification products and no significant amplification of the about 600-bp band was detected.

Three additional samples were amplified with the Taq polymerase obtained as described above using the same primer pairs described previously. Five microliter portions of cDNA were amplified in 100 µl reaction volumes containing 50 mM KCl, 25 mM Tns·HCl (pH 8.0), 10 mM MgCl₂, 200 µg/ml gelatin, 10% DMSO, 1 µM PCO3 or RS45, 1 µM PCO4 or oligo-(dT)25-30, 1.5 mM dATP, 1.5 mM dCTP, 1.5 mM TTP and 1.5 mM dGTP. The samples were heated for five minutes at 98°C, then cooled to room temperature. One microliter of Taq polymerase (1/8 dilution of lot 2) was added to each and overlayed with about 100 µl mineral oil.

The samples were subjected to 9 cycles of amplification in the Peltier device described in the previous example using the following program:
1) 1 min., 35 to 60°C ramp;
2) 12 min., 60 to 70°C ramp (extend);
3) 1 min., 70-95°C ramp (denature);
4) 30 sec., 95°C soak;
5) 1 min., 95 to 35°C ramp (anneal); and
6) 30 sec., 35°C soak

After the last cycle, the samples were incubated an additional 10 minutes at 70°C to complete the final (10th cycle) extension. The final volume of each was about 100 µl.

As before, 1/20th (10 µl) of each sample was analyzed on a 2% agarose gel. In this gel, amplification products were present in all three samples: 110-bp for PCO3/PCO4, about 370-bp for RS45/oligo(dT), and about 600-bp for PCO3/oligo(dT). These results were confirmed by Southern transfer and hybridization with the pBR328:betaA probe.

The production of the 600-bp product with Taq polymerase but not with the Klenow fragment is significant, and suggests that Taq polymerase is capable of producing longer DNA than the Klenow fragment

The following bacteriophage and bacterial strains were deposited with the Cetus Master Culture Collection, 1400 Fifty-Third Street, Emetyville, California, USA (CMCC) and with the American Type Culture Collection, 12301 Parklawn Drive, Rockville, Maryland, USA (ATCC). These deposits were made under the provisions of the Budapest Treaty on the International Recognition of the Deposit of Microorganisms for purposes of Patent Procedure and the Regulations thereunder (Budapest Treaty). This assures maintenance of a viable culture for 30 years from the date of deposit. The organisms will be made available by ATCC under the terms of the Budapest Treaty, and subject to an agreement between applicants and ATCC that assures unrestricted availability upon issuance of the pertinent U.S. patent. Availability of the deposited strains is not to be construed as a license to practice the invention in contravention of the rights granted under the authority of any government in accordance with its patent laws.

| Deposit Designation | CMCC No. | ATCC No. | Deposit |
|---|---|---|---|
| CH35:Taq#4-2 | 3125 | 40,336 | 6/3/87 |
| E. coli DG98/pFC83 | 3128 | 67,421 | 5/29/87 |
| E. coli DG98/pFC85 | 3127 | 67,422 | 5/29/87 |

In summary, the present invention is seen to provide a process for amplifying one or more specific nucleic acid sequences using a temperature-cycled chain reaction and a thermostable enzyme, in which reaction primer extension products are produced that can subsequently act as templates for further primer extension reactions. The process is especially useful in detecting nucleic acid sequences that are initially present in only very small amounts and in detecting nucleotide variations using sequence-specific oligonucleotides. Also, the amplification process herein can be used for molecular cloning.

The process herein results in increased yields of amplified product, greater specificity, and fewer steps necessary to carry out the amplification procedure, over what has been previously disclosed.

## Claims (Claims for the following Contracting State(s): BE, CH, LI, DE, FR, GB, GR, IT, LU, NL, SE)

1. A thermostable Thermus aquaticus polymerase that catalyses the combination of nucleoside triphosphates to form a nucleic acid strand complementary to a nucleic acid template strand, that has a molecular weight of 86,000 to 90,000 as determined according to its migration in SDS-PAGE, when the marker proteins are phosphorylase B (92,500), bovine serum albumin (66,200), ovalbumin (45,000), carbonic anhydrase (31,000), soybean trypsin inhibitor (21,500) and lysozyme (14,400).

2. The thermostable enzyme of claim 1 that is not irreversibly denatured when subjected to the elevated temperatures for the time necessary to effect denaturation of double-stranded nucleic acids.

3. The thermostable enzyme of claim 1 or 2 which is an enzyme derived from Thermus aquaticus YT1 (ATCC 25,104).

4. The thermostable enzyme of any one of claims 1 to 3 that has at least 50% of the activity at pH 6.4 that it has at pH 8.0.

5. A DNA sequence encoding a thermostable Thermus aquaticus DNA polymerase that catalyses the combination of nucleoside triphosphates to form a nucleic acid strand complementary to a nucleic acid template strand according to any one of claims 1 to 4 or a fragment of said DNA sequence encoding an enzymatically active, truncated thermostable enzyme having DNA polymerase activity.

6. The DNA sequence of claim 5, wherein said enzyme or fragment thereof is not irreversibly denatured when subjected to the elevated temperatures for the time necessary to effect denaturation of double-stranded nucleic acids.

7. The Thermus aquaticus DNA sequence of claim 5 that is contained within an approximately 3.5 kb BgIII-Asp718 (partial) restriction fragment of either the genome of Thermus aquaticus or the DNA of bacteriophage CH35:Taq#4-2 (ATCC 40,336).

8. The Thermus aquaticus DNA sequence of claim 5 that is contained within an approximately 2.8 kb HindIII-Asp718 restriction fragment of either the genome of Thermus aquaticus or the plasmid pFC85 (ATCC 67,421).

9. The DNA sequence of claim 7 or 8 which additionally has an ATG start codon.

10. The DNA sequence of any one of claims 7 to 9 which encodes a fusion protein containing said thermostable enzyme.

11. A recombinant vector comprising the DNA sequence or fragment of any one of claims 5 to 10.

12. The recombinant vector of claim 11 which is CH35:Taq#4-2 (ATCC 40,336), pFC83 (ATCC 67,422), pFC85 (ATCC 67,421) or a vector, the insert of which is composed of the about 750 bp BgIII/HindIII fragment of pFC83 and the approximately 2.8 kbp HindIII/Asp718 fragment of pFC85.

13. The recombinant vector of claim 11, wherein said DNA sequence or fragment is operably linked to an expression control sequence.

14. A recombinant host cell containing a vector of any one of claims 11 to 13.

15. The recombinant host cell of claim 14 which is E. coli.

16. A method for the production of a recombinant thermostable enzyme having DNA polymerase activity or fragment thereof having DNA polymerase activity, which catalyses the combination of nucleoside triphosphates to form a nucleic acid strand complementary to a nucleic acid template strand, said method comprising the culturing of a host cell of claim 14 or 15.

17. The method of claim 16, wherein said enzyme or fragment thereof is not irreversibly denatured when subjected to the elevated temperatures for the time necessary to effect denaturation of double-stranded nucleic acids.

18. A recombinant thermostable enzyme having DNA polymerase activity or a modification thereof having DNA polymerase activity that catalyzes the combination of nucleoside triphosphates to form a nucleic acid strand complementary to a nucleic acid template strand, said enzyme or modification thereof having a molecular weight of 86,000 to 90,000 as determined according to its migration in SDS-PAGE, when the marker proteins are phosphorylase B (92,500), bovine serum albumin (66,200), ovalbumin (45,000), carbonic anhydrase (31,000), soybean trypsin inhibitor (21,500) and lysozyme (14,400), said enzyme being obtainable by the method of claim 16 or 17, wherein said host cell is the recombinant host cell of claim 15.

19. A stable enzyme composition comprising a thermostable Thermus aquaticus DNA polymerase that catalyses the combination of nucleoside triphosphates to form a nucleic acid strand complementary to a nucleic acid template strand, that has a molecular weight of 86,000 to 90,000 as determined according to its migration in SDS-PAGE, when the marker proteins are phophorylase B (92,500), bovine serum albumin (66,200), ovalbumin (45,000), carbonic anhydrase (31,000), soybean trypsin inhibitor (21,500) and lysozyme (14,400) in a buffer comprising one or more non-ionic polymeric detergents.

20. A stable enzyme composition comprising a recombinant thermostable Thermus aquaticus DNA polymerase or fragment thereof having DNA polymerase activity
(a) obtained by the method of claim 16 or 17, or
(b) according to claim 18
in a buffer comprising one or more non-ionic polymeric detergents.

21. A stable enzyme composition comprising a recombinant thermostable Thermus aquaticus DNA polymerase or a modification thereof having DNA polymerase activity that catalyses the combination of nucleoside triphophates to form a nucleic acid strand complementary to a nucleic acid template strand, that has a molecular weight of 86,000 to 90,000 as determined according to its migration in SDS-PAGE, when the marker proteins are phosphorylase B (92,500), bovine serum albumin (66,200), ovalbumin (45,000), carbonic anhydrase (31,000), soybean trypsin inhibitor (21,500) and lysozyme (14,400) in a buffer comprising one or more non-ionic polymeric detergents.

22. The stable enzyme composition of claim 21, wherein the recombinant enzyme or modification thereof is not irreversibly denatured when subjected to the elevated temperatures for the time necessary to effect denaturation of double-stranded nucleic acids.

23. The stable enzyme composition of claim 21 or 22, wherein the recombinant enzyme or modification thereof has at least 50% of the activity at pH 6.4 that it has at pH 8.0.

24. The composition of any one of claims 19, 20 or 21, wherein the detergents are each present in a concentration of about 0.1% to about 0.5% volume/volume of the total composition.

25. The composition of any one of claims 19, 20, 21 or 24 wherein the detergents are a polyoxyethylated sorbitan monolaurate and an ethoxylated nonyl phenol.

26. The composition of any one of claims 19, 20, 21, 24 or 25 wherein the buffer comprises glycerol, Tris-Cl, pH 8.0, ethylenediamine tetraacetic acid, dithiothreitol, a polyoxyethylated sorbitan monolaurate, an ethoxylated nonyl phenol, and gelatin.

27. The use of a thermostable enzyme having DNA polymerase activity of any one of claims 1 to 4, of the recombinant thermostable enzyme of claim 18 or of a stable enzyme composition of any one of claims 19 to 21 or 24 to 26 for polymerase chain reactions.

28. A method for the amplification of nucleic acid sequences comprising the use of a thermostable enzyme having DNA polymerase activity of any one of claims 1 to 4, of the recombinant thermostable enzyme of claim 18 or of a stable enzyme composition of any one of claims 19 to 21 or 24 to 26.

## Claims (Claims for the following Contracting State(s): AT, ES)

1. A method for the preparation of a thermostable Thermus aquaticus DNA polymerase that catalyses the combination of nucleoside triphosphates to form a nucleic acid strand complementary to a nucleic acid template strand, that has a molecular weight of 86,000 to 90,000 as determined according to its migration in SDS-PAGE, when the marker proteins are phosphorylase B (92,500), bovine serum albumin (66,200), ovalbumin (45,000), carbonic anhydrase (31,000), soybean trypsin inhibitor (21,500) and lysozyme (14,400) said method comprising culturing a strain of Thermus aquaticus or a recombinant host cell harboring a DNA sequence encoding said DNA polymerase under suitable conditions and isolating said DNA polymerase from the culture.

2. The method of claim 1 wherein said thermostable enzyme is not irreversibly denatured when subjected to the elevated temperatures for the time necessary to effect denaturation of double-stranded nucleic acids.

3. The method of claim 1 or 2, wherein said thermostable enzyme is an enzyme derived from Thermus aquaticus YT1 (ATCC 25,104).

4. The method of any one of claims 1 to 3, wherein said thermostable enzyme has at least 50% of the activity at pH 6.4 that it has at pH 8.0.

5. A method for the preparation of a DNA sequence encoding a thermostable Thermus aquaticus DNA polymerase that catalyses the combination of nucleoside triphosphates to form a nucleic acid strand complementary to a nucleic acid template strand according to any one of claims 1 to 4 or a fragment of said DNA sequence encoding an enzymatically active, truncated thermostable enzyme having DNA polymerase activity, said method comprising the following steps:
(a) identification of a DNA sequence probe for the thermostable enzyme by immunologlical screening of an expression library containing the genome of Thermus aquaticus with antibodies to the Taq thermostable polymerase;
(b) construction of a genomic library of the target DNA;
(c) screening of the genomic library with the radiolabeled probe obtained by step (a); and
(d) isolating phages containing DNA encoding said thermostable enzyme.

6. The method of claim 5, wherein said enzyme or fragment thereof is not irreversibly denatured when subjected to the elevated temperatures for the time necessary to effect denaturation of double-stranded nucleic acids.

7. The method of claim 6, wherein as a probe a portion of the genomic DNA of Thermus aquaticus encoding at least six amino acids of the Taq polymerase is used.

8. The method of claim 7, wherein said probe is selected from the following probes:
(a) the ∼115 bp Eco-RI-adapted Alul Thermus aquaticus DNA fragment of pFC85 (ATCC 67,421);
(b) the ∼750 bp BgIII-HindIII Thermus aquaticus DNA fragment of pFC83 (ATCC 67,422);
(c) the HindIII-BamHI Thermus aquaticus DNA fragment of the insert of pFC85 (ATCC 67,421);
(d) the ∼2.8 kb HindIII-Asp718 Thermus aquaticus DNA fragment of pFC85 (ATCC 67,421); and
(e) the BamHI-NheI Thermus aquaticus DNA fragment of the insert of pFC85 (ATCC 67,421).

9. The method of claim 5, wherein the Thermus aquaticus DNA sequence is contained within an approximately 3.5 kb BgIII-Asp718 (partial) restriction fragment of either the genome of Thermus aquaticus or the DNA of bacteriophage CH35:Taq#4-2 (ATCC 40,336).

10. The method of claim 5, wherein the Thermus aquaticus DNA sequence is contained within an approximately 2.8 kb HindIII-Asp718 restriction fragment of either the genome of Thermus aquaticus or the plasmid pFC85 (ATCC 67,421).

11. The method of any one of claims 5 to 10, wherein the DNA sequence additionally has an ATG start codon.

12. The method of any one of claims 5 to 11, wherein the DNA sequence encodes a fusion protein containing said thermostable enzyme.

13. A method for the preparation of a recombinant vector comprising the insertion of the DNA sequence or fragment of any one of claims 5 to 12 into a suitable vector.

14. The method of claim 13, wherein the recombinant vector is CH35:Taq#4-2 (ATCC 40,336), pFC83 (ATCC 67,422), pFC85 (ATCC 67,421) or a vector, the insert of which is composed of the about 750 bp BgIII/HindIII fragment of pFC83 and the approximately 2.8 kbp HindIII/Asp718 fragment of pFC85.

15. The method of claim 13, wherein said DNA sequence or fragment is operably linked to an expression control sequence.

16. A recombinant host cell containing a vector of any one of claims 13 to 15.

17. The recombinant host cell of claim 16 which is E. coli.

18. A method for the production of a recombinant thermostable enzyme having DNA polymerase activity, fragment or modification thereof having DNA polymerase activity, which catalyses the combination of nucleoside triphosphates to form a nucleic acid strand complementary to a nucleic acid template strand, said method comprising the culturing of a host cell of claim 16 or 17.

19. The method of claim 18, wherein said enzyme or modification thereof have a molecular weight of 86,000 to 90,000 as determined according to its migration in SDS-PAGE, when the marker proteins are phosphorylase B (92,500), bovine serum albumin (66,200), ovalbumin (45,000), carbonic anhydrase (31,000), soybean trypsin inhibitor (21,500) and lysozyme (14,400).

20. The method of claim 18 or 19 wherein the recombinant enzyme, fragment or modification thereof are free from contaminating thermostable deoxyribonuclease activity.

21. The method of any one of claims 18 to 20, wherein said recombinant enzyme, fragment or modification thereof are not irreversibly denatured when subjected to the elevated temperatures for the time necessary to effect deriaturation of double-stranded nucleic acids.

22. The method of any one of claims 18 to 21, wherein the recombinant enzyme, fragment or modification have at least 50% of the activity at pH 6.4 that it has at pH 8.0.

23. A method for the preparatio of a stable enzyme composition comprising combining a thermostable Thermus aquaticus DNA polymerase prepared according to any one of claims 1 to 4, or a recombinant thermostable enzyme, fragment or modification thereof having DNA polymerase activity obtained by the method of any one of claims 18 to 22 with a buffer comprising one or more non-ionic polymeric detergents.

24. The method of claim 23, wherein the detergents are each present in a concentration of about 0.1% to about 0.5% volume/volume of the total composition.

25. The method of claim 23 or 24, wherein the detergents are a polyoxyethylated sorbitan monolaurate and an ethoxylated nonyl phenol.

26. The method of any one of claims 23 to 25, wherein the buffer comprises glycerol, Tris-Cl, pH 8.0, ethylenediamine tetraacetic acid, dithiothreitol, a polyoxyethylated sorbitan monolaurate, an ethoxylated nonyl phenol, and gelatin.

27. A thermostable Thermus aquaticus DNA polymerase that catalyses the combination of nucleoside triphosphates to form a nucleic acid strand complementary to a nucleic acid template strand, that has a molecular weight of 86,000 to 90,000 as determined according to its migration in SDS-PAGE, when the marker proteins are phosphorylase B (92,500), bovine serum albumin (66,200), ovalbumin (45,000), carbonic anhydrase (31,000), soybean trypsin inhibitor (21,500) and lysozyme (14,400).

28. The thermostable enzyme of claim 27 that is not irreversibly denatured when subjected to the elevated temperatures for the time necessary to effect denaturation of double-stranded nucleic acids.

29. The thermostable enzyme of claim 27 or 28 which is an enzyme derived from Thermus aquaticus YT1 (ATCC 25,104).

30. The thermostable enzyme of any one of claims 27 to 29 that has at least 50% of the activity at pH 6.4 that it has at pH 8.0.

31. The thermostable enzyme of claims 27 to 30 obtainable by a method as claimed in claim 1.

32. A DNA sequence encoding a thermostable Thermus aquaticus DNA polymerase that catalyses the combination of nucleoside triphosphates to form a nucleic acid strand complementary to a nucleic acid template strand according to any one of claims 27 to 31 or a fragment of said DNA sequence encoding an enzymatically active, truncated thermostable enzyme having DNA polymerase activity

33. The DNA sequence of claim 32, wherein said enzyme or fragment thereof is not irreversibly denatured when subjected to the elevated temperatures for the time necessary to effect denaturation of double-stranded nucleic acids.

34. The Thermus aquaticus DNA sequence of claim 32 that is contained within an approximately 3.5 kb BgIII-Asp718 (partial) restriction fragment of either the genome of Thermus aquaticus or the DNA of bacteriophage CH35:Taq#4-2 (ATCC 40,336).

35. The Thermus aquaticus DNA sequence of claim 32 that is contained within an approximately 2.8 kb HindIII-Asp718 restriction fragment of either the genome of Thermus aquaticus or the plasmid pFC85 (ATCC 67,421).

36. The DNA sequence of any one of claims 32 to 35 which additionally has an ATG start codon.

37. The DNA sequence of any one of claims 32 to 36 which encodes a fusion protein containing said thermostable enzyme.

38. A recombinant vector comprising the DNA sequence or fragment of any one of claims 32 to 37.

39. The recombinant vector of claim 38 which is CH35:Taq#4-2 (ATCC 40,336), pFC83 (ATCC 67,422), pFC85 (ATCC 67,421) or a vector, the insert of which is composed of the about 750 bp BgIII/HindIII fragment of pFC83 and the approximately 2.8 kbp HindIII/Asp718 fragment of pFC85.

40. The recombinant vector of claim 38, wherein said DNA sequence or fragment is operably linked to an expression control sequence.

41. A recombinant thermostable enzyme having DNA polymerase activity or a modification thereof having DNA polymerase activity that catalyzes the combination of nucleoside triphosphates to form a nucleic acid strand complementary to a nucleic acid template strand, said enzyme or modification thereof having a molecular weight of 86,000 to 90,000 as determined according to its migration in SDS PAGE, when the marker proteins are phosphorylase B (92,500), bovine serum albumin (66,200), ovalbumin (45,000), carbonic anhydrase (31,000), soybean trypsin inhibitor (21,500) and lysozyme (14,400), said enzyme being obtainable by the method of any one of claims 18 to 22, wherein said host cell is the recombinant host cell of claim 17.

42. A stable enzyme composition comprising a thermostable Thermus aquaticus DNA polymerase that catalyses the combination of nucleoside triphosphates to form a nucleic acid strand complementary to a nucleic acid template strand, that has a molecular weight of 86,000 to 90,000 as determined according to its migration in SDS-PAGE, when the marker proteins are phophorylase B (92,500), bovine serum albumin (66,200), ovalbumin (45,000), carbonic anhydrase (31,000), soybean trypsin inhibitor (21,500) and lysozyme (14,400) in a buffer comprising one or more non-ionic polymeric detergents.

43. A stable enzyme composition comprising a recombinant thermostable Thermus aquaticus DNA polymerase or fragment thereof having DNA polymerase activity obtained by
(a) the method of any one of claims 18 to 22, or
(b) according to claim 41
in a buffer comprising one or more non-ionic polymeric detergents.

44. A stable enzyme composition comprising a recombinant thermostable Thermus aquaticus DNA polymerase or a modification thereof having DNA polymerase activity that catalyses the combination of nucleoside triphophates to form a nucleic acid strand complementary to a nucleic acid template strand, that has a molecular weight of 86,000 to 90,000 as determined according to its migration in SDS-PAGE, when the marker proteins are phosphorylase B (92,500), bovine serum albumin (66,200), ovalbumin (45,000), carbonic anhydrase (31,000), soybean trypsin inhibitor (21,500) and lysozyme (14,400) in a buffer comprising one or more non-ionic polymeric detergents.

45. The stable enzyme composition of claim 44, wherein the recombinant enzyme or modification thereof is not irreversibly denatured when subjected to the elevated temperatures for the time necessary to effect denaturation of double-stranded nucleic acids.

46. The stable enzyme composition of any one of claims 44 or 45, wherein the recombinant enzyme or modification thereof has at least 50% of the activity at pH 6.4 that it has at pH 8.0.

47. The composition of any one of claims 42, 43 or 44 wherein the detergents are each present in a concentration of about 0.1% to about 0.5% volume/volume of the total composition.

48. The composition of any one of claims 42, 43, 44 or 47, wherein the detergents are a polyoxyethylated sorbitan monolaurate and an ethoxylated nonyl phenol.

49. The composition of any one of claims 42, 43, 44, 47 or 48, wherein the buffer comprises glycerol, Tris-Cl, pH 8.0, ethylenediamine tetraacetic acid, dithiothreitol, a polyoxyethylated sorbitan monolaurate, an ethoxylated nonyl phenol, and gelatin.

50. The use of a thermostable enzyme having DNA polymerase activity of any one of claims 27 to 31, of the recombinant thermostable enzyme of claim 41 or of a stable enzyme composition of any one of claims 42 to 44 or 47 to 49 for polymerase chain reactions.

51. A method for the amplification of nucleic acid sequences comprising the use of a thermostable enzyme having DNA polymerase activity of any one of claims 27 to 31, of the recombinant thermostable enzyme of claim 41 or of a stable enzyme composition of any one of claims 42 to 44 or 47 to 49.

## Patentansprüche (Patentansprüche für folgende(n) Vertragsstaat(en): BE, CH, LI, DE, FR, GB, GR, IT, LU, NL, SE)

1. Thermostabile Thermus aquaticus-DNA-Polymerase, die die Verknüpfung von Nucleosidtriphosphaten zur Bildung eines zu einem Nucleinsäure-Matrizenstrang komplementären Nucleinsäurestrangs katalysiert, die ein Molekulargewicht von 86.000 bis 90.000 besitzt, bestimmt gemäß ihrer Wanderung in SDS-PAGE, wenn die Markerproteine Phosphorylase B (92.500), Rinderserumalbumin (66.200), Ovalbumin (45.000), Carboanhydrase (31.000), Sojabohnen-Trypsininhibitor (21.500) und Lysozym (14.400) sind.

2. Thermostabiles Enzym nach Anspruch 1, das nicht irreversibel denaturiert wird, wenn es für den zur Erzielung einer Denaturierung von doppelsträngigen Nucleinsäuren benötigten Zeitraum erhöhten Temperaturen ausgesetzt wird.

3. Thermostabiles Enzym nach Anspruch 1 oder 2, das ein von Thermus aquaticus YT1 (ATCC 25,104) stammendes Enzym ist.

4. Thermostabiles Enzym nach einem der Ansprüche 1 bis 3, das bei einem pH-Wert von 6,4 mindestens 50% der Aktivität besitzt, die es bei einem pH-Wert von 8,0 hat.

5. DNA-Sequenz, die eine thermostabile Thermus aquaticus-DNA-Polymerase codiert, die die Verknüpfung von Nucleosidtriphosphaten zur Bildung eines zu einem Nucleinsäure-Matrizenstrang komplementären Nucleinsäurestrangs katalysiert, nach einem der Ansprüche 1 bis 4 oder ein Fragment der DNA-Sequenz, das ein enzymatisch aktives, verkürztes thermostabiles Enzym mit DNA-Polymerase-Aktivität codiert.

6. DNA-Sequenz nach Anspruch 5, wobei das Enzym oder Fragment davon nicht irreversibel denaturiert wird, wenn es für den zur Erzielung einer Denaturierung von doppelsträngigen Nucleinsäuren benötigten Zeitraum erhöhten Temperaturen ausgesetzt wird.

7. Thermus aquaticus-DNA-Sequenz nach Anspruch 5, die in einem nach (partieller) Spaltung gewonnenen, etwa 3,5 kb großen BgIII-Asp718-Restriktionsfragment des Genoms von Thermus aquaticus oder der DNA des Bakteriophagen CH35:Taq#4-2 (ATCC 40,336) enthalten ist.

8. Thermus aquaticus-DNA-Sequenz nach Anspruch 5, die in einem etwa 2,8 kb großen HindIII-Asp718-Restriktionsfragment des Genoms von Thermus aquaticus oder des Plasmids pFC85 (ATCC 67,421) enthalten ist.

9. DNA-Sequenz nach Anspruch 7 oder 8, die zusätzlich ein ATG-Startcodon aufweist.

10. DNA-Sequenz nach einem der Ansprüche 7 bis 9, die ein Fusionsprotein codiert, das das thermostabile Enzym enthält.

11. Rekombinanter Vektor, umfassend die DNA-Sequenz oder das Fragment nach einem der Ansprüche 5 bis 10.

12. Rekombinanter Vektor nach Anspruch 11, der CH35:Taq#4-2 (ATTC 40,336), pFC83 (ATCC 67,422), pFC85 (ATCC 67,421) oder ein Vektor ist, dessen Insertion zusammengesetzt ist aus dem etwa 750 bp großen BgIII/HindIII-Fragment von pFC83 und dem etwa 2,8 kbp großen HindIII/Asp718-Fragment von pFC85.

13. Rekombinanter Vektor nach Anspruch 11, wobei die DNA-Sequenz oder das Fragment mit einer Expressions-Kontrollsequenz funktionell verbunden ist.

14. Rekombinante Wirtszelle, enthaltend einen Vektor nach einem der Ansprüche 11 bis 13.

15. Rekombinante Wirtszelle nach Anspruch 14, die E. coli ist.

16. Verfahren zur Herstellung eines rekombinanten thermostabilen Enzyms mit DNA-Polymerase-Aktivität oder eines Fragments davon mit DNA-Polymerase-Aktivität, das die Verknüpfung von Nucleosidtriphosphaten zur Bildung eines zu einem Nucleinsäure-Matrizenstrang komplementären Nucleinsäurestrangs katalysiert, wobei das Verfahren die Züchtung einer Wirtszelle nach Anspruch 14 oder 15 umfasst.

17. Verfahren nach Anspruch 16, wobei das Enzym oder Fragment davon nicht irreversibel denaturiert wird, wenn es für den zur Erzielung einer Denaturierung von doppelsträngigen Nucleinsäuren benötigten Zeitraum erhöhten Temperaturen ausgesetzt wird.

18. Rekombinantes thermostabiles Enzym mit DNA-Polymerase-Aktivität oder eine Modifikation davon mit DNA-Polymerase-Aktivität, das (die) die Verknüpfung von Nucleosidtriphosphaten zur Bildung eines zu einem Nudeinsäure-Matrizenstrang komplementären Nucleinsäurestrangs katalysiert, wobei das Enzym oder die Modifikation davon ein Molekulargewicht von 86.000 bis 90.000 besitzen, bestimmt gemäß deren Wanderung in SDS-PAGE, wenn die Markerproteine Phosphorylase B (92.500), Rinderserumalbumin (66.200), Ovalbumin (45.000), Carboanhydrase (31.000), Sojabohnen-Trypsininhibitor (21.500) und Lysozym (14.400) sind, wobei das Enzym durch das Verfahren von Anspruch 16 oder 17 erhältlich ist, wobei die Wirtszelle die rekombinante Wirtszelle nach Anspruch 15 ist.

19. Stabile Enzymzusammensetzung, umfassend eine thermostabile Thermus aquaticus-DNA-Polymerase, die die Verknüpfung von Nucleosidtriphosphaten zur Bildung eines zu einem Nucleinsäure-Matrizenstrang komplementären Nucleinsäurestrangs katalysiert, die ein Molekulargewicht von 86.000 bis 90.000 besitzt, bestimmt gemäß ihrer Wanderung in SDS-PAGE, wenn die Markerproteine Phosphorylase B (92.500), Rinderserumalbumin (66.200), Ovalbumin (45.000), Carboanhydrase (31.000), Sojabohnen-Trypsininhibitor (21.500) und Lysozym (14.400), sind in einem Puffer, umfassend ein oder mehrere nicht-ionische(s) polymere(s) Detergenz/Detergenzien.

20. Stabile Enzymzusammensetzung, umfassend eine rekombinante thermostabile Termus aquaticus-DNA-Polymerase oder ein Fragment davon mit DNA-Polymerase-Aktivität
(a) erhalten durch das Verfahren nach Anspruch 16 oder 17, oder
(b) nach Anspruch 18
in einem Puffer, umfassend ein oder mehrere nicht-ionische(s) polymere(s) Detergenz/Detergenzien.

21. Stabile Enzymzusammensetzung, umfassend eine rekombinante thermostabile Thermus aquaticus-DNA-Polymerase oder eine Modifikation davon mit DNA-Polymerase-Aktivität, die die Verknüpfung von Nucleosidtriphosphaten zur Bildung eines zu einem Nucleinsäure-Matrizenstrang komplementären Nucleinsäurestrangs katalysiert, wobei das Enzym oder die Modifikation davon ein Molekulargewicht von 86.000 bis 90.000 besitzen, bestimmt gemäß deren Wanderung in SDS-PAGE, wenn die Markerproteine Phosphorylase B (92.500), Rinderserumalbumin (66.200), Ovalbumin (45.000), Carboanhydrase (31.000), Sojabohnen-Trypsininhibitor (21.500) und Lysozym (14.400) sind, in einem Puffer, umfassend ein oder mehrere nicht-ionische(s) polymere(s) Detergenz/Detergenzien.

22. Stabile Enzymzusammensetzung nach Anspruch 21, wobei das rekombinante Enzym oder die Modifikation davon nicht irreversibel denaturiert werden, wenn sie für den zur Erzielung einer Denaturierung von doppelsträngigen Nucleinsäuren benötigten Zeitraum erhöhten Temperaturen ausgesetzt werden.

23. Stabile Enzymzusammensetzung nach Anspruch 21 oder 22, wobei das rekombinante Enzym oder die Modifikation davon bei einem pH-Wert von 6,4 mindestens 50% der Aktivität besitzen, die sie bei einem pH-Wert von 8,0 haben.

24. Zusammensetzung nach einem der Ansprüche 19, 20 oder 21, wobei die Detergenzien jeweils in einer Konzentration von etwa 0,1% bis etwa 0,5% (Vol./Vol.) der Gesamtzusammensetzung vorhanden sind.

25. Zusammensetzung nach einem der Ansprüche 19, 20, 21 oder 24, wobei die Detergenzien ein polyoxyethyliertes Sorbitanmonolaurat und ein ethoxyliertes Nonylphenol sind.

26. Zusammensetzung nach einem der Ansprüche 19, 20, 21, 24 oder 25, wobei der Puffer Glycerin, Tris-Cl, pH-Wert 8,0, Ethylendiamintetraessigsäure, Dithiothreit, ein polyoxyethyliertes Sorbitanmonolaurat, ein ethoxyliertes Nonylphenol und Gelatine umfasst.

27. Verwendung eines thermostabilen Enzyms mit DNA-Polymerase-Aktivität nach einem der Ansprüche 1 bis 4, des rekombinanten thermostabilen Enzyms nach Anspruch 18, oder einer stabilen Enzymzusammensetzung nach einem der Ansprüche 19 bis 21 oder 24 bis 26 für Polymerasekettenreaktionen.

28. Verfahren zur Amplifikation von Nucleinsäuresequenzen, umfassend die Verwendung eines thermostabilen Enzyms mit DNA-Polymerase-Aktivität nach einem der Ansprüche 1 bis 4, des rekombinanten thermostabilen Enzyms nach Anspruch 18 oder einer stabilen Enzymzusammensetzung nach einem der Ansprüche 19 bis 21 oder 24 bis 26.

## Patentansprüche (Patentansprüche für folgende(n) Vertragsstaat(en): AT, ES)

1. Verfahren zur Herstellung einer thermostabilen Thermus aquaticus-DNA-Polymerase, die die Verknüpfung von Nucleosidtriphosphaten zur Bildung eines zu einem Nucleinsäure-Matrizenstrang komplementären Nucleinsäurestrangs katalysiert, die ein Molekulargewicht von 86.000 bis 90.000 besitzt, bestimmt gemäß ihrer Wanderung in SDS-PAGE, wenn die Markerproteine Phosphorylase B (92.500), Rinderserumalbumin (66.200), Ovalbumin (45.000), Carboanhydrase (31.000), Sojabohnen-Trypsininhibitor (21.500) und Lysozym (14.400) sind, wobei das Verfahren die Züchtung eines Stamms von Thermus aquaticus oder einer rekombinanten Wirtszelle, die eine DNA-Sequenz enthält, die die DNA-Polymerase codiert, unter geeigneten Bedingungen umfasst, und die Isolierung der DNA-Polymerase aus der Kultur.

2. Verfahren nach Anspruch 1, wobei das thermostabile Enzym nicht irreversibel denaturiert wird, wenn es für den zur Erzielung einer Denaturierung von doppelsträngigen Nucleinsäuren benötigten Zeitraum erhöhten Temperaturen ausgesetzt wird.

3. Verfahren nach Anspruch 1 oder 2, wobei das thermostabile Enzym ein aus Thermus aquaticus YT1 (ATCC 25,104) stammendes Enzym ist.

4. Verfahren nach einem der Ansprüche 1 bis 3, wobei das thermostabile Enzym bei einem pH-Wert von 6,4 mindestens 50% der Aktivität besitzt, die es bei einem pH-Wert von 8,0 hat.

5. Verfahren zur Herstellung einer DNA-Sequenz, die eine thermostabile Thermus aquaticus-DNA-Polymerase codiert, die die Verknüpfung von Nucleosidtriphosphaten zur Bildung eines zu einem Nucleinsäure-Matrizenstrang komplementären Nucleinsäurestrangs katalysiert, nach einem der Ansprüche 1 bis 4 oder eines Fragments der DNA-Sequenz, das ein enzymatisch aktives, verkürztes thermostabiles Enzym mit DNA-Polymerase-Aktivität codiert, wobei das Verfahren die folgenden Schritte umfasst:
(a) Identifizierung einer DNA-Sequenz-Sonde für das thermostabile Enzym durch immunologisches Screening einer Expressions-Genbank, die das Genom von Thermus aquaticus enthält, mit Antikörpern gegen die thermostabile Taq-Polymerase;
(b) Konstruktion einer genomischen Genbank der Ziel-DNA;
(c) Screening der genomischen Genbank mit der radioaktiv markierten Sonde, die durch Schrittt (a) gewonnen wurde; und
(d) Isolierung von Phagen, die DNA enthalten, die das thermostabile Enzym codiert.

6. Verfahren nach Anspruch 5, wobei das Enzym oder Fragment davon nicht irreversibel denaturiert werden, wenn sie für den zur Erzielung einer Denaturierung von doppelsträngigen Nucleinsäuren benötigten Zeitraum erhöhten Temperaturen ausgesetzt werden.

7. Verfahren nach Anspruch 6, wobei als Sonde ein Anteil der genomischen DNA von Thermus aquaticus, die mindestens sechs Aminosäuren der Taq-Polymerase codiert, verwendet wird.

8. Verfahren nach Anspruch 7, wobei die Sonde aus folgenden Sonden ausgewählt ist:
(a) das ∼ 115 bp Eco-RI-adaptierte Alul-Thermus aquaticus-DNA-Fragment von pFC85 (ATCC 67,421);
(b) das ∼ 750 bp BaIII-HindIII-Thermus aquaticus-DNA-Fragment von pFC83 (ATCC 67,422),
(c) das HindIII-BamHI-Thermus aquaticus-DNA-Fragment der Insertion von pFC85 (ATCC 67,421);
(d) das ∼ 2,8 kb HindIII-Asp718-Thermus aquaticus-DNA-Fragment von pFC85 (ATCC 67,421); und
(e) das BamHI-NheI-Thermus aquaticus-DNA-Fragment der Insertion von pFC85 (ATCC 67,421).

9. Verfahren nach Anspruch 5, wobei die Thermus aquaticus-DNA-Sequenz in einem nach (partieller) Spaltung gewonnenen, etwa 3,5 kb großen BgIII-Asp718-Restriktionsfragment des Genoms von Thermus aquaticus oder der DNA des Bakteriophagen CH35:Taq#4-2 (ATCC 40,336) enthalten ist.

10. Verfahren nach Anspruch 5, wobei die Thermus aquaticus-DNA-Sequenz in einem etwa 2,8 kb großen HindIII-Asp718-Restriktionsfragment des Genoms von Thermus aquaticus oder des Plasmids pFC85 (ATCC 67,421) enthalten ist.

11. Verfahren nach einem der Ansprüche 5 bis 10, wobei die DNA-Sequenz zusätzlich ein ATG-Startcodon aufweist.

12. Verfahren nach einem der Ansprüche 5 bis 11, wobei die DNA-Sequenz ein Fusionsprotein codiert, das das thermostabile Enzym enthält.

13. Verfahren zur Herstellung eines rekombinanten Vektors, umfassend die Insertion der DNA-Sequenz oder des Fragments nach einem der Ansprüche 5 bis 12 in einen geeigneten Vektor.

14. Verfahren nach Anspruch 13, wobei der rekombinante Vektor CH35:Taq#4-2 (ATCC 40,336), pFC83 (ATCC 67,422), pFC85 (ATCC 67,421) oder ein Vektor ist, dessen Insertion zusammengesetzt ist aus dem etwa 750 bp großen BgIII/HindIII-Fragment von pFC83 und dem etwa 2,8 kbp großen HindIII/Asp718-Fragment von pFC85.

15. Verfahren nach Anspruch 13, wobei die DNA-Sequenz oder das Fragment mit einer Expressions-Kontrollsequenz funktionell verbunden sind.

16. Rekombinante Wirtszelle, enthaltend einen Vektor nach einem der Ansprüche 13 bis 15.

17. Rekombinante Wirtszelle nach Anspruch 16, die E. coli ist.

18. Verfahren zur Herstellung eines rekombinanten thermostabilen Enzyms mit DNA-Polymerase-Aktivität, eines Fragments oder einer Modifikation davon mit DNA-Polymerase-Aktivität, das (die) die Verknüpfung von Nucleosidtriphosphaten zur Bildung eines zu einem Nucleinsäure-Matrizenstrang komplementären Nucleinsäurestrangs katalysiert, wobei das Verfahren die Züchtung einer Wirtszelle nach Anspruch 16 oder 17 umfasst.

19. Verfahren nach Anspruch 18, wobei das Enzym oder die Modifikation davon ein Molekulargewicht von 86.000 bis 90.000 besitzen, bestimmt gemäß ihrer Wanderung in SDS-PAGE, wenn die Markerproteine Phosphorylase B (92.500), Rinderserumalbumin (66.200), Ovalbumin (45.000), Carboanhydrase (31.000), Sojabohnen-Trypsininhibitor (21.500) und Lysozym (14.400) sind.

20. Verfahren nach Anspruch 18 oder 19, wobei das rekombinante Enzym, das Fragment oder die Modifikation davon frei von Verunreinigungen mit thermostabiler Desoxyribonuclease-Aktivität sind.

21. Verfahren nach einem der Ansprüche 18 bis 20, wobei das rekombinante Enzym, das Fragment oder die Modifikation davon nicht irreversibel denaturiert werden, wenn sie für den zur Erzielung einer Denaturierung von doppelsträngigen Nucleinsäuren benötigten Zeitraum erhöhten Temperaturen ausgesetzt werden.

22. Verfahren nach einem der Ansprüche 18 bis 21, wobei das rekombinante Enzym, das Fragment oder die Modifikation davon bei einem pH-Wert von 6,4 mindestens 50% der Aktivität besitzen, die sie bei einem pH-Wert von 8,0 aufweisen.

23. Verfahren zur Herstellung einer stabilen Enzym-Zusammensetzung, umfassend das Kombinieren einer thermostabilen Thermus aquaticus-DNA-Polymerase hergestellt nach einem der Ansprüche 1 bis 4, oder eines rekombinanten thermostabilen Enzyms, eines Fragments oder einer Modifikation davon mit DNA-Polymerase-Aktivität, erhalten nach dem Verfahren nach einem der Ansprüche 18 bis 22 mit einem Puffer, umfassend ein oder mehrere nicht-ionische(s) polymere(s) Detergenz/Detergenzien.

24. Verfahren nach Anspruch 23, wobei die Detergenzien jeweils in einer Konzentration von etwa 0,1% bis etwa 0,5% (Vol./Vol.) der Gesamtzusammensetzung vorhanden sind.

25. Verfahren nach Anspruch 23 oder 24, wobei die Detergenzien ein polyoxyethyliertes Sorbitanmonolaurat und ein ethoxyliertes Nonylphenol sind.

26. Verfahren nach einem der Ansprüche 23 bis 25, wobei der Puffer Glycerin, Tris-Cl, pH-Wert 8,0, Ethylendiamintetraessigsäure, Dithiothreit, ein polyoxyethyliertes Sorbitanmonolaurat, ein ethoxyliertes Nonylphenol und Gelatine umfasst.

27. Thermostabile Thermus aquaticus-DNA-Polymerase, die die Verknüpfung von Nucleosidtriphosphaten zur Bildung eines zu einem Nucleinsäure-Matrizenstrang komplementären Nucleinsäurestrangs katalysiert, die ein Molekulargewicht von 86.000 bis 90.000 besitzt, bestimmt gemäß ihrer Wanderung in SDS-PAGE, wenn die Markerpoteine Phosphorylase B (92.500), Rinderserumalbumin (66.200), Ovalbumin (45.000), Carboanhydrase (31.000) Sojabohnen-Trypsininhibitor (21.500) und Lysozym (14.400) sind.

28. Thermostabiles Enzym nach Anspruch 27, das nicht irreversibel denaturiert wird, wenn es für den zur Erzielung einer Denaturierung von doppelsträngigen Nucleinsäuren benötigten Zeitraum erhöhten Temperaturen ausgesetzt wird.

29. Thermostabiles Enzym nach Anspruch 27 oder 28, das ein aus Thermus aquaticus YT1 (ATCC 25,104) stammendes Enzym ist.

30. Thermostabiles Enzym nach einem der Ansprüche 27 bis 29, das bei einem pH-Wert von 6,4 mindestens 50% der Aktivität besitzt, die es bei einem pH-Wert von 8,0 hat.

31. Thermostabiles Enzym nach einem der Ansprüche 27 bis 30, erhältlich nach einem Verfahren gemäß Anspruch 1.

32. DNA-Sequenz, die eine thermostabile Thermus aquaticus-DNA-Polymerase codiert, die die Verknüpfung von Nucleosidtriphosphaten zur Bildung eines zu einem Nucleinsäure-Matrizenstrang komplementären Nucleinsäurestrangs katalysiert, nach einem der Ansprüche 27 bis 31 oder ein Fragment der DNA-Sequenz, das ein enzymatisch aktives, verkürztes thermostabiles Enzym mit DNA-Polymerase-Aktivität codiert.

33. DNA-Sequenz nach Anspruch 32, wobei das Enzym oder Fragment davon nicht irreversibel denaturiert wird, wenn es für den zur Erzielung einer Denaturierung von doppelsträngigen Nucleinsäuren benötigten Zeitraum erhöhten Temperaturen ausgesetzt wird.

34. Thermus aquaticus-DNA-Sequenz nach Anspruch 32, die in einem nach (partieller) Spaltung gewonnen, etwa 3,5 kb großen BgIII-Asp718-Restriktionsfragment des Genoms von Thermus aquaticus oder der DNA des Bakteriophagen CH35:Taq#4-2 (ATCC 40,336) enthalten ist.

35. Thermus aquaticus-DNA-Sequenz nach Anspruch 32, die in einem etwa 2,8 kb großen HindIII-Asp718-Restriktionsfragment des Genoms von Thermus aquaticus oder des Plasmids pFC85 (ATCC 67,421) enthalten ist.

36. DNA-Sequenz nach einem der Ansprüche 32 bis 35, die zusätzlich ein ATG-Startcodon besitzt.

37. DNA-Sequenz nach einem der Ansprüche 32 bis 36, die ein Fusionsprotein codiert, das das thermostabile Enzym enthält.

38. Rekombinanter Vektor, umfassend die DNA-Sequenz oder das Fragment nach einem der Ansprüche 32 bis 37.

39. Rekombinanter Vektor nach Anspruch 38, der CH35:Taq#4-2 (ATCC 40,336), pFC83 (ATCC 67,422), pFC85 (ATCC 67,421) ein Vektor ist, dessen Insertion zusammengesetzt ist aus dem etwa 750 bp großen BgIII/HindIII-Fragment von pFC83 und dem etwa 2,8 kbp großen HindIII/Asp718-Fragment von pFC85.

40. Rekombinanter Vektor nach Anspruch 38, wobei die DNA-Sequenz oder das Fragment mit einer Expressions-Kontrollsequenz funktionell verbunden sind.

41. Rekombinantes thermostabiles Enzym mit DNA-Polymerase-Aktivität oder eine Modifikation davon mit DNA-Polymerase-Aktivität, das (die) die Verknüpfung von Nucleosidtriphosphaten zur Bildung eines zu einem Nucleinsäure-Matrizenstrang komplementären Nucleinsäurestrangs katalysiert, wobei das Enzym oder die Modifikation davon ein Molekulargewicht von 86.000 bis 90.000 besitzen, bestimmt gemäß ihrer Wanderung in SDS-PAGE, wenn die Markerproteine Phosphorylase B (92.500), Rinderserumalbumin (66.200), Ovalbumin (45.000), Carboanhydrase (31.000), Sojabohnen-Trypsininhibitor (21.500) und Lysozym (14.400) sind, wobei das Enzym durch ein Verfahren nach einem der Ansprüche 18 bis 22 erhältlich ist, wobei die Wirtszelle die rekombinante Wirtszelle nach Anspruch 17 ist.

42. Stabile Enzymzusammensetzung, die eine thermostabile Thermus aquaticus-DNA-Polymerase umfasst, die die Verknüpfung von Nucleosidtriphosphaten zur Bildung eines zu einem Nucleinsäure-Matrizenstrang komplementären Nucleinsäurestrangs katalysiert, die ein Molekulargewicht von 86.000 bis 90.000 besitzt, bestimmt gemäß ihrer Wanderung in SDS-PAGE, wenn die Markerproteine Phosphorylase B (92.500), Rinderserumalbumin (66.200), Ovalbumin (45.000), Carboanhydrase (31.000), Sojabohnen-Trypsininhibitor (21.500) und Lysozym (14.400) sind, in einem Puffer, umfassend ein oder mehrere nichtionische(s) polymere(s) Detergenz/Detergenzien.

43. Stabile Enzymzusammensetzung, die eine rekombinante thermostabile Thermus aquaticus-DNA-Polymerase oder ein Fragment davon umfasst, mit einer DNA-Polymerase-Aktivität, die erhalten wird durch
(a) das Verfahren nach einem der Ansprüche 18 bis 22 oder
(b) nach Anspruch 41
in einem Puffer, umfassend ein oder mehrere nicht-ionische(s) polymere(s) Detergenz/Detergenzien.

44. Stabile Enzymzusammensetzung, die eine rekombinante thermostabile Thermus aquaticus-DNA-Polymerase umfasst oder eine Modifikation davon mit DNA-Polymerase-Aktivität, das (die) die Verknüpfung von Nucleosidtriphosphaten zur Bildung eines zu einem Nucleinsäure-Matrizenstrang komplementären Nucleinsäurestrangs katalysiert, wobei das Enzym oder die Modifikation davon ein Molekulargewicht von 86.000 bis 90.000 besitzen, bestimmt gemäß ihrer Wanderung in SDS-PAGE, wenn die Markerproteine Phosphorylase B (92.500), Rinderserumalbumin (66.200), Ovalbumin (45.000), Carboanhydrase (31.000), Sojabohnen-Trypsininhibitor (21.500) und Lysozym (14.400) sind, in einem Puffer, umfassend ein oder mehrere nicht-ionische(s) polymere(s) Detergenz/Detergenzien.

45. Stabile Enzymzusammensetzung nach Anspruch 44, wobei das rekombinante Enzym oder die Modifikation davon nicht irreversibel denaturiert werden, wenn sie für den zur Erzielung einer Denaturierung von doppelsträngigen Nucleinsäuren benötigten Zeitraum erhöhten Temperaturen ausgesetzt werden.

46. Stabile Enzymzusammensetzung nach einem der Ansprüche 44 oder 45, wobei das rekombinante Enzym oder die Modifikation davon bei einem pH-Wert von 6,4 mindestens 50% der Aktivität besitzen, die sie bei einem pH-Wert von 8,0 aufweisen.

47. Zusammensetzung nach einem der Ansprüche 42, 43 oder 44, wobei die Detergenzien jeweils in einer Konzentration von etwa 0,1% bis etwa 0,5% (Vol./Vol.) der Gesamtzusammensetzung vorhanden sind.

48. Zusammensetzung nach einem der Ansprüche 42, 43, 44, oder 47, wobei die Detergenzien ein polyoxyethyliertes Sorbitanmonolaurat und ein ethoxyliertes Nonylphenol sind.

49. Zusammensetzung nach einem der Ansprüche 42, 43, 44, 47 oder 48, wobei der Puffer Glycerin, Tris-Cl, pH-Wert 8,0, Ethylendiamintetraessigsäure, Dithiothreit, ein polyoxyethyliertes Sorbitanmonolaurat, ein ethoxyliertes Nonylphenol und Gelatine umfasst.

50. Verwendung eines thermostabilen Enzyms mit DNA-Polymerase-Aktivität nach einem der Ansprüche 27 bis 31, des rekombinanten thermostabilen Enzyms nach Anspruch 41 oder einer stabilen Enzymzusammensetzung nach einem der Ansprüche 42 bis 44 oder 47 bis 49 für Polymerasekettenreaktionen.

51. Verfahren zur Amplifikation von Nucleinsäure-Sequenzen, umfassend die Verwendung eines thermostabilen Enzyms mit DNA-Polymerase-Aktivität nach einem der Ansprüche 27 bis 31, des rekombinanten thermostabilen Enzyms nach Anspruch 41 oder einer stabilen Enzymzusammensetzung nach einem der Ansprüche 42 bis 44 oder 47 bis 49.

## Revendications (Revendications pour l'(les) Etat(s) contractant(s) suivant(s): BE, CH, LI, DE, FR, GB, GR, IT, LU, NL, SE)

1. ADN polymérase de *Thermus aquaticus* qui catalyse la combinaison de nucléoside triphosphates pour la formation d'un brin d'acide nucléique complémentaire d'un brin matrice d'acide nucléique et qui a une masse moléculaire de 86 000 à 90 000, telle que déterminée en fonction de sa migration sur SDS-PAGE, lorsque les protéines de marquage sont la phosphorylase B (92 500), l'albumine de sérum bovin (66 200), l'ovalbumine (45 000), l'anhydrase carbonique (31 000), l'inhibiteur de trypsine de soja (21 500) et le lysozyme (14 400).

2. Enzyme thermostable selon la revendication 1, qui n'est pas irrémédiablement dénaturée quant elle est soumise à des températures élevées pendant le temps nécessaire pour effectuer la dénaturation d'acides nucléiques double brin.

3. Enzyme thermostable selon la revendication 1 ou 2, qui est une enzyme provenant de *Thermus aquaticus* YT1 (ATCC 25 104).

4. Enzyme thermostable selon l'une quelconque des revendications 1 à 3, qui a, à pH 6,4, au moins 50 % de l'activité qu'elle a à pH 8,0.

5. Séquence d'ADN codant pour une ADN polymérase de *Thermus aquaticus* qui catalyse la combinaison de nucléoside triphosphates pour la formation d'un brin d'acide nucléique complémentaire d'un brin matrice d'acide nucléique selon l'une quelconque des revendications 1 à 4, ou fragment de ladite séquence d'ADN codant pour une enzyme thermostable tronquée, enzymatiquement active, ayant une activité ADN polymérase.

6. Séquence d'ADN selon la revendication 5, dans laquelle ladite enzyme ou ledit fragment de celle-ci n'est pas irrémédiablement dénaturé(e) quand il(elle) est soumis(e) à des températures élevées pendant le temps nécessaire pour effectuer la dénaturation d'acides nucléiques double brin.

7. Séquence d'ADN de *Thermus aquaticus* selon la revendication 5, qui est contenue dans un fragment de restriction (partielle) *Bgl* II-*Asp* 718 d'approximativement 3,5 kb soit du génome de *Thermus aquaticus*, soit de l'ADN du bactériophage C35:Taq#4-2 (ATCC 40 336).

8. Séquence d'ADN de *Thermus aquaticus* selon la revendication 5, qui est contenue dans un fragment de restriction *Hin* dIII-*Asp* 718 d'approximativement 2,8 kb soit du génome de *Thermus aquaticus*, soit du plasmide pFC85 (ATCC 67 421).

9. Séquence d'ADN selon la revendication 7 ou 8, qui contient en outre un codon d'initiation ATG.

10. Séquence d'ADN selon l'une quelconque des revendications 7 à 9, qui code pour une protéine de fusion contenant ladite enzyme thermostable.

11. Vecteur recombinant comprenant la séquence d'ADN ou le fragment selon l'une quelconque des revendications 5 à 10.

12. Vecteur recombinant selon la revendication 11, qui est CH35:Taq#4-2 (ATCC 40 336), pFC83 (ATCC 67 422), pFC85 (ATCC 67 421), ou un vecteur dont l'insert est composé du fragment *Bgl* II-*Hin* dIII d'environ 750 pb de pFC83 et du fragment *Hin* dIII-*Asp* 718 d'environ 2,8 kb de pFC85.

13. Vecteur recombinant selon la revendication 11, dans lequel ladite séquence d'ADN ou ledit fragment est fonctionnellement lié(e) à une séquence régulatrice d'expression.

14. Cellule hôte recombinante contenant un vecteur selon l'une quelconque des revendications 11 à 13.

15. Cellule hôte recombinante selon la revendication 14, qui est *E*. *coli*.

16. Procédé pour la production d'une enzyme thermostable recombinante ayant une activité ADN polymérase ou d'un fragment de celle-ci ayant une activité ADN polymérase, qui catalyse la combinaison de nucléoside triphosphates pour former un brin d'acide nucléique complémentaire d'un brin matrice d'acide nucléique, ledit procédé comprenant la culture d'une cellule hôte selon la revendication 14 ou 15.

17. Procédé selon la revendication 16, dans lequel ladite enzyme ou ledit fragment de celle-ci ne sont pas irrémédiablement dénaturés quand ils sont soumis à des températures élevées pendant le temps nécessaire pour effectuer la dénaturation d'acides nucléiques double brin.

18. Enzyme recombinante thermostable ayant une activité ADN polymérase, ou modification de celle-ci ayant une activité ADN polymérase, qui catalyse la combinaison de nucléoside triphosphates pour former un brin d'acide nucléique complémentaire d'un brin matrice d'acide nucléique, ladite enzyme ou modification de celle-ci ayant une masse moléculaire de 86 000 à 90 000, telle que déterminée selon sa migration sur SDS-PAGE, lorsque les protéines de marquage sont la phosphorylase B (92 500), l'albumine de sérum bovin (66 200), l'ovalbumine (45 000), l'anhydrase carbonique (31 000), l'inhibiteur de trypsine de soja (21 500) et le lysozyme (14 400), ladite enzyme pouvant être obtenue par le procédé selon la revendication 16 ou 17, dans lequel ladite cellule hôte est la cellule hôte recombinante selon la revendication 15.

19. Composition d'enzyme stable comprenant une ADN polymérase thermostable de *Thermus aquaticus* qui catalyse la combinaison de nucléoside triphosphates pour former un brin d'acide nucléique complémentaire d'un brin matrice d'acide nucléique et qui a une masse moléculaire de 86 000 à 90 000, telle que déterminée selon sa migration sur SDS-PAGE, lorsque les protéines de marquage sont la phosphorylase B (92 500), l'albumine de sérum bovin (66 200), l'ovalbumine (45 000), l'anhydrase carbonique (31 000), l'inhibiteur de trypsine de soja (21 500) et le lysozyme (14 400) dans un tampon comprenant un ou plusieurs détergents polymères non ioniques.

20. Composition d'enzyme stable comprenant une ADN polymérase recombinante thermostable de *Thermus aquaticus* ou un fragment de celle-ci ayant une activité ADN polymérase
(a) obtenue par le procédé de la revendication 16 ou 17 ou
(b) selon la revendication 18
dans un tampon comprenant un ou plusieurs détergents polymères non ioniques.

21. Composition d'enzyme stable comprenant une ADN polymérase recombinante thermostable de *Thermus aquaticus* ou une modification de celle-ci ayant une activité ADN polymérase, qui catalyse la combinaison de nucléoside triphosphates pour former un brin d'acide nucléique complémentaire d'un brin matrice d'acide nucléique et a une masse moléculaire de 86 000 à 90 000, telle que déterminée selon sa migration sur SDS-PAGE, lorsque les protéines de marquage sont la phosphorylase B (92 500), l'albumine de sérum bovin (66 200), l'ovalbumine (45 000), l'anhydrase carbonique (31 000), l'inhibiteur de trypsine de soja (21 500) et le lysozyme (14 400) dans un tampon comprenant un ou plusieurs détergents polymères non ioniques.

22. Composition d'enzyme stable selon la revendication 21, dans laquelle l'enzyme recombinante ou la modification de celle-ci n'est pas irrémédiablement dénaturée lorsqu'elle est soumise aux températures élevées nécessaires pour effectuer la dénaturation d'acides nucléiques double brin.

23. Composition d'enzyme stable selon la revendication 21 ou 22, dans laquelle l'enzyme recombinante ou la modification de celle-ci a, à pH 6,4, au moins 50 % de l'activité qu'elle a à pH 8,0.

24. Composition selon l'une quelconque des revendications 19, 20 ou 21, dans laquelle les détergents sont présents chacun à une concentration d'environ 0,1 % à environ 0,5 % volume/volume de la composition totale.

25. Composition selon l'une quelconque des revendications 19, 20, 21 ou 24, dans laquelle les détergents consistent en un monolaurate polyoxyéthylénique de sorbitanne et un nonylphénol oxyéthylé.

26. Composition selon l'une quelconque des revendications 19, 20, 21, 24 ou 25, dans laquelle le tampon comprend du glycérol, le Tris-Cl (pH 8,0), de l'acide éthylènediaminetétraacétique, du dithiothréitol, un monolaurate polyoxyéthylénique de sorbitanne, un nonylphénol oxyéthylé et de la gélatine.

27. Utilisation d'une enzyme thermostable ayant une activité ADN polymérase selon l'une quelconque des revendications 1 à 4, de l'enzyme recombinante thermostable selon la revendication 18 ou d'une composition d'enzyme stable selon l'une quelconque des revendications 19 à 21 ou 24 à 26, pour des réactions d'amplification en chaîne par polymérase.

28. Procédé pour l'amplification de séquence d'acide nucléique comprenant l'utilisation d'une enzyme thermostable ayant une activité ADN polymérase selon l'une quelconque des revendications 1 à 4, ou de l'enzyme recombinante thermostable selon la revendication 18 ou d'une composition d'enzyme stable selon l'une quelconque des revendications 19 à 21 ou 24 à 26.

## Revendications (Revendications pour l'(les) Etat(s) contractant(s) suivant(s): AT, ES)

1. Procédé pour la préparation d'une ADN polymérase thermostable de *Thermus aquaticus* qui catalyse la combinaison de nucléoside triphosphates pour former un brin d'acide nucléique complémentaire d'un brin matrice d'acide nucléique et qui a une masse moléculaire de 86 000 à 90 000, telle que déterminée en fonction de sa migration sur SDS-PAGE, lorsque les protéines de marquage sont la phosphorylase B (92 500), l'albumine de sérum bovin (66 200), l'ovalbumine (45 000), l'anhydrase carbonique (31 000), l'inhibiteur de trypsine de soja (21 500) et le lysozyme (14 400), ledit procédé comprenant la culture d'une souche de *Thermus aquaticus* ou d'une cellule hôte recombinante hébergeant une séquence d'ADN codant pour ladite ADN polymérase, dans des conditions appropriées, et l'isolement de ladite ADN polymérase à partir de la culture.

2. Procédé selon la revendication 1, dans lequel ladite enzyme thermostable n'est pas irrémédiablement dénaturée lorsqu'elle est soumise aux températures élevées pendant le temps nécessaire pour effectuer la dénaturation d'acides nucléiques double brin.

3. Procédé selon la revendication 1 ou 2, dans lequel ladite enzyme thermostable est une enzyme issue de *Thermus aquaticus* YT1 (ATCC 25 104).

4. Procédé selon l'une quelconque des revendications 1 à 3, dans lequel ladite enzyme thermostable a, à pH 6,4, au moins 50 % de l'activité qu'elle a à pH 8,0.

5. Procédé pour la préparation d'une séquence d'ADN codant pour une ADN polymérase thermostable de *Thermus aquaticus*, qui catalyse la combinaison de nucléoside triphosphates pour former un brin d'acide nucléique complémentaire d'un brin matrice d'acide nucléique selon l'une quelconque des revendications 1 à 4, ou d'un fragment de ladite séquence d'ADN codant pour une enzyme thermostable tronquée, enzymatiquement active, à activité ADN polymérase, ledit procédé comprenant les étapes suivantes :
(a) identification d'une sonde de séquence d'ADN pour l'enzyme thermostable, par criblage immunologique d'une banque d'expression contenant le génome de *Thermus aquaticus*, à l'aide d'anticorps dirigés contre la polymérase thermostable Taq ;
(b) construction d'une banque génomique de l'ADN cible ;
(c) criblage de la banque génomique à l'aide de la sonde radiomarquée obtenue par l'étape (a) ; et
(d) isolement de phages contenant l'ADN codant pour ladite enzyme thermostable

6. Procédé selon la revendication 5, dans lequel ladite enzyme ou ledit fragment de celle-ci n'est pas irrémédiablement dénaturé(e) quand il(elle) est soumis(e) aux températures élevées pendant le temps nécessaire pour effectuer la dénaturation d'acides nucléiques double brin.

7. Procédé selon la revendication 6, dans lequel on utilise en tant que sonde une partie de l'ADN génomique de *Thermus aquaticus* codant pour au moins six aminoacides de la polymérase Taq.

8. Procédé selon la revendication 7, dans lequel ladite sonde est choisie parmi les sondes suivantes :
(a) le fragment d'ADN de *Thermus aquaticus Alu* I de ∼ 115 pb *Eco* RI-adapté de pFC85 (ATCC 67 421) ;
(b) le fragment d'ADN de *Thermus aquaticus Bgl* II-*Hin* dIII de ∼ 750 pb de pFC83 (ATCC 67 422) ;
(c) le fragment d'ADN de *Thermus aquaticus Hin* dIII-*Bam* HI de l'insert de pFC85 (ATCC 67 421) ;
(d) le fragment d'ADN de *Thermus aquaticus Hin* dIII-*Asp* 718 de ∼ 2,8 kb de pFC85 (ATCC 67 421) ; et
(e) le fragment d'ADN de *Thermus aquaticus Bam HI-Nhe* I de l'insert de pFC85 (ATCC 67 421).

9. Procédé selon la revendication 5, dans lequel la séquence d'ADN de *Thermus aquaticus* est contenue dans un fragment de restriction (partielle) *Bgl* II*-Asp* 718 d'approximativement 3,5 kb soit du génome de *Thermus aquaticus*, soit de l'ADN du bactériophage CH35:Taq#4-2 (ATCC 40 336).

10. Procédé selon la revendication 5, dans lequel la séquence d'ADN de *Thermus aquaticus* est contenue dans un fragment de restriction *Hin* dIII-Asp 718 d'approximativement 2,8 kb soit du génome de *Thermus aquaticus*, soit du plasmide pFC85 (ATCC 67 421).

11. Procédé selon l'une quelconque des revendications 5 à 10, dans lequel la séquence d'ADN contient en outre un codon d'initiation ATG.

12. Procédé selon l'une quelconque des revendications 5 à 11, dans lequel la séquence d'ADN code pour une protéine de fusion contenant ladite enzyme thermostable.

13. Procédé pour la préparation d'un vecteur recombinant, comprenant l'insertion de la séquence d'ADN ou du fragment selon l'une quelconque des revendications 5 à 12, dans un vecteur approprié.

14. Procédé selon la revendication 13, dans lequel le vecteur recombinant est CH35:Taq#4-2 (ATCC 40 336), pFC83 ATCC 67 422), pFC85 (ATCC 67 421), ou un vecteur dont l'insert est composé du fragment *Bgl* II-*Hin* dIII d'environ 750 pb de pFC83 et du fragment *Hin* dIII-*Asp* 718 d'environ 2,8 kb de pFC85.

15. Procédé selon la revendication 13, dans lequel ladite séquence d'ADN ou ledit fragment est fonctionnellement lié(e) à une séquence régulatrice d'expression.

16. Cellule hôte recombinante contenant un vecteur selon l'une quelconque des revendications 13 à 15.

17. Cellule hôte recombinante selon la revendication 16, qui est *E*. *coli*.

18. Procédé pour la production d'une enzyme recombinante thermostable ayant une activité ADN polymérase, d'un fragment ou d'une modification de celle-ci à activité ADN polymérase, qui catalyse la combinaison de nucléoside triphosphates pour former un brin d'acide nucléique complémentaire d'un brin matrice d'acide nucléique, ledit procédé comprenant la culture d'une cellule hôte selon la revendication 16 ou 17.

19. Procédé selon la revendication 18, dans lequel ladite enzyme ou modification de celle-ci a une masse moléculaire de 86 000 à 90 000, telle que déterminée selon sa migration sur SDS-PAGE, lorsque les protéines de marquage sont la phosphorylase B (92 500), l'albumine de sérum bovin (66 200), l'ovalbumine (45 000), l'anhydrase carbonique (31 000), l'inhibiteur de trypsine de soja (21 500) et le lysozyme (14 400).

20. Procédé selon la revendication 18 ou 19, dans lequel l'enzyme recombinante, le fragment ou la modification de celle-ci sont exempts d'activité désoxyribonucléase thermostable contaminante.

21. Procédé selon l'une quelconque des revendications 18 à 20, dans lequel ladite enzyme recombinante, ledit fragment ou ladite modification de celle-ci ne sont pas irrémédiablement dénaturés lorsqu'ils sont soumis aux températures élevées pendant le temps nécessaire pour effectuer la dénaturation d'acides nucléiques double brin.

22. Procédé selon l'une quelconque des revendications 18 à 21, dans lequel l'enzyme recombinante, le fragment ou la modification de celle-ci ont, à pH 6,4, au moins 50 % de l'activité qu'ils ont à pH 8,0.

23. Procédé pour la préparation d'une composition d'enzyme stable comprenant l'association d'une ADN polymérase thermostable de *Thermus aquaticus*, produite selon l'une quelconque des revendications 1 à 4, ou d'une enzyme recombinante thermostable, d'un fragment ou d'une modification de celle-ci à activité ADN polymérase, obtenu(e) par le procédé selon l'une quelconque des revendications 18 à 22, avec un tampon comprenant un ou plusieurs détergents polymères non ioniques.

24. Procédé selon la revendication 23, dans lequel les détergents sont présents chacun à une concentration d'environ 0,1 % à environ 0,5 % volume/volume de la composition totale.

25. Procédé selon la revendication 23 ou 24, dans lequel les détergents sont un monolaurate polyoxyéthylénique de sorbitanne et un nonylphénol oxyéthylé.

26. Procédé selon l'une quelconque des revendications 23 à 25, dans lequel le tampon comprend du glycérol, du Tris-Cl (pH 8,0), de l'acide éthylènediaminetétraacétique, du dithiothréitol, un monolaurate polyoxyéthylénique de sorbitanne, un nonylphénol oxyéthylé et de la gélatine.

27. ADN polymérase thermostable de *Thermus aquaticus*, qui catalyse la combinaison de nucléoside triphosphates pour former un brin d'acide nucléique complémentaire d'un brin matrice d'acide nucléique, et qui a une masse moléculaire de 86 000 à 90 000, telle que déterminée en fonction de sa migration sur SDS-PAGE, lorsque les protéines de marquage sont la phosphorylase B (92 500), l'albumine de sérum bovin (66 200), l'ovalbumine (45 000), l'anhydrase carbonique (31 000), l'inhibiteur de trypsine de soja (21 500) et le lysozyme (14 400).

28. Enzyme thermostable selon la revendication 27, qui n'est pas irrémédiablement dénaturée lorsqu'elle est soumise aux températures élevées pendant le temps nécessaire pour effectuer la dénaturation d'acides nucléiques double brin.

29. Enzyme thermostable selon la revendication 27 ou 28, qui est une enzyme issue de *Thermus aquaticus* YT1 (ATCC 25 104).

30. Enzyme thermostable selon l'une quelconque des revendications 27 à 29, qui a, à pH 6,4, au moins 50 % de l'activité qu'elle a à pH 8,0.

31. Enzyme thermostable selon l'une quelconque des revendications 27 à 30, qui peut être obtenue par un procédé tel que revendiqué dans la revendication 1.

32. Séquence d'ADN codant pour une ADN polymérase thermostable de *Thermus aquaticus*, qui catalyse la combinaison de nucléoside triphosphates pour former un brin d'acide nucléique complémentaire d'un brin matrice d'acide nucléique selon l'une quelconque des revendications 27 à 31, ou fragment de ladite séquence d'ADN codant pour une enzyme thermostable tronquée, enzymatiquement active, à activité ADN polymérase.

33. Séquence d'ADN selon la revendication 32, dans laquelle ladite enzyme ou ledit fragment de celle-ci n'est pas irrémédiablement dénaturé(e) quand il(elle) est soumis(e) aux températures élevées pendant le temps nécessaire pour effectuer la dénaturation d'acides nucléiques double brin.

34. Séquence d'ADN de *Thermus aquaticus* selon la revendication 32, qui est contenue dans un fragment de restriction (partielle) *Bgl II-Asp* 718 d'approximativement 3,5 kb soit du génome de *Thermus aquaticus*, soit de l'ADN du bactériophage CH35:Taq#4-2 (ATCC 40 336).

35. Séquence d'ADN de *Thermus aquaticus* selon la revendication 32, qui est contenue dans un fragment de restriction *Hin* dIII-Asp 718 d'approximativement 2,8 kb soit du génome de *Thermus aquaticus*, soit du plasmide pFC85 (ATCC 67 421).

36. Séquence d'ADN selon l'une quelconque des revendications 32 à 35, qui contient en outre un codon d'initiation ATG.

37. Séquence d'ADN selon l'une quelconque des revendications 32 à 36, qui code pour une protéine de fusion contenant ladite enzyme thermostable.

38. Vecteur recombinant comprenant la séquence d'ADN ou un fragment de celle-ci selon l'une quelconque des revendications 32 à 37.

39. Vecteur recombinant selon la revendication 38, qui est CH35:Taq#4-2 (ATCC 40 336), pFC83 (ATCC 67 422), pFC85 (ATCC 67 421), ou un vecteur dont l'insert est composé du fragment *Bgl* II-*Hin* dIII d'environ 750 pb de pFC83 et du fragment *Hin* dIII-*Asp* 718 d'environ 2,8 kb de pFC85.

40. Vecteur recombinant selon la revendication 38, dans lequel ladite séquence d'ADN ou ledit fragment est fonctionnellement lié(e) à une séquence régulatrice d'expression.

41. Enzyme recombinante thermostable à activité ADN polymérase, ou modification de celle-ci à activité ADN polymérase, qui catalyse la combinaison de nucléoside triphosphates pour former un brin d'acide nucléique complémentaire d'un brin matrice d'acide nucléique, ladite enzyme ou modification de celle-ci ayant une masse moléculaire de 86 000 à 90 000, telle que déterminée selon sa migration sur SDS-PAGE, lorsque les protéines de marquage sont la phosphorylase B (92 500), l'albumine de sérum bovin (66 200), l'ovalbumine (45 000), l'anhydrase carbonique (31 000), l'inhibiteur de trypsine de soja (21 500) et le lysozyme (14 400), ladite enzyme pouvant être obtenue par le procédé selon l'une quelconque des revendication 18 à 22, ladite cellule hôte étant la cellule hôte recombinante selon la revendication 17.

42. Composition d'enzyme stable comprenant une ADN polymérase thermostable de *Thermus aquaticus*, qui catalyse la combinaison de nucléoside triphosphates pour former un brin d'acide nucléique complémentaire d'un brin matrice d'acide nucléique et qui a une masse moléculaire de 86 000 à 90 000, telle que déterminée selon sa migration sur SDS-PAGE, lorsque les protéines de marquage sont la phosphorylase B (92 500), l'albumine de sérum bovin (66 200), l'ovalbumine (45 000), l'anhydrase carbonique (31 000), l'inhibiteur de trypsine de soja (21 500) et le lysozyme (14 400) dans un tampon comprenant un ou plusieurs détergents polymères non ioniques.

43. Composition d'enzyme stable comprenant une ADN polymérase recombinante thermostable de *Thermus aquaticus* ou un fragment de celle-ci ayant une activité ADN polymérase obtenu(e) par
(a) le procédé de la revendication 18 ou 22 ou
(b) selon la revendication 41
dans un tampon comprenant un ou plusieurs détergents polymères non ioniques.

44. Composition d'enzyme stable comprenant une ADN polymérase recombinante thermostable de *Thermus aquaticus* ou une modification de celle-ci ayant une activité ADN polymérase, qui catalyse la combinaison de nucléoside triphosphates pour former un brin d'acide nucléique complémentaire d'un brin matrice d'acide nucléique et a une masse moléculaire de 86 000 à 90 000, telle que déterminée selon sa migration sur SDS-PAGE, lorsque les protéines de marquage sont la phosphorylase B (92 500), l'albumine de sérum bovin (66 200), l'ovalbumine (45 000), l'anhydrase carbonique (31 000), l'inhibiteur de trypsine de soja (21 500) et le lysozyme (14 400) dans un tampon comprenant un ou plusieurs détergents polymères non ioniques.

45. Composition d'enzyme stable selon la revendication 44, dans laquelle l'enzyme recombinante ou la modification de celle-ci n'est pas irrémédiablement dénaturée lorsqu'elle est soumise aux températures élevées nécessaires pour effectuer la dénaturation d'acides nucléiques double brin.

46. Composition d'enzyme stable selon la revendication 44 ou 45, dans laquelle l'enzyme recombinante ou la modification de celle-ci a, à pH 6,4, au moins 50 % de l'activité qu'elle a à pH 8,0.

47. Composition selon l'une quelconque des revendications 42, 43 et 44, dans laquelle les détergents sont présents chacun à une concentration d'environ 0,1 % à environ 0,5 % volume/volume de la composition totale.

48. Composition selon l'une quelconque des revendications 42, 43, 44 ou 47, dans laquelle les détergents consistent en un monolaurate polyoxyéthylénique de sorbitanne et un nonylphénol oxyéthylé.

49. Composition selon l'une quelconque des revendications 42, 43, 44, 47 ou 48, dans laquelle le tampon comprend du glycérol, Tris-Cl (pH 8,0), de l'acide éthylènediaminetétraacétique, du dithiothréitol, un monolaurate polyoxyéthylénique de sorbitanne, un nonylphénol oxyéthylé et de la gélatine.

50. Utilisation d'une enzyme thermostable à activité ADN polymérase selon l'une quelconque des revendications 27 à 31, de l'enzyme recombinante thermostable selon la revendication 41 ou d'une composition d'enzyme stable selon l'une quelconque des revendications 42 à 44 ou 47 à 49, pour des réactions d'amplification en chaîne par polymérase.

51. Procédé pour l'amplification de séquences d'acide nucléique comprenant l'utilisation d'une enzyme thermostable à activité ADN polymérase selon l'une quelconque des revendications 27 à 31, ou de l'enzyme recombinante thermostable selon la revendication 41 ou d'une composition d'enzyme stable selon l'une quelconque des revendications 42 à 44 ou 47 à 49.
